# EUROPEAN PATENT APPLICATION

(11) **EP 4 410 962 A1**
(43) Date of publication of application: **07.08.2024**
(21) Application number: 22876261.3
(22) Date of filing: 27.09.2022
(51) Int. Cl.: C12N 5/071, A61K 35/12, A61K 35/30, A61K 35/545, A61P 5/10, C12N 5/0735, C12N 5/079

(54) **CELL AGGREGATE INCLUDING PITUITARY HORMONE-PRODUCING CELLS AND METHOD FOR PRODUCING SAME**

(30) Priority: 30.09.2021 JP 2021162253; 21.07.2022 JP 2022116715
(71) Applicant: Sumitomo Pharma Co., Ltd., Osaka 541-0045 (JP); National University Corporation Tokai National Higher Education and Research System, Nagoya-shi, Aichi 464-8601 (JP); Sumitomo Chemical Company, Limited, Tokyo 103-6020 (JP)
(72) Inventor: TAGA, Shiori, Kobe-shi, Hyogo 650-0047 (JP); KUWAHARA, Atsushi, Kobe-shi, Hyogo 650-0047 (JP); SUGA, Hidetaka, Nagoya-shi, Aichi 464-8601 (JP); NAKANO, Tokushige, Osaka-shi, Osaka 554-8558 (JP)
(74) Representative: J A Kemp LLP
(86) International application number: PCT/JP2022/036018
(87) International publication number: WO 2023/054395

(57) **Abstract**

The present invention provides a method for efficiently producing a cell aggregate containing pituitary hormone-producing cells from pluripotent stem cells.

## Description

### [Technical Field]

The present invention relates to cell aggregates containing pituitary hormone-producing cells induced to differentiate from pluripotent stem cells, in vitro, and production methods thereof.

### [Background Art]

The pituitary gland is a small endocrine organ that exists adjacent to the lower part of the diencephalon and plays a control center in the regulation of various hormones. For example, it produces various pituitary hormones including adrenocorticotropic hormone (ACTH), which promotes the production of adrenocortical hormones essential for life support, growth hormone, which promotes the growth of children, and the like. Therefore, pituitary dysfunction causes serious systemic diseases.

In recent years, a method for inducing differentiation of human ES/iPS cells into the adenohypophysis comprising functional pituitary hormone-producing cells has been developed, and its application to hypophyseal regenerative medicine is expected (Patent Literature 1, Non Patent Literatures 1 and 2). In these methods, a hypothalamic-pituitary composite tissue is formed in a cell mass by three-dimensional culture. In the composite tissue, the adenohypophysis and its precursor tissue are mainly located on the surface layer of the cell mass. By detaching and transplanting them to under the renal capsule of hypophysectomized mice, therapeutic effects such as improvement of activity, survival, body weight decrease and the like have been confirmed (Patent Literature 1, Non Patent Literature 1). In addition, it has been reported that a cell mass containing pituitary tissue can be efficiently produced by culturing human ES/iPS cells in suspension in the presence of a Wnt signal transduction pathway inhibiting substance, and adding a BMP signal transduction pathway activating substance and a Sonic hedgehog signal transduction pathway activating substance (Patent Literature 2). Also in this method, it was found that neuroepithelial tissue of the ventral diencephalon and hypothalamus is present inside the cell mass, and a Rathke's pouch-like structure and pituitary placode are formed in a part of the non-neuroepithelial tissue of the oral cavity that exists outside. However, even though methods for differentiating human ES/iPS cells into cell masses containing functional pituitary hormone-producing cells are known, cell markers for efficiently separating only pituitary hormone-producing cells from the cell masses and the timing of the separation were not known at all.

### [Citation List]

### [Patent Literature]

[Patent Literature 1]
   WO 2016/013669
[Patent Literature 2]
   WO 2019/103129

### [Non Patent Literature]

[Non Patent Literature 1]
   Nature Communications, 7:10351, 2016
[Non Patent Literature 2]
   Cell Reports, 30, 18-24, January 7, 2020

### [Summary of Invention]

### [Technical Problem]

The present inventors aim to provide aggregates of pituitary hormone-producing cells with superior pituitary hormone secretory capacity, by searching for a timing suitable for separating pituitary hormone-producing cells and progenitor cells thereof (pituitary progenitor cells) from cell masses undergoing cultivation to differentiate pluripotent stem cells into pituitary tissue, reaggregating the cells separated at that timing, and further culturing the cells.

### [Solution to Problem]

The present inventors have conducted intensive studies and found the timing at which pituitary hormone-producing cells and pituitary progenitor cells can be most effectively separated and purified from the cell masses undergoing cultivation to differentiate pluripotent stem cells into pituitary hormone-producing cells, by identifying an epithelial cell adhesion molecule (hereinafter abbreviated as EpCAM) as a surface antigen specific to pituitary hormone-producing cells and pituitary progenitor cells and sorting using an anti-EpCAM antibody. In addition, it was found that aggregates resulting from further differentiation culture of the separated and purified pituitary hormone-producing cells and pituitary progenitor cells and reaggregation thereof exhibit superior pituitary hormone secretory capacity, which resulted in the completion of the present invention.

Accordingly, the present invention provides the following.
[1] A cell aggregate containing a pituitary hormone-producing cell, wherein
   (1) the percentage of cells positive for at least one of EpCAM and E-cadherin in the cell aggregate is not less than 80%,
   (2) the percentage of neural cells in the cell aggregate is not more than 20%;
   (3) the percentage of ACTH-positive cells in the cell aggregate is not less than 5%;
   (4) the cell density of the cell aggregate is 3,000 to 20,000 cells/mm²,
   (5) the major axis of the cell aggregate is 200 to 3,000 µm,
   (6) on the surface of the cell aggregate, cells positive for at least one of EpCAM and E-cadherin form a plane attachment to each other to form an epithelial structure, and
   (7) the cell aggregate forms a sponge-like structure.
[2] The cell aggregate of [1], wherein the percentage of pituitary stem cells in the cell aggregate is not less than 3%.
[3] The cell aggregate of [1], wherein the cell aggregate has an ACTH secretory capacity of 200 to 1,000,000 pg/mL.
[4] The cell aggregate of [1], wherein the cell aggregate has a GH secretory capacity of 0.05 to 100 ng/mL.
[5] A cell population comprising the cell aggregates of any one of [1] to [4] in not less than 40% of the total number of cell aggregates.
[6] A therapeutic drug for a disease caused by a disorder of the pituitary gland, comprising the cell aggregate of any one of [1] to [4].
[7] A method for producing a cell aggregate containing a pituitary hormone-producing cell, including the following steps:
   (1) a step of separating a cell expressing EpCAM from a dispersed cell population derived from a pluripotent stem cell and including a hypothalamic cell, a pituitary progenitor cell, and a pituitary hormone-producing cell, and
   (2) a step of suspension culturing the separated EpCAM-expressing cell for not less than 14 days under conditions that allow the pituitary progenitor cell to mature into a pituitary hormone-producing cell.
[8] The production method of [7], wherein the cell population of step (1) is obtained by the following step (A) and step (B):
   (A) a step of culturing a pluripotent stem cell or a cell aggregate thereof under conditions that can induce differentiation into a cell aggregate containing a hypothalamic cell, a pituitary progenitor cell, and a pituitary hormone-producing cell, and
   (B) a step of dispersing the cell aggregate containing a hypothalamic cell, a pituitary progenitor cell, and a pituitary hormone-producing cell, obtained in (A), into a cell population.
[9] The production method of [8], wherein the step (A) comprises the following steps:
   (1') a first step of culturing pluripotent stem cells in the presence of a Wnt signal transduction pathway inhibiting substance to form a cell aggregate,
   (2) a second step of culturing the cell aggregate, obtained in the first step, in the presence of a BMP signal transduction pathway activating substance and a Shh signal transduction pathway activating substance to obtain a cell aggregate containing a hypothalamic cell, a pituitary progenitor cell, and a pituitary hormone-producing cell.
[10] The production method of [8], wherein the step (A) comprises the following steps:
   (a) step a of culturing pluripotent stem cells in the absence of feeder cells and in a medium comprising 1) a TGFβ family signal transduction pathway inhibiting substance and/or a Shh signal transduction pathway activating substance, and 2) a factor for maintaining an undifferentiated state,

   (1') a first step of suspension culturing the pluripotent stem cells, obtained in step a, in the presence of a Wnt signal transduction pathway inhibiting substance to form a cell aggregate,
   (2) a second step of suspension culturing the cell aggregate, obtained in the first step, in the presence of a BMP signal transduction pathway activating substance and a Shh signal transduction pathway activating substance to obtain a cell aggregate containing a hypothalamic cell, a pituitary progenitor cell, and a pituitary hormone-producing cell.
[11] The production method of [8], wherein the step (A) comprises the following steps:
   (a) step a of culturing pluripotent stem cells in the absence of feeder cells and in a medium comprising 1) a TGFβ family signal transduction pathway inhibiting substance and/or an Shh signal transduction pathway activating substance, and 2) a factor for maintaining an undifferentiated state,

   (1) a first step of suspension culturing the pluripotent stem cells, obtained in step a, in the presence of a JNK signal transduction pathway inhibiting substance and a Wnt signal transduction pathway inhibiting substance to form a cell aggregate,
   (2) a second step of suspension culturing the cell aggregate, obtained in the first step, in the presence of a BMP signal transduction pathway activating substance and a Shh signal transduction pathway activating substance to obtain a cell aggregate containing a hypothalamic cell, a pituitary progenitor cell, and a pituitary hormone-producing cell.
[12] The production method of [11], wherein the suspension culturing in the first step is further in the presence of a Shh signal transduction pathway activating substance, and a period of the culture in the presence of a Shh signal transduction pathway activating substance in the first step and the second step is 30 days.
[13] The production method of any one of [9] to [12], wherein the cell aggregate obtained in the second step is further suspension cultured in the absence of a Shh signal transduction pathway activating substance.
[14] The production method of any one of [8] to [13], wherein the culture period in step (A) is a period with which a cell aggregate containing a pituitary hormone-producing cell that secretes ACTH and not comprising a pituitary hormone-producing cell that secretes GH or TSH is obtained.
[15] The production method of any one of [8] to [14], wherein the culture period in step (A) is not less than 30 days and not more than 100 days.
[16] The production method of any one of [7] to [15], wherein the cell aggregate containing a pituitary hormone-producing cell has the following characteristics:
   (1) the percentage of cells positive for at least one of EpCAM and E-cadherin in the cell aggregate is not less than 80%,
   (2) the percentage of neural cells in the cell aggregate is not more than 20%;
   (3) the percentage of ACTH-positive cells in the cell aggregate is not less than 5%;
   (4) the cell density of the cell aggregate is 3,000 to 20,000 cells/mm²,
   (5) the major axis of the cell aggregate is 200 to 3,000 um,
   (6) on the surface of the cell aggregate, cells positive for at least one of EpCAM and E-cadherin form a plane attachment to each other to form an epithelial structure, and
   (7) the cell aggregate forms a sponge-like structure.
[17] The production method of [16], wherein the percentage of pituitary stem cells in the cell aggregate is not less than 3%.
[18] The production method of [16], wherein the cell aggregate has an ACTH secretory capacity of 200 to 1,000,000 pg/mL.
[19] The production method of [16], wherein the cell aggregate has a GH secretory capacity of 0.05 to 100 ng/mL.

### [Advantageous Effects of Invention]

According to the present invention, functional pituitary hormone-producing cells and pituitary progenitor cells thereof can be efficiently separated and purified, by utilizing EpCAM and separating EpCAM positive cell population at a specific timing from the cell masses undergoing cultivation to differentiate pluripotent stem cells into pituitary tissue. In addition, aggregates of pituitary hormone-producing cells obtained by reaggregating the separated and purified pituitary hormone-producing cells and pituitary progenitor cells, followed by further differentiation culture exhibit superior pituitary hormone secreting ability by physiological stimulation of pituitary hormone secretion. Therefore, the diseases relating to pituitary gland can be treated using the cells.

### [Brief Description of Drawings]

[Fig. 1]

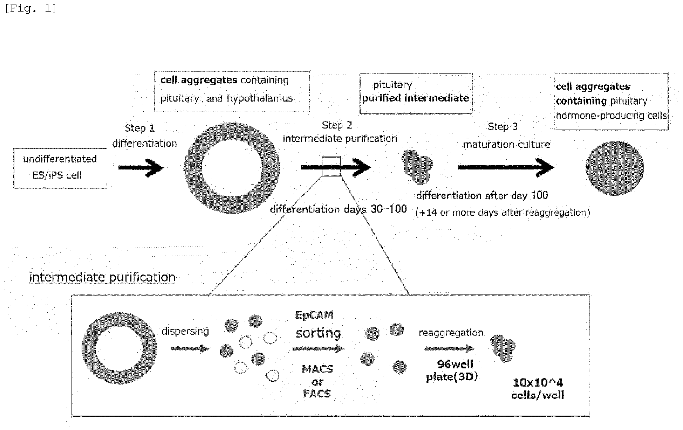

   Fig. 1 is a diagram showing the entire steps for producing a cell aggregate containing pituitary hormone-producing cells from human ES/iPS cells. The production steps include 3 steps of (1) a step of differentiating human ES/iPS cells into a cell aggregate containing a hypothalamic cell, a pituitary progenitor cell, and a pituitary hormone-producing cell (step 1 differentiation), (2) a step of separating an EpCAM-positive cell population from the cell aggregate containing a hypothalamic cell, a pituitary progenitor cell, and a pituitary hormone-producing cell (step 2 intermediate purification), (3) a step of producing a cell aggregate containing pituitary hormone-producing cells by culturing the separated EpCAM-positive cell population for a long period (step 3 maturation culture).
[Fig. 2]

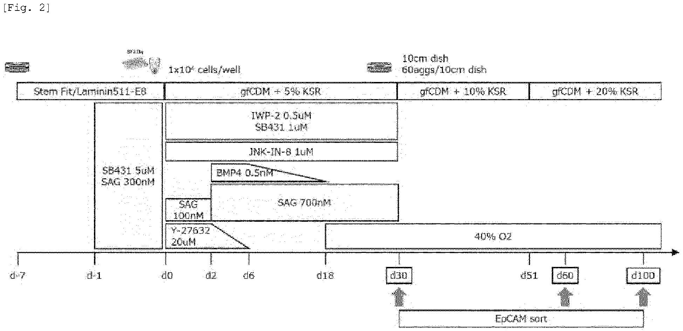

   Fig. 2 shows a protocol for differentiating human ES cells into a cell aggregate containing a hypothalamic cell, a pituitary progenitor cell, and a pituitary hormone-producing cell.
[Fig. 3]

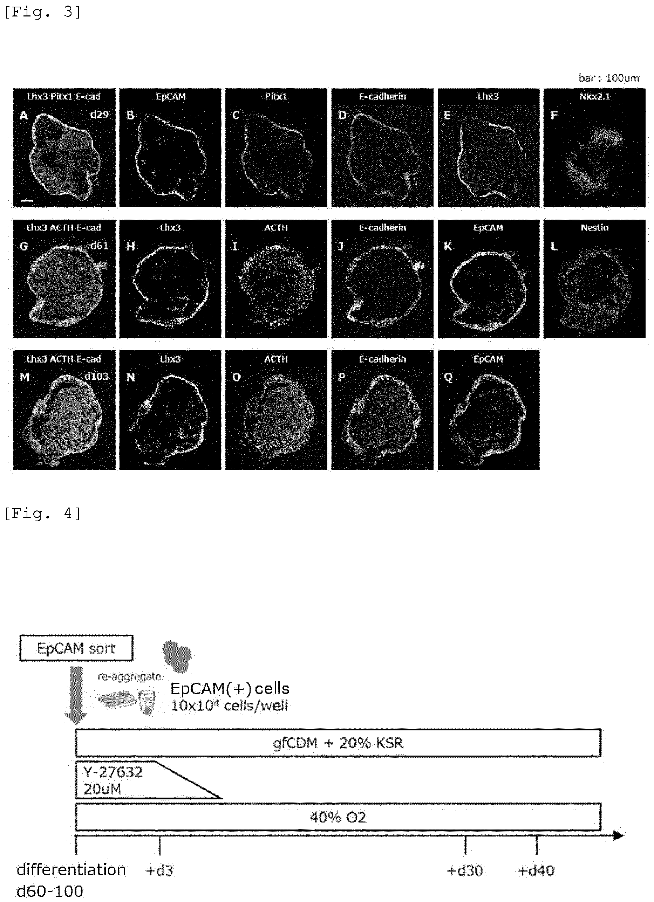

   Fig. 3 shows diagrams confirming the expression of various cell markers in cell aggregates on days 29, 61, and 103 after the start of differentiation induction.
[Fig. 4]
   Fig. 4 shows a protocol for reaggregating purified EpCAM-positive cells and suspension culturing them for a long period.
[Fig. 5]

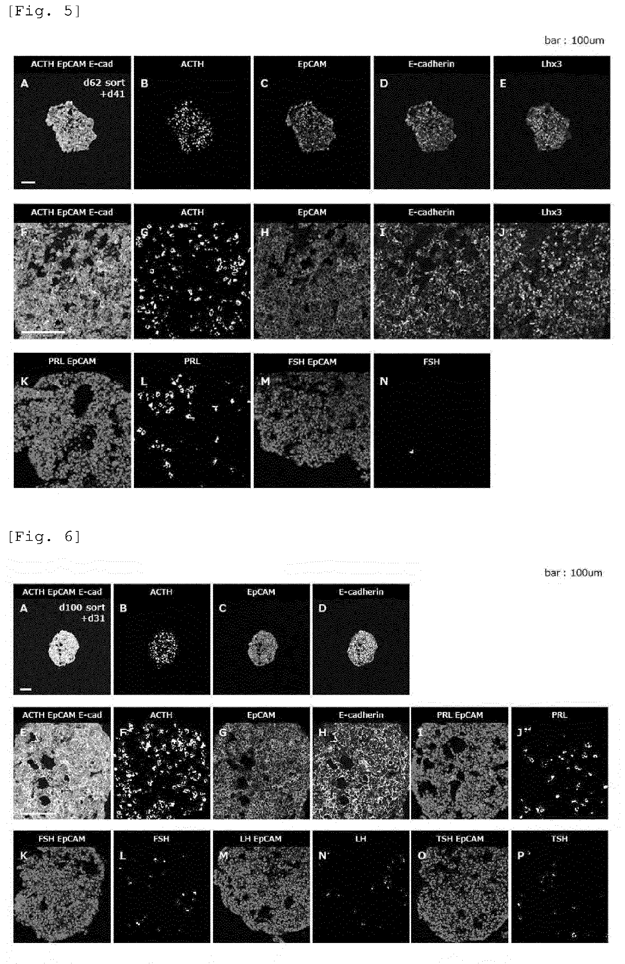

   Fig. 5 shows diagrams confirming the expression of various cell markers in cell aggregates obtained by purifying cell aggregates on day 62 after the start of differentiation induction, reaggregating them, and then culturing them for a long period of 41 days (day 62 sort + day 41).
[Fig. 6]
   Fig. 6 shows diagrams confirming the expression of various cell markers in cell aggregates obtained by purifying cell aggregates on day 100 after the start of differentiation induction, reaggregating them, and then culturing them for a long period of 31 days (day 100 sort + day 31).
[Fig. 7]

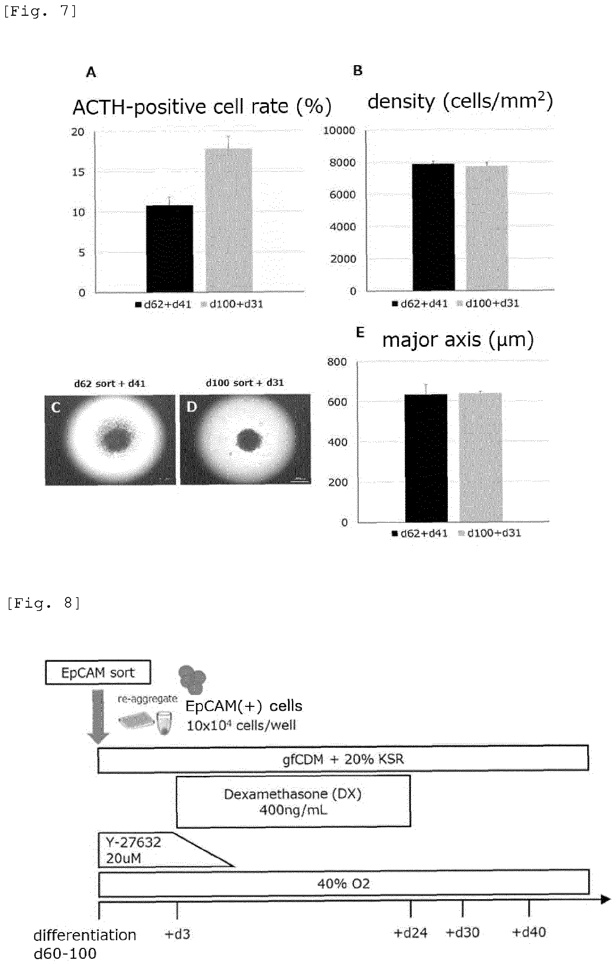

   Fig. 7 shows diagrams and a bright field image quantifying the ACTH-positive cell rate, cell density, and major axis in cell aggregates of day 62 sort + day 41 and of day 100 sort + day 31.
[Fig. 8]
   Fig. 8 shows a protocol for reaggregating purified EpCAM-positive cells and suspension culturing them for a long period under dexamethasone (DX) addition conditions.
[Fig. 9]

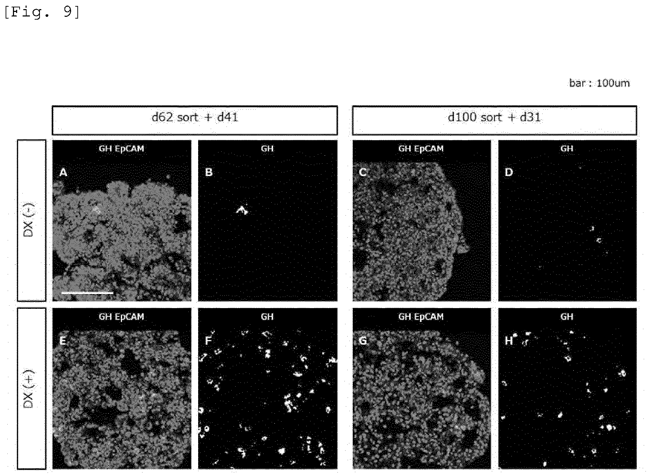

   Fig. 9 shows diagrams confirming the expression of an epithelial cell marker EpCAM and growth hormone (GH) which is a pituitary hormone-producing cell marker in cell aggregate of day 62 sort + day 41 and of day 100 sort + day 31.
[Fig. 10]

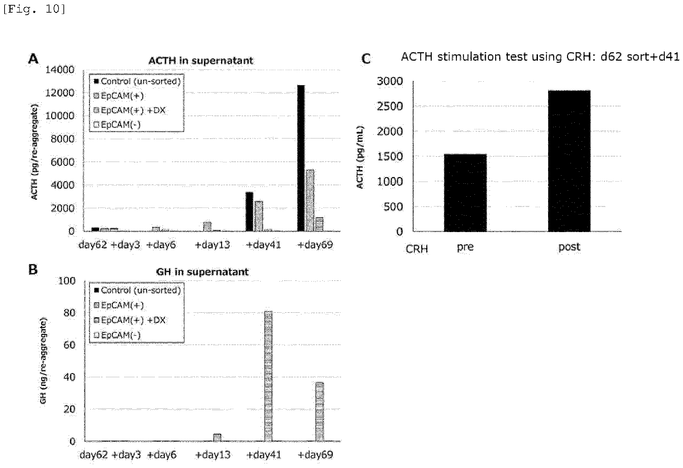

   Fig. 10A and 10B are diagrams showing the measurement results over time of the amounts of ACTH and GH in the culture supernatants of the EpCAM-positive cell aggregates and EpCAM-negative cell aggregates respectively obtained by purifying and reaggregating the cell aggregates obtained on day 62 after the start of differentiation induction. Fig. 10C shows a diagram with the results of an ACTH stimulation test using CRH and performed using the cell aggregates obtained by purifying and reaggregating the cell aggregates on day 62 after the start of differentiation induction, and then suspension culturing them for 41 days.
[Fig. 11]

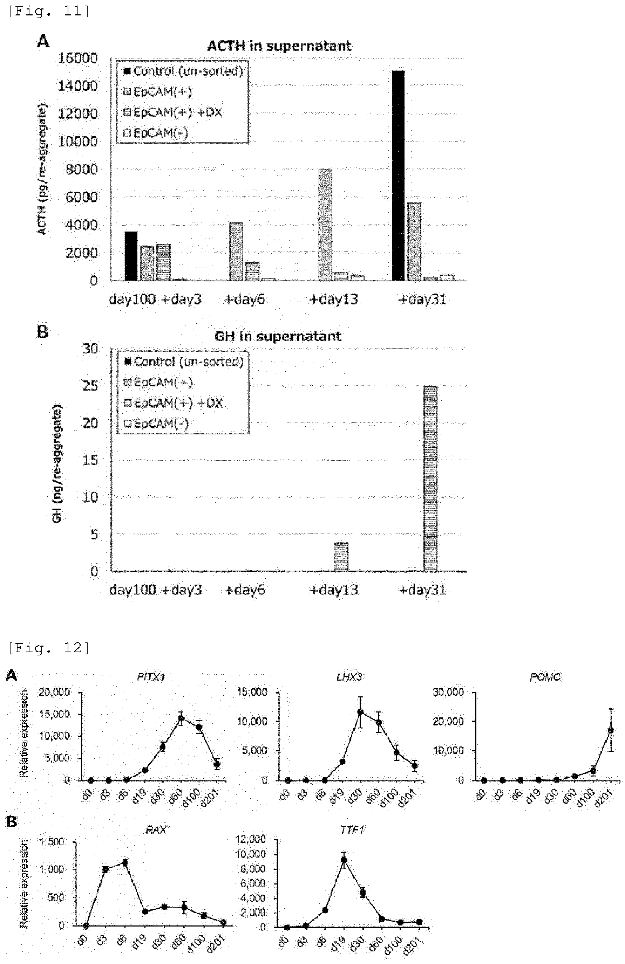

   Fig. 11A and 11B are diagrams showing the measurement results over time of the amounts of ACTH and GH in the culture supernatants of the EpCAM-positive cell aggregate and EpCAM-negative cell aggregate respectively obtained by purifying and reaggregating the cell aggregates obtained on day 100 after the start of differentiation induction.
[Fig. 12]
   Fig. 12 shows diagrams with the measurement results of the expression levels of various cell markers in the cell aggregates on days 3, 6, 19, 30, 60, 100, and 201 after the start of differentiation induction.
[Fig. 13]

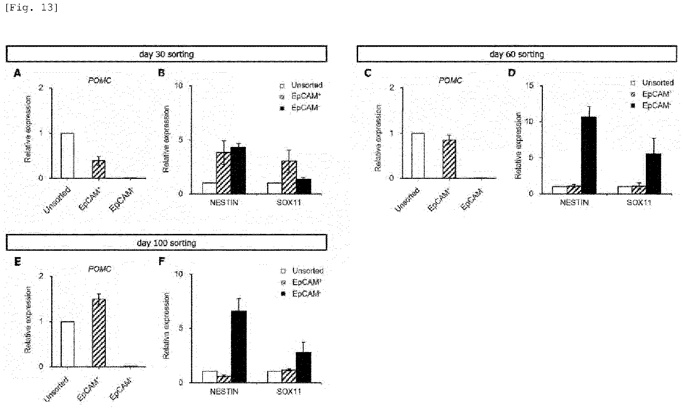

   Fig. 13 shows diagrams confirming the expression of various cell markers in cell aggregates obtained by purifying and reaggregating the cell aggregates obtained on days 30, 60, and 100 after the start of differentiation induction, and culturing them up to day 131 after the start of differentiation induction.
[Fig. 14]

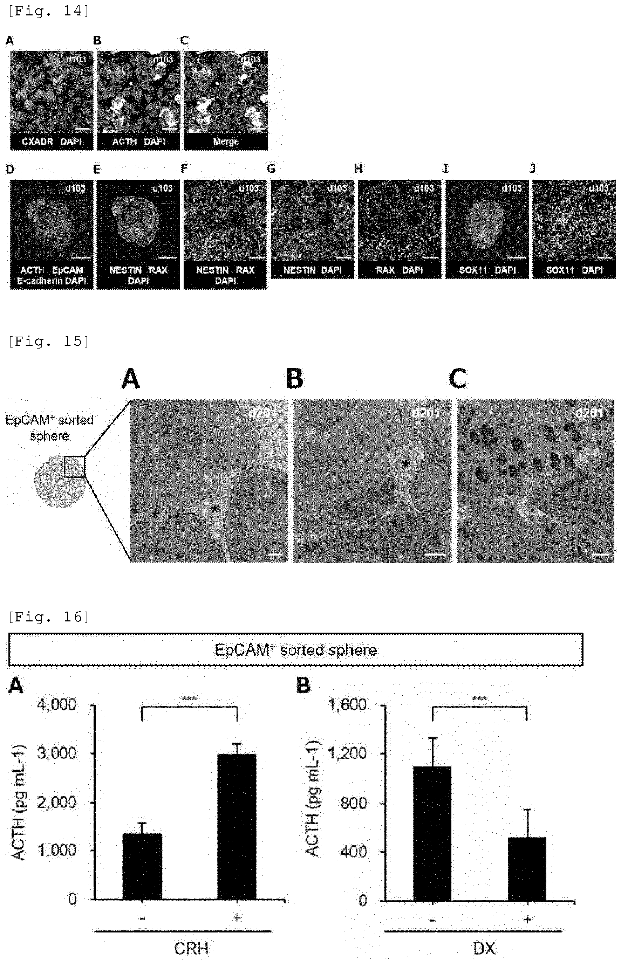

   Fig. 14 shows diagrams confirming the expression of various cell markers in cell aggregates obtained by purifying and reaggregating the cell aggregates obtained on day 60 after the start of differentiation induction, and culturing them up to day 103 after the start of differentiation induction. A to C are reaggregates of EpCAM-positive cells, and D to J are reaggregates of EpCAM-negative cells. The scale bars in D, E, and I indicate 200 um, and the scale bars in A to C, F to H, and J indicate 50 µm.
[Fig. 15]
   Fig. 15 shows diagrams of electron microscopy of cell aggregates obtained by purifying and reaggregating the cell aggregates obtained on day 60 after the start of differentiation induction, and culturing them up to day 201 after the start of differentiation induction. The scale bars in A and B indicate 2 um, and the scale bar in C indicates 500 nm.
[Fig. 16]
   Fig. 16 shows diagrams with the results of an ACTH stimulation test using CRH or dexamethasone and performed using cell aggregates obtained by purifying and reaggregating the cell aggregates obtained on day 60 after the start of differentiation induction, and culturing them up to day 103 after the start of differentiation induction. **:p<0.01, paired t-test.
[Fig. 17]

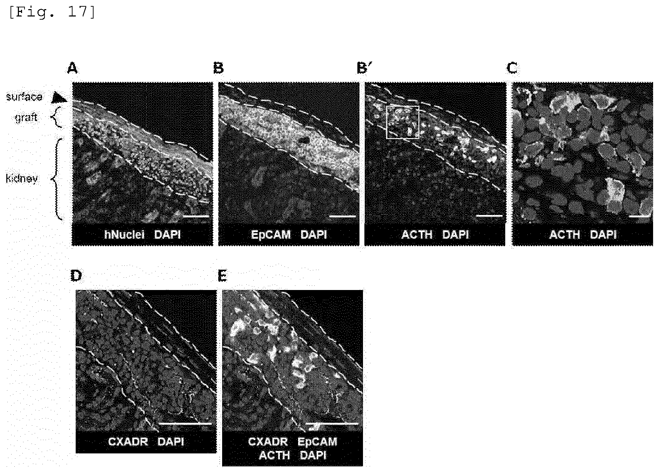

   Fig. 17 shows diagrams confirming the expression of various cell markers in the kidney of hypopituitary mice 24 weeks after transplantation of reaggregated EpCAM-positive cells into the kidney capsule.
[Fig. 18]

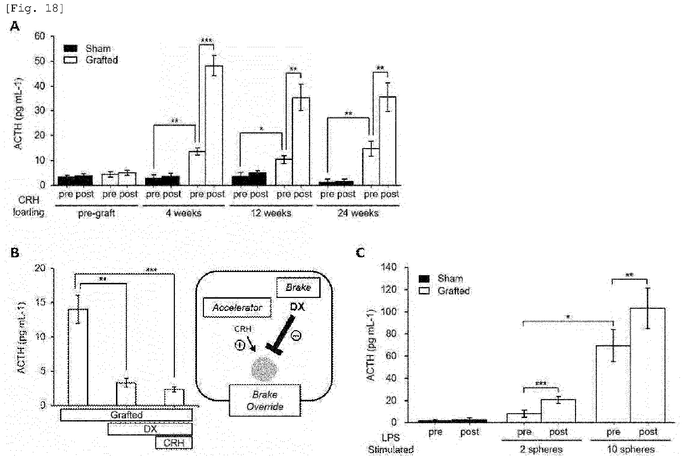

   Fig. 18 shows diagrams confirming the effects of CRH, dexamethasone, or LPS stimulation on ACTH secretion in hypopituitry mice transplanted with EpCAM-positive cells into the kidney capsule. In Fig. 18A, *:p<0.05, **:p<0.01, ***:p<0.001, Mann-Whitney test (sham versus grafted), paired t-test (grafted pre versus post). In Figs. 18B and C, **:p<0.01, ***:p<0.001, paired t-test.
[Fig. 19]

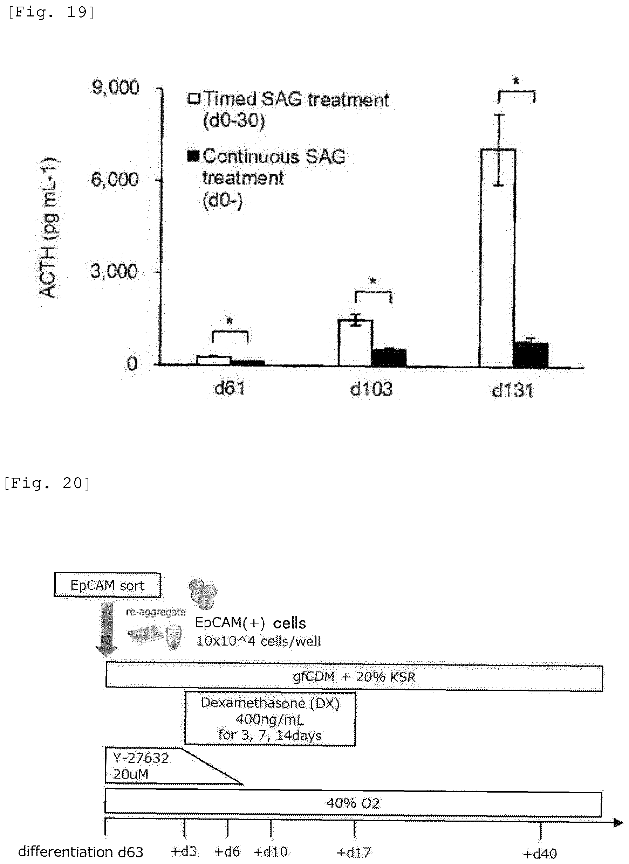

   Fig. 19 is a diagram comparing the ACTH secretory capacity between when, in a protocol for differentiating human iPS cells into a cell aggregate containing a hypothalamic cell, a pituitary progenitor cell, and a pituitary hormone-producing cell, SAG treatment was performed up to day 30 after the start of differentiation induction, and when SAG treatment was performed continuously thereafter. *:p<0.05, Student's t-test.
[Fig. 20]
   Fig. 20 shows a protocol for reaggregating purified EpCAM-positive cells and suspension culturing them for a long period under DX addition conditions with different addition periods.
[Fig. 21]

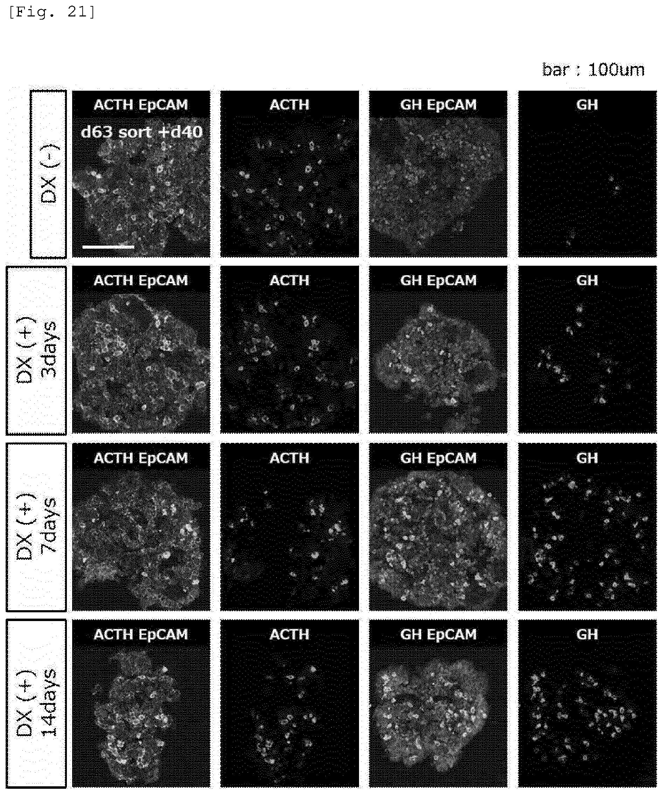

   Fig. 21 shows diagrams confirming the expression of an epithelial cell marker EpCAM and growth hormone (GH) which is a pituitary hormone-producing cell marker in cell aggregates obtained by purifying cell aggregates on day 63 after the start of differentiation induction, reaggregating them, treating them with DX for 3, 7, or 14 days after 3 days later, and culturing them up to day 103 after the start of differentiation induction.
[Fig. 22]

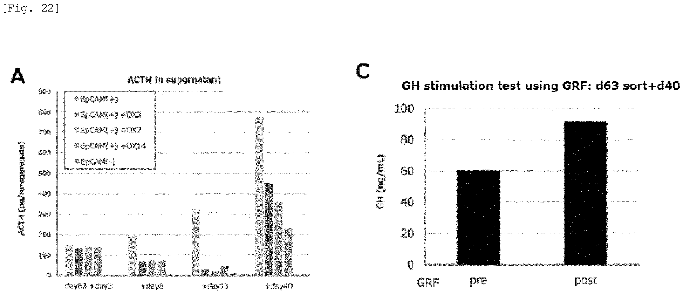

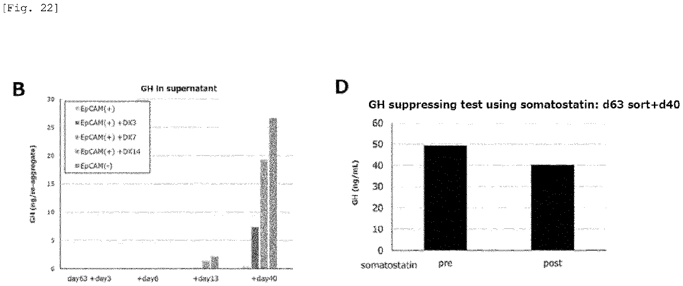

   Fig. 22A and 22B are diagrams showing the measurement results over time of the amounts of ACTH and GH in the culture supernatants of cell aggregates obtained by purifying cell aggregates on day 63 after the start of differentiation induction, reaggregating them, treating them with DX for 3, 7, or 14 days after 3 days later, and culturing them up to day 103 after the start of differentiation induction. Fig. 22C shows a diagram with the results of a GH stimulation test using CRF and performed using the cell aggregates obtained by purifying cell aggregates on day 63 after the start of differentiation induction, reaggregating them, treating them with DX for 14 days after 3 days later, and culturing them up to day 103 after the start of differentiation induction. Fig. 22D shows a diagram with the results of a GH suppressing test using somatostatin and performed using the cell aggregates obtained by purifying cell aggregates on day 63 after the start of differentiation induction, reaggregating them, treating them with DX for 14 days after 3 days later, and culturing them up to day 103 after the start of differentiation induction.

### [Description of Embodiments]

### [Production method of cell aggregate containing pituitary hormone-producing cell]

In the present specification, the definitions of the terms are as follows. The "stem cell" means an undifferentiated cell having differentiation potency and proliferative capacity (particularly self-renewal competence). The stem cell includes pluripotent stem cell, multipotent stem cell, unipotent stem cell and the like according to the differentiation potency. The "pluripotent stem cell" refers to a stem cell capable of being cultured in vitro and having a potency to differentiate into any cell constituting living organisms (pluripotency). Any cell refers to cells derived from three germ layers of ectoderm, mesoderm, and endoderm. The "multipotent stem cell" means a stem cell having a potency to differentiate into plural types of tissues or cells, though not all kinds. The "unipotent stem cell" means a stem cell having a potency to differentiate into a particular tissue or cell.

Pluripotent stem cell can be induced from fertilized egg, clone embryo, germ stem cell, stem cell in a tissue, somatic cell or the like. Examples of the pluripotent stem cell include embryonic stem cell (ES cell), EG cell (embryonic germ cell), and induced pluripotent stem cell (iPS cell). Muse cell (Multi-lineage differentiating stress enduring cell) obtained from mesenchymal stem cell (MSC), and GS cell produced from reproductive cell (e.g., testis) are also encompassed in the pluripotent stem cell. Human embryonic stem cells were established from human embryos within 14 days of fertilization.

Embryonic stem cell was first established in 1981, and has also been applied to the generation of knockout mouse since 1989. In 1998, human embryonic stem cell was established, which is also being utilized for regenerative medicine. ES cell can be produced by culturing an inner cell population on a feeder cell or in a medium containing leukemia inhibitory factor (LIF). The production methods of ES cell are described in, for example, WO 96/22362, WO 02/101057, US Patent No. 5843780, US Patent No. 6200806, US Patent No. 6280718, and the like. Embryonic stem cells are available from given organizations, or a commercially available product can be purchased. For example, human embryonic stem cells, KhES-1, KhES-2 and KhES-3, are available from Kyoto University's Institute for Frontier Medical Sciences.

EG cell can be produced by culturing a primordial germ cell in a medium containing mouse stem cell factor (mSCF), LIF, and basic fibroblast growth factor (bFGF) (Cell, 70: 841-847, 1992) .

The "induced pluripotent stem cell" is a cell induced to have pluripotency by reprogramming a somatic cell by a known method and the like. Specifically, as the induced pluripotent stem cell, a cell induced to have pluripotency by reprogramming differentiated somatic cells such as fibroblast, and peripheral blood mononuclear cell by the expression of a plurality of genes selected from the group consisting of reprogramming genes including Oct3/4, Sox2, Klf4, Myc (c-Myc, N-Myc, L-Myc), Glis1, Nanog, Sall4, lin28, and Esrrb can be mentioned. Induced pluripotent stem cell was established by Yamanaka et al. in mouse cell in 2006 (Cell, 2006, 126(4), pp.663-676). In 2007, Induced pluripotent stem cell was also established from human fibroblast, and has pluripotency and self-renewal competence similar to those of embryonic stem cells (Cell, 2007, 131(5), pp.861-872; Science, 2007, 318(5858), pp.1917-1920; Nat. Biotechnol., 2008, 26(1), pp.101-106). Besides the production method based on direct reprogramming by gene expression, induced pluripotent stem cell can also be obtained from somatic cell by the addition of a compound and the like (Science, 2013, 341, pp. 651-654).

While the somatic cell used for producing induced pluripotent stem cell is not particularly limited, tissue-derived fibroblast, blood-lineage cells (e.g., peripheral blood mononuclear cell, T cell), hepatocyte, pancreatic cell, intestinal epithelial cell, and smooth muscle cell can be mentioned.

When induced pluripotent stem cell is produced by reprogramming by the expression of several kinds of genes (e.g., 4 factors of Oct3/4, Sox2, Klf4, and Myc), the means for gene expression is not particularly limited. Examples of the aforementioned means for gene expression include an infection method using a virus vector (e.g., retrovirus vector, lentivirus vector, Sendaivirus vector, adenovirus vector, adeno-associated virus vector), a gene transfer method using a plasmid vector (e.g., plasmid vector, episomal vector) (e.g., calcium phosphate method, lipofection method, retronectin method, electroporation method), a gene transfer method using an RNA vector (e.g., calcium phosphate method, lipofection method, electroporation method), and a method with direct injection of protein.

It is also possible to obtain established induced pluripotent stem cells. For example, human induced pluripotent cell lines established in Kyoto University, such as 201B7 cell, 201B7-Ff cell, 253G1 cell, 253G4 cell, 1201C1 cell, 1205D1 cell, 1210B2 cell, 1231A3 cell, and the like, are available from Kyoto University and iPS Academia Japan, Inc. As induced pluripotent stem cells strain, for example, Ff-I01 cell, Ff-I14 cell, and QHJI01s04 cell, established in Kyoto University, are available from Kyoto University.

The pluripotent stem cells may be genetically modified. Genetically-modified pluripotent stem cells can be produced by using, for example, a homologous recombination technique. Examples of the gene on the chromosome to be modified include a cell marker gene, a histocompatibility antigen gene, a gene related to a disease due to a disorder of neural cell and so on. A target gene on the chromosome can be modified using the methods described in Manipulating the Mouse Embryo, A Laboratory Manual, Second Edition, Cold Spring Harbor Laboratory Press (1994); Gene Targeting, A Practical Approach, IRL Press at Oxford University Press (1993); Biomanual Series 8, Gene Targeting, Making of Mutant Mouse using ES cell, YODOSHA CO., LTD. (1995); and so on.

To be specific, for example, the genome gene of the target gene to be modified (e.g., cell marker gene, histocompatibility antigen gene, disease-related gene and so on) is isolated, and a targeting vector used for homologous recombination of the target gene is produced using the isolated genome gene. The produced targeting vector is introduced into stem cells and the cells that showed homologous recombination between the target gene and the targeting vector are selected, whereby stem cells having the modified gene on the chromosome can be produced.

Examples of the method for isolating genome gene of the target gene include known methods described in Molecular Cloning, A Laboratory Manual, Second Edition, Cold Spring Harbor Laboratory Press (1989), Current Protocols in Molecular Biology, John Wiley & Sons (1987-1997) and so on. Genomic DNA library screening system (manufactured by Genome Systems), Universal GenomeWalker Kits (manufactured by CLONTECH) and so on can be used.

Production of targeting vector used for homologous recombination of the target gene, and efficient selection of a homologous recombinant can be performed according to the methods described in Gene Targeting, A Practical Approach, IRL Press at Oxford University Press (1993); Biomanual Series 8, Gene Targeting, Making of Mutant Mouse using ES cell, YODOSHA CO., LTD. (1995); and so on. As the targeting vector, any of replacement type or insertion type can be used. As the selection method, methods such as positive selection, promoter selection, negative selection, polyA selection and so on can be used. Examples of a method for selecting the desired homologous recombinant from the selected cell lines include Southern hybridization method, PCR method and so on for the genomic DNA.

As the pluripotent stem cells, pluripotent stem cells that have undergone genome editing can also be used as pluripotent stem cells. The "genome editing" is a technique that intentionally modifies a target gene or genomic region by using principles such as site-specific cleavage of genomic DNA strands using nucleases or chemical conversion of bases. Site-specific nuclease includes zinc finger nuclease (ZFN), TALEN, CRISPR/Cas9, and the like. By using genome editing technique, knockout cell line in which a specific gene is deleted, knock-in cell line in which another sequence is artificially inserted into a specific gene locus, and the like can be produced.

Disease-specific pluripotent stem cells may be used as pluripotent stem cells. The "disease-specific pluripotent stem cell" refers to a pluripotent stem cell that has gene mutation or genetic background involved in the onset of disease. Disease-specific pluripotent stem cells can be produced by a method of establishing induced pluripotent stem cells from patients with the target disease or their close relatives by the aforementioned method and the like, or by a method of modifying the genome of already established pluripotent stem cells by using genome editing techniques such as zinc finger nuclease (ZFN), TALEN, and CRISPR.

The "mammal" encompasses rodents, ungulata, carnivora, lagomorpha, primates, etc. The rodents encompass mouse, rat, hamster, guinea pig, etc. Ungulata encompass swine, bovine, goat, horse, sheep, etc. Carnivora encompasses dog, cat, etc. Lagomorpha encompass rabbit and the like. The "primates" in the present invention refers to mammals belonging to the primate, and the primates include prosimian such as lemur, loris, and tupai, and anthropoidea such as monkey, ape, and human.

The pluripotent stem cells to be used in the present invention are mammalian pluripotent stem cells, preferably pluripotent stem cells of rodents (e.g., mouse, rat) or primates (e.g., human, monkey), most preferably a human pluripotent stem cell.

The "suspension culturing" refers to culturing while maintaining a state in which cells are suspended in a culture medium. That is, the suspension culturing is performed under conditions in which cells are not adhered to a culture vessel or feeder cells or the like on the culture vessel (hereinafter denoted as "culture vessel, etc.") and is distinguished from culturing performed under conditions in which cells are adhered to a culture vessel and the like (adhesion culturing). More particularly, suspension culturing refers to culturing under conditions in which a strong cell-substrate junction is not formed between a cell and a culture vessel and the like. Those of ordinary skill in the art can easily determine whether the cells being cultured are in a suspension culture state or in an adhesion culturing by, for example, shaking the culture vessel during microscopic observation.

In a cell aggregate in suspension culturing, a plane attachment is formed between a cell and a cell. In a cell aggregate in suspension culturing, a strong cell-substrate junction is not formed between the cell and the culture vessel, etc. and a cell-substrate junction is hardly formed and, even if it is formed, its contribution is small. An inherent cell-substrate junction may be present inside the cell aggregates during suspension culturing. The "plane attachment between a cell and a cell" means that a cell attaches to another cell via planes. More particularly, the "plane attachment between a cell and a cell" means that, for example, not less than 1%, preferably not less than 3%, more preferably not less than 5%, of the surface area of a cell adheres to the surface of another cell. A surface of a cell can be observed by methods such as staining with a reagent (e.g., DiI) that stains membranes, immunostaining with cell adhesion factors (e.g., E-cadherin, N-cadherin, etc.), and the like.

The culture vessel to be used when performing suspension culturing is not particularly limited as long as it enables "culturing in suspension" and those of ordinary skill in the art can appropriately determine same. Examples of such culture vessel include flask, tissue culture flask, dish, petri dish, tissue culture dish, multidish, microplate, microwell plate, micropore, multiplate, multiwell plate, chamber slide, schale, tube, tray, culture bag, spinner flask, and roller bottle. To enable suspension culturing, these culture vessels are preferably non-cell-adhesive. As the cell non-adhesive culture vessel, a culture vessel that has not been processed by artificial treatment as described above for the purpose of improving adhesion to cells, or the like can be used. As a non-cell-adhesive culture vessel, culture vessels whose surfaces have been artificially treated to decrease adhesiveness to the cells can also be used. The culture surface of the culture vessel may be a flat bottom, U-bottom, or V-bottom, or may have concaves and convexes. Examples of the treatment to decrease adhesiveness to the cells include superhydrophilic treatment by coating with 2-methacryloyloxyethyl phosphorylcholine (MPC) polymer, Poly (2-hydroxyethyl methacrylate) (Poly-HEMA), polyethylene glycol (PEG), and the like, and protein low adsorption treatment, and the like.

The medium to be used for culturing cells can be prepared from a medium generally used for culturing animal cells as a basal medium. Examples of the basal medium include Basal Medium Eagle (BME), BGJb medium, CMRL 1066 medium, Glasgow Minimum Essential Medium (Glasgow MEM), Improved MEM Zinc Option, Iscove's Modified Dulbecco's Medium (IMDM), Medium 199, Eagle Minimum Essential Medium (Eagle MEM), Alpha Modified Eagle Minimum Essential Medium (αMEM), Dulbecco's Modified Eagle Medium (DMEM), F-12 medium, DMEM/F12, IMDM/F12, Ham's medium, RPMI 1640, Fischer's medium, mixed media of these, and the like.

For culturing pluripotent stem cells, a medium for culturing pluripotent stem cells using the above-mentioned basal medium as the base, preferably a known medium for embryonic stem cells and/or induced pluripotent stem cells, a medium for culturing pluripotent stem cells under feeder free (feeder-free medium) and the like can be used. As feeder-free medium, many synthetic media have been developed and are commercially available and, for example, Essential 8 medium can be mentioned. Essential 8 medium is obtained by supplementing DMEM/F12 medium with L-ascorbic acid-2-phosphate magnesium (64 mg/l), sodium selenium (14 µg/1), insulin (19.4 mg/l), NaHCO₃ (543 mg/l), transferrin (10.7 mg/l), bFGF (100 ng/mL), and TGFβ family signal transduction pathway activating substance (TGFβ1 (2 ng/mL) or Nodal (100 ng/mL)) as additives (Nature Methods, 8, 424-429 (2011)). Examples of the commercially available feeder-free medium include Essential 8 (manufactured by Thermo Fisher Scientific), S-medium (manufactured by DS Pharma Biomedical Co., Ltd.), StemPro (manufactured by Thermo Fisher Scientific), hESF9, mTeSR1 (manufactured by STEMCELL Technologies), mTeSR2 (manufactured by STEMCELL Technologies), TeSR-E8 (manufactured by STEMCELL Technologies), mTeSR Plus (manufactured by STEMCELL Technologies), StemFit (manufactured by Ajinomoto Co., Inc.), ReproMed iPSC Medium (manufactured by REPROCELL), NutriStem XF (manufactured by Biological Industries), NutriStem V9 (manufactured by Biological Industries), Cellartis DEF-CS Xeno-Free Culture medium (manufactured by Takara Bio Inc.), Stem-Partner SF (manufactured by KYOKUTO PHARMACEUTICAL INDUSTRIAL CO., LTD), PluriSTEM Human ES/iPS Cell Medium (manufactured by Merck), StemSure hPSC MediumΔ (manufactured by FUJIFILM Wako Pure Chemical Corporation), and the like.

The serum-free medium may contain a serum replacement. Examples of the serum replacement include one appropriately containing albumin, transferrin, fatty acid, collagen precursor, trace element, 2-mercaptoethanol or 3' thiolglycerol, or equivalents of these, and so on. Such serum replacement may be prepared by, for example, the method described in WO 98/30679. The serum replacement may be a commercially available product. Examples of such commercially available serum replacement include Knockout Serum Replacement (manufactured by Thermo Fisher Scientific) (hereinafter sometimes also to be denoted as "KSR"), Chemically-defined Lipid concentrated (manufactured by Thermo Fisher Scientific), Glutamax (manufactured by Thermo Fisher Scientific), B27 Supplement (manufactured by Thermo Fisher Scientific), N2 Supplement (manufactured by Thermo Fisher Scientific) and the like.

The serum-free medium to be used for suspension culturing and adhesion culturing may appropriately contain a fatty acid or lipid, amino acid (e.g., non-essential amino acids), vitamin, growth factor, cytokine, antioxidant, 2-mercaptoethanol, pyruvic acid, buffering agent, inorganic salts and so on.

To avoid complicated preparation, a serum-free medium supplemented with an appropriate amount (e.g., about 0.5% to about 30%, preferably about 1% to about 20%) of commercially available KSR (manufactured by Thermo Fisher Scientific) (e.g., medium of 1:1 mixture of F-12 medium and IMDM medium supplemented with 1×chemically-defined Lipid concentrated, 5% KSR, and 450 µM 1-monothioglycerol) may be used as such serum-free medium. In addition, as a product equivalent to KSR, the medium disclosed in JP-A-2001-508302 can be mentioned.

The "serum-containing medium" means a medium containing unadjusted or unpurified serum. The medium may contain a fatty acid, lipid, amino acid (e.g., non-essential amino acids), vitamin, growth factor, cytokine, antioxidant, 2-mercaptoethanol, 1-monothioglycerol, pyruvic acid, buffering agent, inorganic salts and so on.

The culturing in the present invention is preferably performed under xeno-free conditions. The "xeno-free" means conditions eliminating components derived from species different from that of the cell to be cultured.

The medium to be used in the present invention is preferably a medium containing chemically determined components (Chemically defined medium; CDM) to avoid contamination with chemically undetermined components.

In the present specification, "in the absence of a substance X" refers to a medium not supplemented with an exogenous substance X or a medium not containing an exogenous substance X, or a state in which an exogenous substance X is not present.

In the present specification, the "derivative" refers to a group of compounds produced by substituting a part of the molecule of a specific compound with another functional group or other atom. In the present specification, a "variant" of a protein refers to a protein in which mutations such as deletion, addition, or substitution of amino acid residues have been made to the extent that the properties of the original protein can be maintained. The number of mutated amino acids is not particularly limited, and may be, for example, 1 to 4, 1 to 3, 1 or 2, or 1. A "variant" of a protein may be a protein having an amino acid sequence showing an identity of at least not less than 90%, not less than 91%, not less than 92%, not less than 93%, not less than 94%, not less than 95%, not less than 96%, not less than 97%, not less than 98%, not less than 99%, or not less than 99.5%, with that of the original protein.

In the present specification, "after A hour (day A)" includes the A hour (day A) and refers to time and day after the A hour (day A). "Within B hour (day B)" includes the B hour (day B) and refers to time and day before B hour (day B).

The "feeder cell" refers to a cell other than a stem cell that co-exists when culturing the stem cell. Examples of the feeder cells used for culturing pluripotent stem cells while maintaining an undifferentiated state include mouse fibroblasts (MEF), human fibroblasts, and SNL cells. As the feeder cells, feeder cells that underwent a growth suppression treatment is preferable. Examples of the growth suppression treatment include treatment with a growth inhibitor (e.g., mitomycin C), and UV irradiation. Feeder cells used for culturing pluripotent stem cells while maintaining an undifferentiated state contributes to the maintenance of undifferentiated state of pluripotent stem cell by secretion of humoral factors (preferably factor for maintaining an undifferentiated state), or production of scaffolds for cell adhesion (extracellular matrix).

In the present specification, the absence of feeder cells (feeder-free) means culturing in the absence of a feeder cell. The absence of feeder cells means, for example, a condition without addition of a feeder cell or a condition substantially free of feeder cells (e.g., the ratio of the number of feeder cells relative to the total number of cells is not more than 3%).

The "cell aggregate" refers to a clump formed by assembly of cells, wherein the cells are adhered to each other. Cell populations, embryoid bodies, spheres, spheroids, and organoids are also encompassed in the cell aggregates. In cell aggregates, a plane attachment is preferably formed between a cell and a cell. In some embodiments, cells adhere to each other to form, for example, adhesionic junction (adherence junction) in some or all of the cell aggregates. In some embodiments, two or more cell aggregates can also be further artificially adhered or aggregated. Cell aggregates also include clusters in which cell populations are further adhered or aggregated to each other, and assembleoids.

The "uniformed cell aggregates" means that the size of each cell aggregate is constant when a plurality of cell aggregates are cultured, and that the variance in the length of the maximum diameter is small when the size of the cell aggregates are evaluated by the length of the maximum diameter. More specifically, it means that not less than 75% of a plurality of cell aggregates are within mean ± 100%, preferably mean ± 50%, more preferably mean ± 20%, of the mean maximum diameter in the plurality of cell aggregates.

A "cell population" refers to a cell group consisting of two or more cells. A cell population may be composed of one type of cell, or may be composed of multiple types of cells. The cells constituting the cell population may be suspended in a medium or may be attached to a culture vessel or the like. The cells constituting a cell population may be single cells, or the cells may adhere to each other to form a cell population in at least a portion of the cell population. As used herein, for example, the "single cell" refers to a cell almost free of mutual adhesion of cells (e.g., plane attachment). In some embodiments, being dispersed into single cells refers to a state almost free of cell-cell junction (e.g., adhesive junction). A cell population may contain cell aggregates.

The "tissue" refers to the structure of a cell population that has a structure in which multiple types of cells with different morphologies and properties are three-dimensionally configured in a certain pattern.

In the present specification, cell aggregates, cell populations, and tissues are cell aggregates, cell populations, and tissues of mammals, preferably cell aggregates, cell populations, and tissues of rodents (e.g., mouse, rat) or primates (e.g., human, monkey), most preferably cell aggregates, cell populations, and tissues of humans.

The present invention provides a method for producing a cell aggregate containing pituitary hormone-producing cells (the method of the present invention), which includes the following steps:
(1) a step of separating a cell expressing EpCAM from a dispersed cell population derived from a pluripotent stem cell and including a hypothalamic cell, a pituitary progenitor cell, and a pituitary hormone-producing cell, and
(2) a step of suspension culturing the separated EpCAM-expressing cell for not less than 14 days under conditions that allow the pituitary progenitor cell to mature into a pituitary hormone secretory cell.

The method of the present invention includes a step of separating a cell expressing EpCAM from a dispersed cell population derived from a pluripotent stem cell and including a hypothalamic cell, a pituitary progenitor cell, and a pituitary hormone-producing cell (step (1) of the present invention).

The dispersed cell population derived from a pluripotent stem cell and including a hypothalamic cell, a pituitary progenitor cell, and a pituitary hormone-producing cell in step (1) of the present invention may be a dispersed cell population derived from a living organism and including a hypothalamic cell, a pituitary progenitor cell, and a pituitary hormone-producing cell, or a dispersed cell population derived from a pluripotent stem cell and including a hypothalamic cell, a pituitary progenitor cell, and a pituitary hormone-producing cell, but the latter is preferred. The dispersed cell population derived from a pluripotent stem cell and including a hypothalamic cell, a pituitary progenitor cell, and a pituitary hormone-producing cell is obtained, for example, by the following steps (A) and (B).
(A) a step of culturing a pluripotent stem cell or a cell aggregate thereof under conditions that can induce differentiation into a cell aggregate containing a hypothalamic cell, a pituitary progenitor cell, and a pituitary hormone-producing cell.
(B) a step of dispersing the cell aggregate containing a hypothalamic cell, a pituitary progenitor cell, and a pituitary hormone-producing cell, obtained in (A), into a cell population.

The cell aggregate containing a hypothalamic cell, a pituitary progenitor cell, and a pituitary hormone-producing cell that is induced to differentiate in step (A), for example, can be classified based on the differentiation stage of pituitary tissue containing a pituitary progenitor cell and/or a pituitary hormone-producing cell. The hypothalamic tissue containing hypothalamic cell and the pituitary tissue influence differentiation of each other, and therefore, the differentiation stage of the pituitary tissue and the differentiation stage of the hypothalamic tissue are correlated. While multiple classifications are possible, in the present specification, the differentiation stage from pluripotent stem cells to pituitary tissue is classified into two differentiation stages of pituitary precursor tissue, which contains only a pituitary progenitor cell, and pituitary tissue containing a pituitary progenitor cell and a pituitary hormone-producing cell, the former is further classified into early stage and late stage, and the latter is classified into early stage, middle stage, and late stage. These differentiation stages are explained in the following.

In the present specification, the "pituitary precursor tissue" refers to a tissue in which pituitary progenitor cells exist, but pituitary hormone-producing cells do not exist. On the other hand, in the present specification, the "pituitary tissue" refers to a tissue that contains at least one type of pituitary hormone-producing cell (e.g., ACTH-producing cell), and may contain a pituitary precursor tissue.

The pituitary precursor tissue can be further classified into early stage (referred to as early stage pituitary precursor tissue) and late stage (referred to as late stage pituitary precursor tissue). In the early stage pituitary precursor tissue, the expression of the aforementioned pituitary progenitor cell marker (e.g., Lhx3 or Pitx1) begins to be induced (detected), and in the late stage pituitary precursor tissue, a continuous increase in these expressions is observed. In one embodiment, the tissue at a stage where the early stage pituitary precursor tissue or the tissue wherein an expression of pituitary progenitor cell markers (e.g., Lhx3 or Pitx1) begins to be induced (detected) includes a tissue in which the proportion of the Lhx3 positive cell or Pitx1 positive cell with respect to the total number of cells is less than 3%. On the other hand, as the late stage pituitary precursor tissue, a tissue in which the proportion of cells positive for at least one of Lhx3 and Pitx1 with respect to the total number of cells is not less than 3% can be mentioned. Those of ordinary skill in the art can measure the proportion by a known method. For example, it can be measured using antibody against Lhx3 or Pitx1 and according to methods such as immunostaining method and flow cytometry.

In the differentiation stage where pituitary precursor tissue is induced, hypothalamic tissue is induced. In this case, it is a stage where the expression of the aforementioned hypothalamic marker (e.g., Rx or Nkx2.1) begins to be induced in the hypothalamic tissue in the cell aggregate containing the early stage pituitary precursor tissue and hypothalamic tissue. On the other hand, a continuous increase in their expression is observed in the hypothalamic tissue in a cell aggregate containing the late stage pituitary precursor tissue and hypothalamic tissue. Therefore, as one embodiment, in a cell aggregate containing pituitary precursor tissue and hypothalamic tissue in the same aggregate, a cell aggregate containing early stage pituitary precursor tissue is, for example, a cell aggregate containing a tissue in which the expression of Nkx2.1 has not reached a peak, and a cell aggregate containing late stage pituitary precursor tissue is, for example, a cell aggregate in which the expression of Nkx2.1 is not less than 3%, not less than 10%, preferably not less than 20%.

Pituitary tissues can be classified into early stage, middle stage, and late stage. In a pituitary tissue, the expression of pituitary progenitor cell markers reaches a peak, and the expression thereof is maintained almost constant regardless of the early stage, middle stage, or late stage.

In the present specification, the early stage pituitary tissue is defined to be a tissue at a stage where induction of pituitary hormone-producing cells begins, to a stage where only one or two types of pituitary hormone-producing cells (e.g., adrenocorticotropic hormone (ACTH)-producing cells and/or prolactin (PRL)-producing cells) exist as pituitary hormone-producing cells.

In the present specification, the middle stage pituitary tissue is defined to be a tissue in the stage where 3 to 4 types of pituitary hormone-producing cells (e.g., including ACTH-producing cells and PRL-producing cells, and further including one or two types of cells selected from the group consisting of luteinizing hormone (LH)-producing cells and follicle-stimulating hormone (FSH)-producing cells) are present.

In the present specification, the late stage pituitary tissue is defined to be a tissue in the stage where not less than five types of pituitary hormone-producing cells (e.g., cells such as ACTH-producing cell, PRL-producing cell, LH-producing cell, and FSH-producing cell, as well as growth hormone (GH)-producing cell or thyroid-stimulating hormone (TSH)-producing cell and the like) are present.

In the present specification, a continuous increase in ACTH secretory capacity is observed in the pituitary tissue during the early stage, middle stage, and late stage. In one embodiment, the ACTH secretory capacity in a cell aggregate containing pituitary tissue is not less than 0.5 pg/mL in the early stage pituitary tissue, not less than 2 pg/mL in the middle stage pituitary tissue, and not less than 20 pg/mL in the late stage pituitary tissue. In one embodiment, ACTH secretory capacity in the cell aggregate containing pituitary tissue is less than 2 pg/mL in the early stage pituitary tissue, and less than 20 pg/mL in the middle stage pituitary tissue.

In the hypothalamic tissue in a cell aggregate containing pituitary tissue and hypothalamic tissue, the expression of Rx reaches a peak in the hypothalamic tissue in the cell aggregate containing early stage pituitary tissue and then decreases. Almost no expression of Rx is observed in the hypothalamic tissue in a cell aggregate containing tissues after middle stage pituitary tissue. On the other hand, the expression of Nkx2.1 continues to decrease in the hypothalamic tissue of cell aggregates containing pituitary tissue. In one embodiment, in hypothalamic tissue in a cell aggregate containing late stage pituitary tissue, the expression rate of Nkx2.1 is not more than 10%, not more than 5%, not more than 4%, not more than 3%, not more than 2%, or not more than 1%. In one embodiment, in hypothalamic tissue in a cell aggregate containing late stage pituitary tissue, the expression rate of Nkx2.1 may be not less than 1%, not less than 2%, not less than 3%, not less than 4%, or not less than 5%.

In one embodiment, the cell aggregate containing middle stage pituitary tissue and hypothalamic tissue is a cell aggregate in which 3 to 4 types of pituitary hormone-producing cells are present and Nkx2.1-expressing cells are present in the hypothalamic tissue. The proportion of Nkx2.1-expressing cells to the total cells in the cell aggregate may be, for example, more than 2%, not less than 5%, not less than 10%, or not less than 20%. The proportion of Nkx2.1-expressing cells to the total cells in the cell aggregate may be, for example, not more than 30%, not more than 20%, or not more than 10%. In another embodiment, the cell aggregate containing middle stage pituitary tissue and hypothalamic tissue is a cell aggregate in which 3 to 4 types of pituitary hormone-producing cells are present and Nkx2.1-expressing cells are not substantially present in the hypothalamic tissue.

In one embodiment, the cell aggregate containing late stage pituitary tissue and hypothalamic tissue is a cell aggregate in which not less than 5 types of pituitary hormone-producing cells are present and Rx-expressing cells and Nkx2.1-expressing cells are not substantially present.

That "a specific cell does not substantially present" means that the proportion of the specific cells to the total cells in the cell aggregate is not more than 2% or not more than 1%.

By confirming the time course of expression of these markers, those of ordinary skill in the art can link these stages to the specific number of differentiation days in a specific differentiation induction method. For example, in the below-mentioned differentiation induction method, cell aggregates around 12 to 30 days after the start of differentiation contain late stage pituitary precursor tissue, cell aggregates around 30 to 60 days after the start of differentiation contain early stage pituitary tissue, and cell aggregates around 60 to 100 days after the start of differentiation contain middle stage pituitary tissue.

One embodiment of the cell aggregate derived from a pluripotent stem cell and containing a hypothalamic cell, a pituitary progenitor cell, and a pituitary hormone-producing cell, obtained in step (A), is, for example, a cell aggregate (cell aggregate around 30 to 100 days after the start of differentiation) containing hypothalamic tissue (optionally containing Nkx2.1-expressing cell) and pituitary tissue containing not less than one and not more than 4 types of pituitary hormone-producing cells.

One embodiment of the cell aggregate derived from a pluripotent stem cell and containing a hypothalamic cell, a pituitary progenitor cell, and a pituitary hormone-producing cell, obtained in step (A), is, for example, a cell aggregate (cell aggregate around 30 to 60 days after the start of differentiation) containing hypothalamic tissue (optionally containing Nkx2.1-expressing cell) and the aforementioned pituitary tissue.

One embodiment of the cell aggregate derived from a pluripotent stem cell and containing a hypothalamic cell, a pituitary progenitor cell, and a pituitary hormone-producing cell, obtained in step (A), is, for example, a cell aggregate (cell aggregate around 60 to 100 days after the start of differentiation) containing hypothalamic tissue (optionally containing Nkx2.1-expressing cell) and middle stage pituitary tissue.

The cell aggregate containing a hypothalamic cell, a pituitary progenitor cell, and a pituitary hormone-producing cell obtained in step (A) can be obtained by culturing pluripotent stem cells under conditions that can induce differentiation into a cell aggregate containing a hypothalamic cell, a pituitary progenitor cell, and a pituitary hormone-producing cell.

Examples of the method for differentiating pluripotent stem cells into an aggregate containing a hypothalamic cell, a pituitary progenitor cell, and a pituitary hormone-producing cell include the method described in WO 2019/103129. It can also be performed, for example, according to the following steps.

One embodiment of the method for differentiating pluripotent stem cells into an aggregate containing a hypothalamic cell, a pituitary progenitor cell, and a pituitary hormone-producing cell is a method for producing a cell population containing pituitary tissue, including the following steps (1') and (2) (the production method of the present invention):
(1') a first step of culturing pluripotent stem cells in the presence of a Wnt signal transduction pathway inhibiting substance to form a cell population,
(2) a second step of culturing the cell population obtained in the first step in the presence of a BMP signal transduction pathway activating substance and a Sonic hedgehog (Shh) signal transduction pathway activating substance, thereby obtaining a cell population comprising pituitary tissue.

Preferably, the first step is a step of forming a cell aggregate, and the cell population obtained in the first step and subjected to the second step may be a cell aggregate.

In step (1'), preferably, a JNK signal transduction pathway inhibiting substance is used in combination with a Wnt signal transduction pathway inhibiting substance.

Depending on the length of culture period, the cell population containing pituitary tissue obtained in step (2) can become a cell aggregate containing a hypothalamic cell, a pituitary progenitor cell, and a pituitary hormone-producing cell.

A more preferred embodiment of the production method of the present invention is a method for producing a cell population containing pituitary tissue, including the following step (a) and the following steps (1') and (2):
(a) step a of culturing pluripotent stem cells in the absence of feeder cells and in a medium comprising 1) a TGFβ family signal transduction pathway inhibiting substance and/or a Shh signal transduction pathway activating substance, and 2) a factor for maintaining an undifferentiated state,

(1') a first step of culturing (preferably suspension-culturing) the pluripotent stem cells obtained in step a, in the presence of a Wnt signal transduction pathway inhibiting substance to form a cell population,
(2) a second step of culturing (preferably suspension-culturing) the cell population obtained in the first step in the presence of a BMP signal transduction pathway activating substance and a Shh signal transduction pathway activating substance, thereby obtaining a cell population comprising pituitary tissue.

Preferably, the first step is a step of forming a cell aggregate, and the cell population obtained in the first step and subjected to the second step may be a cell aggregate.

In step (1'), preferably, a JNK signal transduction pathway inhibiting substance is used in combination with a Wnt signal transduction pathway inhibiting substance.

Depending on the length of culture period, the cell population comprising pituitary tissue obtained in step (2) can become a cell aggregate containing a hypothalamic cell, a pituitary progenitor cell, and a pituitary hormone-producing cell.

A still more preferred embodiment of the production method of the present invention is a method for producing a cell population containing pituitary tissue, including the following step (a) and the following steps (1) and (2):
(a) step a of culturing pluripotent stem cells in the absence of feeder cells and in a medium comprising 1) a TGFβ family signal transduction pathway inhibiting substance and/or a Shh signal transduction pathway activating substance, and 2) a factor for maintaining an undifferentiated state,

(1) a first step of culturing (preferably suspension-culturing) the pluripotent stem cells obtained in step a, in the presence of a JNK signal transduction pathway inhibiting substance and a Wnt signal transduction pathway inhibiting substance to form a cell population,
(2) a second step of culturing (preferably suspension-culturing) the cell population obtained in the first step in the presence of a BMP signal transduction pathway activating substance and a Shh signal transduction pathway activating substance, thereby obtaining a cell population comprising pituitary tissue.

Preferably, the first step is a step of forming a cell aggregate, and the cell population obtained in the first step and subjected to the second step may be a cell aggregate.

Depending on the length of culture period, the cell population comprising pituitary tissue obtained in step (2) can become a cell aggregate containing a hypothalamic cell, a pituitary progenitor cell, and a pituitary hormone-producing cell.

The production method described above may further include the following third step :
(3) a third step of culturing (preferably suspension-culturing) the cell population obtained in second step in the absence of a Shh signal transduction pathway activating substance to obtain a cell population comprising pituitary tissue.

### <Step (a)>: step a

Step a of culturing pluripotent stem cells in the absence of feeder cells and in a medium comprising 1) a TGFβ family signal transduction pathway inhibiting substance and/or a Shh signal transduction pathway activating substance, and 2) a factor for maintaining an undifferentiated state is explained.

In step (a), the pluripotent stem cells are treated with a TGFβ family signal transduction pathway inhibiting substance and/or a Shh signal transduction pathway activating substance, and then subjected to culture (preferably suspension-culturing) in the first step. As a result, the state of pluripotent stem cells changes, the efficiency of forming non-neural epithelial tissue is improved, the quality of the resulting cell population (aggregate) is improved, differentiation becomes easier, cell death is less likely to occur, and the production efficiency of pituitary cells is improved.

Step (a) is performed in the absence of feeder cells.

The absence of feeder cells (feeder-free) in the present invention means a condition substantially free of feeder cells (e.g., the percentage of the number of feeder cells relative to the total number of cells is not more than 3%).

In the production method of the pituitary in the present invention, pluripotent stem cell is preferably an embryonic stem cell or an induced pluripotent stem cell. Induced pluripotent stem cell is available from given organizations, or a commercially available product can be purchased. For example, human induced pluripotent stem cell line 201B7 strain, 201B7-Ff cell, 253G1 cell, 253G4 cell, 1201C1 cell, 1205D1 cell, 1210B2 cell, and 1231A3 cell are available from Kyoto University and iPS Academia Japan, Inc. As induced pluripotent stem cell lines, for example, Ff-I01 cell, Ff-I14 cell, and QHJI01s04 cell, established in Kyoto University, are available from Kyoto University. Also, HC-6 #10 strain, 1231A3 strain, and 1383D2 strain are available from RIKEN.

The TGFβ family signal transduction pathway (i.e., TGFβ superfamily signal transduction pathway) is a signal transduction pathway intracellularly transduced by Smad family with transformation growth factor β (TGFβ), Nodal/Activin or BMP as a ligand.

The TGFβ family signal transduction pathway inhibiting substance is a substance that inhibits TGFβ family signal transduction pathway, that is, a signal transduction pathway transduced by the Smad family. Specifically, a TGFβ signal transduction pathway inhibiting substance, a Nodal/Activin signal transduction pathway inhibiting substance and a BMP signal transduction pathway inhibiting substance can be mentioned. As the TGFβ family signal transduction pathway inhibiting substance, a TGFβ signal transduction pathway inhibiting substance is preferable.

The TGFβ signal transduction pathway inhibiting substance is not particularly limited as long as it is a substance inhibiting a signal transduction pathway caused by TGFβ, and may be any of nucleic acid, protein and low-molecular organic compound. As the substance, for example, a substance directly acting on TGFβ (e.g., protein, antibody, and aptamer), a substance suppressing expression of gene encoding TGFβ (e.g., antisense oligonucleotide, and siRNA), a substance that inhibits the binding of TGFβ receptor and TGFβ, and a substance that inhibits physiological activity caused by signal transduction by the TGFβ receptor (e.g., TGFβ receptor inhibitor, and Smad inhibitor) can be mentioned. As a protein known as a TGFβ signal transduction pathway inhibiting substance, Lefty can be mentioned.

As a TGFβ signal transduction pathway inhibiting substance, compounds well known to those of ordinary skill in the art can be used. Specifically, Alk5/TGFβR1 inhibitors such as SB431542 (sometimes to be abbreviated as "SB431") (4-[4-(3,4-Methylenedioxyphenyl)-5-(2-pyridyl)-1H-imidazol-2-yl]benzamide), SB505124 (2-[4-(1,3-Benzodioxol-5-yl)-2-(1,1-dimethylethyl)-1H-imidazol-5-yl]-6-methylpyridine), SB525334 (6-[2-(1,1-Dimethylethyl)-5-(6-methyl-2-pyridinyl)-1H-imidazol-4-yl]quinoxaline), LY2157299 (4-[5,6-Dihydro-2-(6-methyl-2-pyridinyl)-4H-pyrrolo[1,2-b]pyrazol-3-yl]-6-quinolinecarboxamide), LY2109761 (4-[5,6-dihydro-2-(2-pyridinyl)-4H-pyrrolo[1,2-b]pyrazol-3-yl]-7-[2-(4-morpholinyl)ethoxy]-quinoline), GW788388 (4-{4-[3-(Pyridin-2-yl)-1H-pyrazol-4-yl]-pyridin-2-yl}-N-(tetrahydro-2H-pyran-4-yl)benzamide), LY364947 (4-[3-(2-Pyridinyl)-1H-pyrazol-4-yl]quinoline), SD-208 (2-(5-Chloro-2-fluorophenyl)pteridin-4-yl)pyridin-4-ylamine), EW-7197 (N-(2-fluorophenyl)-5-(6-methyl-2-pyridinyl)-4-[1,2,4]triazolo[1,5-a]pyridin-6-yl-1H-Imidazole-2-methanamine), A83-01 (3-(6-Methylpyridin-2-yl)-4-(4-quinolyl)-1-phenylthiocarbamoyl-1H-pyrazole), RepSox (2-[5-(6-Methylpyridin-2-yl)-1H-pyrazol-4-yl]-1,5-naphthyridine), SM16 (4-[4-(1,3-Benzodioxol-5-yl)-5-(6-methyl-2-pyridinyl)-1H-imidazol-2-yl]bicyclo[2.2.2]octane-1-carboxamide), R268712 (4-[2-Fluoro-5-[3-(6-methyl-2-pyridinyl)-1H-pyrazol-4-yl]phenyl]-1H-pyrazole-1-ethanol), IN1130 (3-[[5-(6-Methyl-2-pyridinyl)-4-(6-quinoxalinyl)-1H-imidazol-2-yl]methyl]benzamide), Galunisertib (4-[5,6-Dihydro-2-(6-methyl-2-pyridinyl)-4H-pyrrolo[1,2-b]pyrazol-3-yl]-6-quinolinecarboxamide), AZ12799734 (4-({4-[(2,6-dimethylpyridin-3-yl)oxy]pyridin-2-yl}amino)benzenesulfonamide), A77-01 (4-[3-(6-Methylpyridin-2-yl)-1H-pyrazol-4-yl]quinoline), KRCA 0008 (1,1-[(5-Chloro-2,4-pyrimidinediyl)bis[imino(3-methoxy-4,1-phenylene)-4,1-piperazinediyl]]bisethanone), GSK 1838705 (2-[[2-[[1-[(Dimethylamino)ethanoyl]-5-(methyloxy)-2,3-dihydro-1H-indol-6-yl]amino]-7H-pyrrolo[2,3-d]pyrimidin-4-yl]amino]-6-fluoro-N-methylbenzamide), Crizotinib (3-[(1R)-1-(2,6-Dichloro-3-fluorophenyl)ethoxy]-5-[1-(piperidin-4-yl)-1H-pyrazol-4-yl]-2-aMinopyridine), Ceritinib (5-Chloro-N2-[2-isopropoxy-5-Methyl-4-(4-piperidyl)phenyl]-N4-(2-isopropylsulfonylphenyl)pyriMidine-2,4-diaMine), ASP 3026 (N2-[2-Methoxy-4-[4-(4-methyl-1-piperazinyl)-1-piperidinyl]phenyl]-N4-[2-[(1-methylethyl)sulfon), TAE684 (5-Chloro-N2-[2-methoxy-4-[4-(4-methyl-1-piperazinyl)-1-piperidinyl]phenyl]-N4-[2-[(1-methylethyl)sulfonyl]phenyl]-2,4-pyrimidinediamine), AZD3463 (N-[4-(4-Amino-1-piperidinyl)-2-methoxyphenyl]-5-chloro-4-(1H-indol-3-yl)-2-pyrimidinamine), TP0427736 (6-[4-(4-methyl-1,3-thiazol-2-yl)-1H-imidazol-5-yl]-1,3-benzothiazole), TGFBR1-IN-1 (5-(1,3-benzothiazol-6-yl)-N-(4-hydroxyphenyl)-1-(6-methylpyridin-2-yl)pyrazole-3-carboxamide), TEW-7197 (2-fluoro-N-[[5-(6-methylpyridin-2-yl)-4-([1,2,4]triazolo[1,5-a]pyridin-6-yl)-1H-imidazol-2-yl]methyl]aniline), LY3200882 (2-[4-[[4-[1-cyclopropyl-3-(oxan-4-yl)pyrazol-4-yl]oxypyridin-2-yl]amino]pyridin-2-yl]propan-2-ol), BIBF-0775 ((3Z)-N-Ethyl-2,3-dihydro-N-methyl-2-oxo-3-[phenyl[[4-(1-piperidinylmethyl)phenyl]amino]methylene]-1H-indole-6-carboxamide, and the like, SMAD3 inhibitors such as SIS3 (1-(3,4-dihydro-6,7-dimethoxy-2(1H)-isoquinolinyl)-3-(1-methyl-2-phenyl-1H-pyrrolo[2,3-b]pyridin-3-yl)-2-propen-1-one) and the like, receptor degradation promoters such as ITD-1 (4-[1,1'-Biphenyl]-4-yl-1,4,5,6,7,8-hexahydro-2,7,7-trimethyl-5-oxo-3-quinolinecarboxylic acid ethyl ester) and the like, derivatives of these compounds, and the like can be mentioned. These substances may be used alone or in combination. SB431542 is a compound known as an inhibitor of TGFβ receptor (ALK5) and Activin receptor (ALK4/7) (i.e., TGFβR inhibitor). SIS3 is a TGFβ signal transduction pathway inhibiting substance that inhibits phosphorylation of SMAD3 which is an intracellular signal transduction factor under the control of TGFβ receptor. ITD-1 is a proteasomal degradation promoter of TGF-β type II receptor.

The TGFβ signal transduction pathway inhibiting substance preferably contains an Alk5/TGFβR1 inhibitor. The Alk5/TGFβR1 inhibitor preferably contains at least one selected from the group consisting of SB431542, SB505124, SB525334, LY2157299, GW788388, LY364947, SD-208, EW-7197, A83-01, RepSox, SM16, R268712, IN1130, Galunisertib, AZ12799734, A77-01, KRCA 0008, GSK 1838705, Crizotinib, Ceritinib, ASP 3026, TAE684, AZD3463, and TP0427736, further preferably SB431542 or A83-01.

The concentration of a TGFβ signal transduction pathway inhibiting substance in the medium can be appropriately determined according to the substances used within a range capable of achieving the aforementioned effects. When SB431542 is used as the TGFβ transduction pathway inhibiting substance in step (a), it is generally used at a concentration of about 1 nM to about 100 µM, preferably about 10 nM - about 100 µM, more preferably about 10 nM to about 50 µM, further preferably about 100 nM to about 50 µM, particularly preferably about 1 µM to about 10 µM. When a TGFβ signal transduction pathway inhibiting substance other than SB431542 is used, it is desirably used at a concentration that shows TGFβ signal transduction pathway inhibiting activity equivalent to that of SB431542 at the above-mentioned concentration.

The Shh signal pathway agonist is a substance capable of enhancing signal transduction mediated by Shh. Examples of the Shh signal transduction pathway activating substance include proteins belonging to the Hedgehog family (e.g., Shh, Ihh), Shh receptor, Shh receptor agonist, Smo agonist, Purmorphamine (9-cyclohexyl-N-[4-(morpholinyl)phenyl]-2-(1-naphthalenyloxy)-9H-purin-6-amine), GSA-10 (Propyl 4-(1-hexyl-4-hydroxy-2-oxo-1,2-dihydroquinoline-3-carboxamido)benzoate), Hh-Ag1.5, 20(S)-Hydroxycholesterol, SAG (Smoothened Agonist: N-Methyl-N'-(3-pyridinylbenzyl)-N'-(3-chlorobenzo[b]thiophene-2-carbonyl)-1,4-diaminocyclohexane), 20(S)-hydroxy Cholesterol(3S,8S,9S,10R,13S,14S,17S)-17-[(2R)-2-hydroxy-6-methylheptan-2-yl]-10,13-dimethyl-2,3,4,7,8,9,11,12,14,15,16,17-dodecahydro-1H-cyclopenta[a]phenanthren-3-ol), and the like. These substances may be used alone or in combination.

The Shh signal transduction pathway activating substance preferably contains at least one selected from the group consisting of SAG, Purmorphamine, and GSA-10, more preferably SAG. The concentration of the Shh signal transduction pathway activating substance in the medium can be appropriately determined, according to the substances to be used, to fall within a range capable of achieving the aforementioned effects. In step (a), SAG is generally used at a concentration of about 1 nM to about 2000 nM, preferably about 10 nM to about 1000 nM, more preferably about 10 nM to about 700 nM, further preferably about 50 nM to about 700 nM, particularly preferably about 100 nM to about 600 nM, most preferably about 100 nM to about 500 nM. When a Shh signal transduction pathway activating substance other than SAG is used, it is desirably used at a concentration that shows Shh signal transduction promoting activity equivalent to that of SAG at the aforementioned concentration. Shh signal transduction promoting activity can be determined by a method well known to those of ordinary skill in the art, for example, reporter gene assay focusing on the expression of Gli1 gene (Oncogene (2007) 26, 5163-5168).

To enable culturing to maintain undifferentiated state, the medium used in step (a) contains a factor for maintaining an undifferentiated state. The factor for maintaining an undifferentiated state is not particularly limited as long as it is a substance having an action to suppress differentiation of pluripotent stem cells. Examples of the factor for maintaining an undifferentiated state widely used by those of ordinary skill in the art include an FGF signal transduction pathway activating substance, a TGFβ family signal transduction pathway activating substance, and insulin in the case of primed pluripotent stem cells (e.g., human ES cells, human iPS cells). As the FGF signal transduction pathway activating substance, fibroblast growth factors (e.g., bFGF, FGF4, FGF8) can be specifically mentioned. As the TGFβ family signal transduction pathway activating substance, a TGFβ signal transduction pathway activating substance, a Nodal/Activin signal transduction pathway activating substance can be mentioned. As the TGFβ signal transduction pathway activating substance, for example, TGFβ1, TGFβ2 can be mentioned. As the Nodal/Activin signal transduction pathway activating substance, for example, Nodal, Activin A, Activin B can be mentioned. These substances may be used alone or in combination. When human pluripotent stem cells (e.g., human ES cells, human iPS cells) are cultured, the medium in step (a) preferably contains bFGF as a factor for maintaining an undifferentiated state.

The factor for maintaining an undifferentiated state to be used is generally a factor for maintaining an undifferentiated state of mammals. Since the factor for maintaining an undifferentiated state may have cross-reactivity among mammal species, a factor for maintaining an undifferentiated state of any mammal may also be used as long as the undifferentiated state of the pluripotent stem cells to be cultured can be maintained. Preferably, the factor for maintaining an undifferentiated state is a factor for maintaining an undifferentiated state of a mammal of the same species as the cells to be cultured. For example, for the culturing of human pluripotent stem cells, human factor for maintaining an undifferentiated state (e.g., bFGF, FGF4, FGF8, EGF, Nodal, Activin A, Activin B, TGFβ 1, and TGFβ 2) are used. The factor for maintaining an undifferentiated state is preferably isolated.

The factor for maintaining an undifferentiated state can be produced by any host or artificially synthesized as long as it has the ability to maintain an undifferentiated state of the pluripotent stem cells to be cultured. The factor for maintaining an undifferentiated state used in the present invention is preferably one that has undergone the same modification as that produced in vivo, and further preferably one produced under conditions that do not contain foreign components, in cells of the same type as the pluripotent stem cells to be cultured.

One embodiment of the production method of the present invention includes a step of providing an isolated factor for maintaining an undifferentiated state. One embodiment of the production method of the present invention includes a step of extrinsically (exogenously) adding an isolated factor for maintaining an undifferentiated state to a medium used in step (a). A factor for maintaining an undifferentiated state may be added in advance to a medium to be used in step (a).

The concentration of the factor for maintaining an undifferentiated state in the medium to be used in step (a) is a concentration capable of maintaining the undifferentiated state of the pluripotent stem cells to be cultured, and can be appropriately determined by those of ordinary skill in the art. For example, when bFGF is used as a factor for maintaining an undifferentiated state in the absence of feeder cells, the concentration thereof is generally about 4 ng/mL - about 500 ng/mL, preferably about 10 ng/mL - about 200 ng/mL, more preferably about 30 ng/mL - about 150 ng/mL.

Step (a) is performed in the absence of feeder cells. In step (a), the pluripotent stem cells may be cultured under any conditions of suspension culturing and adhesion culturing, preferably adhesion culturing. For culturing pluripotent stem cells under feeder-free conditions, an appropriate matrix may be used as a scaffold to provide a scaffold in stead of the feeder cells to the pluripotent stem cell. The pluripotent stem cells are subjected to adhesion culturing in a culture vessel whose surface is coated with a matrix as a scaffold.

As a matrix available as a scaffold, laminin (Nat Biotechnol 28, 611-615 (2010)), laminin fragment (Nat Commun 3, 1236 (2012)), basement membrane preparation (Nat Biotechnol 19, 971-974 (2001)), gelatin, collagen, heparan sulfate proteoglycan, entactin, vitronectin and the like can be mentioned. Laminin 511 is preferably used as the matrix (Nat Biotechnol 28, 611-615 (2010)).

A laminin fragment is not particularly limited as long as it has adhesiveness to pluripotent stem cells and enables maintenance culturing of pluripotent stem cell under feeder-free conditions, and is preferably E8 fragment. Laminin E8 fragment was identified as a fragment with strong cell adhesion activity among the fragments obtained by digestion of laminin 511 with elastase (EMBO J., 3:1463-1468, 1984, J. Cell Biol., 105:589-598, 1987). E8 fragment of laminin 511 is preferably used (Nat Commun 3, 1236 (2012), Scientific Reports 4, 3549 (2014)). The laminin E8 fragment is not required to be an elastase digestion product of laminin and may be a recombinant. Alternatively, it may be produced by a gene recombinant animal (Bombyx mori, etc.). To avoid contamination of unidentified components, a recombinant laminin fragment is preferably used. An E8 fragment of laminin 511 is commercially available and can be purchased from, for example, Nippi, Inc., and the like.

To avoid contamination with unidentified components, the laminin or laminin fragment to be used in the present invention is preferably isolated. Preferably, in the culturing of pluripotent stem cells under feeder-free conditions in step (a), pluripotent stem cells are cultured in an adhered state in a culture vessel with surface coated with isolated laminin 511 or E8 fragment of laminin 511, more preferably E8 fragment of laminin 511.

The medium to be used in step (a) is not particularly limited as long as it is a medium enabling culturing of pluripotent stem cells to maintain undifferentiated state under feeder-free conditions (feeder-free medium).

The medium to be used in step (a) may be a serum-containing medium or serum-free medium. To avoid contamination with chemically undetermined components, the medium to be used in step (a) is preferably a serum-free medium. The medium may contain a serum replacement.

While the period for the culturing of pluripotent stem cells in step (a) is not particularly limited as long as the effect of improving the quality of the cell population (aggregate) formed in subsequent first step can be achieved, it is generally 0.5 to 144 hr, preferably 2 to 96 hr, more preferably 6 to 48 hr, further preferably 12 to 48 hr, particularly preferably 18 to 28 hr, for example, 24 hr. That is, step (a) is started 0.5 to 144 hr (preferably 18 to 28 hr) before the start of first step, and the first step is continuously performed on completion of step (a).

In one preferable embodiment of step (a), human pluripotent stem cells are cultured in an adhered state in the absence of feeder cells and in a serum-free medium containing bFGF. The adhesion culturing is preferably performed in a culture vessel with surface coated with laminin 511, E8 fragment of laminin 511 or vitronectin. The adhesion culturing is preferably performed using StemFit medium as a feeder-free medium. The StemFit medium contains bFGF as a component for maintaining an undifferentiated state (Scientific Reports (2014)4, 3594).

In one preferred embodiment of step (a), human pluripotent stem cells are cultured in suspension in the absence of feeder cells in a serum-free medium containing bFGF. In the suspension culturing, the human pluripotent stem cells may form aggregates of human pluripotent stem cells.

In step (a) and the below-mentioned steps, culture conditions such as culture temperature, CO₂ concentration and the like can be appropriately set. The culture temperature is, for example, about 30°C to 40°C, preferably about 37°C. When a bicarbonate-buffered medium is used, the CO₂ concentration is, for example, about 1% to 10%, preferably about 5%.

### <Step (1)>, <step (1')>: the first step

In step (1'), pluripotent stem cells are cultured in the presence of a Wnt signal transduction pathway inhibiting substance to obtain a cell population.

The Wnt signal transduction pathway is a signal transduction pathway that uses a Wnt family protein as a ligand and mainly uses Frizzled as a receptor. Examples of the signal pathway include classical Wnt pathway (Canonical Wnt pathway), non-classical Wnt pathway (Non-Canonical Wnt pathway), and the like. The classical Wnt pathway is transmitted by β-Catenin. The Non-classical Wnt pathway includes Planar Cell Polarity (PCP) pathway, Wnt/JNK pathway, Wnt/Calcium pathway, Wnt-RAP1 pathway, Wnt-Ror2 pathway, Wnt-PKA pathway, Wnt-GSK3MT pathway, Wnt-aPKC pathway, Wnt-RYK pathway, and Wnt-mTOR pathway. In the Non-classical Wnt pathway, a common signal transduction factor which is also activated in other signaling pathways other than Wnt is present. In the present invention, such factors other than the aforementioned JNK pathway are also considered the constitution factors of the Wnt signal transduction pathway and inhibiting substances of the factors are also included in the Wnt signal transduction pathway inhibiting substance.

The Wnt signal transduction pathway inhibiting substance is not limited as long as it can suppress signal transduction induced by Wnt family proteins. The inhibiting substance may be any of nucleic acid, protein and low-molecular organic compound. Examples of the substance include a substance that inhibits Wnt processing and extracellular secretion, a substance that directly acts on Wnt (e.g., protein, antibody, and aptamer), a substance that suppresses expression of a gene encoding Wnt (e.g., antisense oligonucleotide, and siRNA), a substance that suppresses binding of Wnt receptor and Wnt, and a substance that suppresses physiological activity caused by signal transduction by Wnt receptor.

As a protein known as a Wnt signal transduction pathway inhibiting substance, proteins belonging to secreted Frizzled Related Protein (sFRP) class (sFRP1 to 5, Wnt inhibitory Factor-1 (WIF-1), Cerberus), proteins belonging to Dickkopf (Dkk) class (Dkk1 to 4, Kremen), PCDD1, APCDD1L, proteins belonging to Draxin family, IGFBP-4, Notum, proteins belonging to SOST/Sclerostin family, and the like can be mentioned.

As the Wnt signal transduction pathway inhibiting substance, a compound well known to those of ordinary skill in the art can be used. As inhibiting substance for the classical Wnt signal transduction pathway, for example, Frizzled inhibitor, Dishevelled (Dvl) inhibitor, Tankyrase inhibitor, casein kinase 1 inhibitor, catenin responsive transcription inhibitor, p300 inhibitor, CREB-binding protein (CBP) inhibitor, BCL-9 inhibitor, TCF degrader (Am J Cancer Res. 2015; 5(8): 2344-2360), and the like can be mentioned. As inhibiting substance for the non-classical Wnt signal transduction pathway, for example, Porcupine (PORCN) inhibitor, Calcium/calmodulin-dependent protein kinase II (CaMKII) inhibitor, (TGF-β-activated kinase 1 (TAK1) inhibitor, Nemo-Like Kinase (NLK) inhibitor, LIM Kinase inhibitor, mammalian target of rapamycin (mTOR) inhibitor, Rac inhibitor, c-Jun NH 2-terminal kinase (JNK) inhibitor, protein kinase C (PKC) inhibitor, Methionine Aminopeptidase 2 (MetAP2) inhibitor, Calcineurin inhibitor, nuclear factor of activated T cells (NFAT) inhibitor, ROCK inhibitor, and the like can be mentioned. While the action mechanism has not been reported, KY-02111 (N-(6-Chloro-2-benzothiazolyl)-3,4-dimethoxybenzenepropanamide) and KY03-I (2-(4-(3,4-dimethoxyphenyl)butanamide)-6-Iodobenzothiazole) can be recited as the Wnt signal transduction pathway inhibiting substance. These substances may be used alone or in combination.

As the PORCN inhibitor for example, IWP-2 (N-(6-Methyl-2-benzothiazolyl)-2-[(3,4,6,7-tetrahydro-4-oxo-3-phenylthieno[3,2-d]pyrimidin-2-yl)thio]-acetamide), IWP-3 (2-[[3-(4-fluorophenyl)-3,4,6,7-tetrahydro-4-oxothieno[3,2-d]pyrimidin-2-yl]thio]-N-(6-methyl-2-benzothiazolyl)-acetamide), IWP-4 (N-(6-methyl-2-benzothiazolyl)-2-[[3,4,6,7-tetrahydro-3-(2-methoxyphenyl)-4-oxothieno[3,2-d]pyrimidin-2-yl]thio]-acetamide), IWP-L6 (N-(5-phenyl-2-pyridinyl)-2-[(3,4,6,7-tetrahydro-4-oxo-3-phenylthieno[3,2-d]pyrimidin-2-yl)thio]-acetamide), IWP-12 (N-(6-Methyl-2-benzothiazolyl)-2-[(3,4,6,7-tetrahydro-3,6-dimethyl-4-oxothieno[3,2-d]pyrimidin-2-yl)thio]acetamide), IWP-O1 (1H-1,2,3-Triazole-1-acetamide,5-phenyl-N-(5-phenyl-2-pyridinyl)-4-(4-pyridinyl)-), LGK-974 (2-(2',3-Dimethyl-2,4'-bipyridin-5-yl)-N-(5-(pyrazin-2-yl)pyridin-2-yl)acetamide), Wnt-C59 (2-[4-(2-Methylpyridin-4-yl)phenyl]-N-[4-(pyridin-3-yl)phenyl]acetamide), ETC-131, ETC-159 (1,2,3,6-Tetrahydro-1,3-dimethyl-2,6-dioxo-N-(6-phenyl-3-pyridazinyl)-7H-purine-7-acetamide), GNF-1331 (N-(6-methoxy-1,3-benzothiazol-2-yl)-2-[(4-propyl-5-pyridin-4-yl-1,2,4-triazol-3-yl)sulfanyl]acetamide), GNF-6231 (N-[5-(4-Acetyl-1-piperazinyl)-2-pyridinyl]-2'-fluoro-3-methyl[2,4'-bipyridine]-5-acetamide), Porcn-IN-1 (N-[[5-fluoro-6-(2-methylpyridin-4-yl)pyridin-3-yl]methyl]-9H-carbazole-2-carboxamide), RXC004, CGX1321 and derivatives of these, and the like can be mentioned. These substances may be used alone or in combination.

The Wnt signal transduction pathway inhibiting substance preferably contains at least one selected from the group consisting of PORCN inhibitor, KY02111, and KY03-I, more preferably PORCN inhibitor. The Wnt signal transduction pathway inhibiting substance also preferably contains a substance having inhibitory activity against non-classical Wnt pathway of Wnt. The Wnt signal transduction pathway inhibiting substance more preferably contains a substance having inhibitory activity against Wnt/Planar Cell Polarity (PCP) pathway. The PORCN inhibitor used in the present invention preferably contains at least one selected from the group consisting of IWP-2, IWP-3, IWP-4, IWP-L6, IWP-12, LGK-974, Wnt-C59, ETC-159, and GNF-6231, more preferably IWP-2 or Wnt-C59, further preferably IWP-2.

The concentration of a Wnt signal transduction pathway inhibiting substance in the medium can be appropriately determined according to the substances used within a range capable of achieving the aforementioned effects. From the aspects of improving the production efficiency of the cells constituting the pituitary, for example, when IWP-2 which is one kind of PORCN inhibitor is used as the Wnt signal transduction pathway inhibiting substance, the concentration thereof is generally about 10 nM to about 50 µM, preferably about 10 nM to about 30 µM, further preferably about 100 nM to about 10 µM, most preferably about 0.5 µM. When Wnt-C59 which is one kind of PORCN inhibitor is used, the concentration thereof is generally about 10 nM to about 30 µM, preferably about 20 nM to about 10 µM, more preferably about 500 nM. When KY02111 is used, the concentration thereof is generally about 10 nM to about 50 µM, preferably 10 nM to about 30 µM, more preferably about 100 nM to about 10 µM, further preferably about 5 µM.

In the medium in step (1'), c-Jun N-terminal kinase (JNK) signal transduction pathway inhibiting substance is further preferably present. Such first step is step (1). That is, step (1) is the first step of culturing pluripotent stem cells in the presence of a JNK signal transduction pathway inhibiting substance and a Wnt signal transduction pathway inhibiting substance.

JNK is a kinase belonging to the MAPK family and is involved in intracellular signal transduction stimulated by various environmental stresses, inflammatory cytokines, growth factors, and GPCR agonists.

In the present invention, the JNK signal transduction pathway inhibiting substance is not limited as long as it can suppress the signal transduction transmitted by JNK. JNK signal transduction pathway inhibiting substance includes, for example, substances having the activity to inhibit signal transduction by mechanisms that inhibit enzymatic activity, multimerization, binding with other factors or nucleic acids, promote degradation, and the like, of factors upstream or downstream of the JNK signal transduction mechanism, or JNK itself. Examples of the JNK signal transduction pathway inhibiting substance include, but are not limited to, JNK inhibitor, Rac inhibitor, MKK inhibitor, MEK inhibitor, Src inhibitor, receptor tyrosine kinase (RTK) inhibitor, ASK inhibitor, and the like.

Examples of the c-Jun N-terminal kinase (JNK) inhibitor include JNK-IN-8 ((E)-3-(4-(dimethylamino)but-2-enamido)-N-(3-methyl-4-((4-(pyridin-3-yl)pyrimidin-2-yl)amino)phenyl)benzamide), SP600125 (Anthra[1-9-cd]pyrazol-6(2H)-one), DB07268 (2-[[2-[(3-Hydroxyphenyl)amino]-4-pyrimidinyl]amino]benzamide), Tanzisertib (trans-4-[[9-[(3S)-Tetrahydro-3-furanyl]-8-[(2,4,6-trifluorophenyl)amino]-9H-purin-2-yl]amino]cyclohexanol), Bentamapimod (1,3-Benzothiazol-2-yl)[2-[[4-[(morpholin-4-yl)methyl]benzyl]oxy]pyrimidin-4-yl]acetonitrile, TCS JNK 6o (N-(4-Amino-5-cyano-6-ethoxy-2-pyridinyl)-2,5-dimethoxybenzeneacetamide), SU3327 (5-[(5-Nitro-2-thiazolyl)thio]-1,3,4thiadiazol-2-amine), CEP1347 ((9S,10R,12R)-5-16-Bis[(ethylthio)methyl]-2,3,9,10,11,12-hexahydro-10-hydroxy-9-methyl-1-oxo-9,12-epoxy-1H-diindolo[1,2,3-fg:3',2',1'-kl]pyrrolo[3,4-i][1,6]benzodiazocine-10-carboxylic acid methyl ester), c-JUN peptide, AEG3482 (6-Phenylimidazo[2,1-b]-1,3,4-thiadiazole-2-sulfonamide), TCS JNK 5a (N-(3-Cyano-4,5,6,7-tetrahydrobenzo[b]thienyl-2-yl)-1-naphthalenecarboxamide), BI-78D3 (4-(2,3-Dihydro-1,4-benzodioxin-6-yl)-2,4-dihydro-5-[(5-nitro-2-thiazolyl)thio]-3H-1,2,4-triazol-3-one), IQ-3 (11H-Indeno[1,2-b]quinoxalin-11-one O-(2-furanylcarbonyl)oxime), SR 3576 (3-[4-[[[(3-Methylphenyl)amino]carbonyl]amino]-1H-pyrazol-1-yl]-N-(3,4,5-trimethoxyphenyl)benzamide), IQ-1S (11H-Indeno[1,2-b]quinoxalin-11-one oxime sodium salt), JIP-1 (153-163), CC-401 (3-[3-[2-(1-Piperidinyl)ethoxy]phenyl]-5-(1H-1,2,4-triazol-5-yl)-1H-indazole dihydrochloride), BI-87G3 (2-(5-Nitrothiazol-2-ylthio)benzo[d]thiazole), AS601245 (1,3-Benzothiazol-2-yl-(2-{ [2-(3-pyridinyl)ethyl]amino}-4-pyrimidinyl) acetonitrile), CV-65 (3,7-Dimethyl-1,9-dihydropyrido[3,2-g]quinoline-2,5,8,10-tetrone), D-JNK1 (CAS No. 1445179-97-4), ER-358063, ER-409903, ER-417258, CC-359 ((4S)-4-(2,4-difluoro-5-pyrimidin-5-ylphenyl)-4-methyl-5,6-dihydro-1,3-thiazin-2-amine), CC-401 (3-[3-(2-piperidin-1-ylethoxy)phenyl]-5-(1H-1,2,4-triazol-5-yl)-1H-indazole), CC-930 (4-[[9-[(3S)-oxolan-3-yl]-8-(2,4,6-trifluoroanilino)purin-2-yl]amino]cyclohexan-1-ol), SB203580 (4-[4-(4-fluorophenyl)-2-(4-methylsulfinylphenyl)-1H-imidazol-5-yl]pyridine) and derivatives of these, and the like can be mentioned. These substances may be used alone or in combination.

Examples of the Rac inhibitor include EHT1864 (5-(5-(7-(Trifluoromethyl)quinolin-4-ylthio)pentyloxy)-2-(morpholinomethyl)-4H-pyran-4-one dihydrochloride), NSC23766 (N6-[2-[[4-(Diethylamino)-1-methylbutyl]amino]-6-methyl-4-pyrimidinyl]-2-methyl-4,6-quinolinediamine trihydrochloride), EHop-016 (N4-(9-Ethyl-9H-carbazol-3-yl)-N2-[3-(4-morpholinyl)propyl]-2,4-pyrimidinediamine), 1A-116 (N-(3,5-Dimethylphenyl)-N'-[2-(trifluoromethyl)phenyl]guanidine), ZCL278 (2-(4-broMo-2-chlorophenoxy)-N-(4-(N-(4,6-diMethylpyriMidin-2-yl) sulfaMoyl)phenylcarbaMothioyl) acetaMide), MBQ-167 (9-ethyl-3-(5-phenyl-1H-1,2,3-triazol-1-yl)-9H-carbazole), KRpep-2d (actinium; [(2S)-1-[[(2S)-1-[[(2S)-1-[[(2S)-1-[[(3R,8R,11S,14S,20S,23S,26S,29S,32S,35S,38S)-8-[[(2S)-1-[[(2S)-1-[[(2S)-1-[[(2S)-1-amino-5-carbamimidamido-1-oxopentan-2-yl]amino]-5-carbamimidamido-1-oxopentan-2-yl]amino]-5-carbamimidamido-1-oxopentan-2-yl]amino]-5-carbamimidamido-1-oxopentan-2-yl]carbamoyl]-29-[(2R)-butan-2-yl]-20-(carboxymethyl)-26-(hydroxymethyl)-23,32-bis[(4-hydroxyphenyl)methyl]-35-(2-methylpropyl)-2,10,13,19,22,25,28,31,34,37-decaoxo-11-propan-2-yl-5,6-dithia-1,9,12,18,21,24,27,30,33,36-decazatricyclo[36.3.0.014,18]hentetracontan-3-yl]amino]-5-carbamimidamido-1-oxopentan-2-yl]amino]-5-carbamimidamido-1-oxopentan-2-yl]amino]-5-carbamimidamido-1-oxopentan-2-yl]amino]-5-carbamimidamido-1-oxopentan-2-yl]azanide), ARS-853 (1-[3-[4-[2-[[4-Chloro-2-hydroxy-5-(1-methylcyclopropyl)phenyl]amino]acetyl]-1-piperazinyl]-1-azetidinyl]-2-propen-1-one), Salirasib (2-(((2E,6E)-3,7,11-Trimethyldodeca-2,6,10-trien-1-yl)thio)benzoic acid), ML141 (4-(5-(4-methoxyphenyl)-3-phenyl-4,5-dihydropyrazol-1-yl)benzenesulfonamide) and derivatives of these, and the like can be mentioned. These substances may be used alone or in combination.

The timing of addition of the JNK signal transduction pathway inhibiting substance in the present invention is not limited as long as the effect of improving the efficiency of producing pituitary tissue from human pluripotent stem cells is exerted. In the below-mentioned step (2), it is preferable that the BMP signal transduction pathway inhibiting substance should be added already when a BMP signal transduction pathway activating substance is added, which is within 72 hours after the start of differentiation induction. A more preferred timing for adding the JNK signal transduction pathway inhibiting substance is at the same time as the start of differentiation induction.

The medium in the first step (step (1) or step (1')) preferably further contains a TGFβ signal transduction pathway inhibiting substance. As a TGFβ signal transduction pathway inhibiting substance to be used in the first step, those similar to the ones exemplified in step (a) can be used. The TGFβ signal transduction pathway inhibiting substances in step (a) and the first step may be the same or different, preferably the same.

The concentration of a TGFβ signal transduction pathway inhibiting substance in the medium can be appropriately determined according to the substances used within a range capable of achieving the aforementioned effects. When SB431542 is used as the TGFβ transduction pathway inhibiting substance, it is generally used at a concentration of about 1 nM to about 100 µM, preferably about 10 nM - about 100 µM, more preferably about 100 nM to about 50 µM, further preferably about 500 nM to about 10 µM. When a TGFβ signal transduction pathway inhibiting substance other than SB431542 is used, it is desirably used at a concentration that shows TGFβ signal transduction pathway inhibiting activity equivalent to that of SB431542 at the above-mentioned concentration.

In the first step and the steps thereafter, from the aspects of suppressing differentiation into mesendoderm and improving the production efficiency of ectoderm ·placode-like tissues, it is also preferable to add an inhibiting substance against Transforming growth factor-β-activated kinase 1 (TAK1). TAK1 is a serine-threonine protein kinase of the MAP kinase kinase kinase (MAPKKK) family that mediates signal transduction activated by TGFβ, bone morphogenetic protein (BMP), interleukin 1 (IL-1), TNF-α, and the like.

The TAK1 inhibiting substance is not limited as long as it can suppress signal transduction mediated by TAK1. It may be a nucleic acid, a protein, or a low-molecular organic compound. Such substances include, for example, substances that inhibit the binding of TAK1 to a substrate, substances that inhibit phosphorylation of TAK1, substances that promote dephosphorylation of TAK1, substances that inhibit transcription or translation of TAK1, substances that promote degradation of TAK1, and the like.

As the TAK1 inhibiting substance, for example, (5Z)-7-Oxozeaenol((3S,5Z,8S,9S,11E)-3,4,9,10-tetrahydro-8,9,16-trihydroxy-14-methoxy-3-methyl-1H-2-benzoxacyclotetradecin-1,7(8H)-dione), N-Des(aminocarbonyl)AZ-TAK1 inhibitor(3-Amino-5-[4-(4-morpholinylmethyl)phenyl]-2-thiophenecarboxamide), Takinib (N1-(1-Propyl-1H-benzimidazol-2-yl)-1,3-benzenedicarboxamide), NG25 (N-[4-[(4-Ethyl-1-piperazinyl)methyl]-3-(trifluoromethyl)phenyl]-4-methyl-3-(1H-pyrrolo[2,3-b]pyridin-4-yloxy)-benzamide trihydrochloride), Sarsasapogenin, and derivatives and analogs of these can be mentioned. These substances may be used alone or in combination.

The TAK1 inhibiting substance is preferably (5Z)-7-Oxozeaenol. When (5Z)-7-Oxozeaenol is used as the TAK1 inhibiting substance in the first step, it is generally used at a concentration of about 1 nM to about 100 µM, preferably about 10 nM to about 50 µM, more preferably about 100 nM to about 25 µM, further preferably about 500 nM to about 10 µM. When a TAK1 inhibiting substance other than (5Z)-7-Oxozeaenol is used, it is preferably used at a concentration that exhibits TAK1 inhibitory activity equivalent to that of (5Z)-7-Oxozeaenol at the above-mentioned concentration. From the aspect of controlling the proportion of cells contained in pituitary tissue, the above-mentioned TAK1 inhibiting substance can be added at any stage of the first step and the steps thereafter, and then removed. In one preferred embodiment, the above-mentioned TAK1 inhibiting substance is added at the beginning of the below-mentioned step (b).

The medium used in the first step is not particularly limited as long as it is as described in the above-mentioned definition. The medium to be used in the first step may be a serum-containing medium or serum-free medium. In order to avoid contamination with chemically undetermined components, a serum-free medium is preferably used in the present invention. In order to avoid complicated preparation, for example, a serum-free medium supplemented with an appropriate amount of a commercially available serum replacement such as KSR is preferably used. The amount of KSR to be added to a serum-free medium in the case of human ES cell is generally about 1% to about 30%, preferably about 2% to about 20%. Examples of the serum-free medium include a 1:1 mixture medium of IMDM and F-12 which is supplemented with 5% KSR, 450 µM 1-monothioglycerol, and 1xChemically Defined Lipid Concentrate, or GMEM medium supplemented with 5% to 20% KSR, NEAA, pyruvic acid, and 2-mercaptoethanol.

At the start of the first step, cells may be in either an adherent state or a floating state. In a preferred embodiment, pluripotent stem cells are dispersed into single cells and then reaggregated to form cell aggregates in a floating state. For this purpose, it is preferable to perform an operation to disperse the pluripotent stem cells, for example the pluripotent stem cells obtained in step (a), into single cells before the start of the first step. The "dispersed cells" obtained by the dispersing operation are preferably single cells, but may also include masses of cells consisting of a small number of cells, for example, not less than 2 and not more than 100, and may contain masses of cells consisting of not less than 2 and not more than 50 cells. The "dispersed cells" may contain, for example, 70% or more of single cells and 30% or less of cell masses, preferably 80% or more of single cells and 20% or less of cell masses.

As a method for dispersing pluripotent stem cells, mechanical dispersion treatment, cell dissociation solution treatment, and cell protectant addition treatment can be mentioned, and these treatments may be performed in combination. As a method for dispersing the cells, preferably, a cell dissociation solution is performed simultaneously with a cell protectant addition treatment, and then a mechanical dispersion treatment is performed.

As a cell protectant to be used for a cell protectant addition treatment, FGF signal transduction pathway activating substance, heparin, Rho-associated protein kinase (ROCK) inhibiting substance, myosin inhibiting substance, polyamines, integrated stress response (ISR) inhibitor, caspase inhibitor, serum, or serum replacement can be mentioned. As a preferred cell protectant, ROCK inhibiting substances can be mentioned. In order to suppress cell death of pluripotent stem cells (particularly, human pluripotent stem cells) induced by dispersing, it is preferable to add a ROCK inhibiting substance from the start of culture in the first step. Examples of the ROCK inhibiting substance include Y-27632 ((R)-(+)-trans-4-(1-Aminoethyl)-N-(4-pyridyl)cyclohexanecarboxamide, dihydrochloride), Fasudil (HA1077) (1-(5-Isoquinolinylsulfonyl)homopiperazine, hydrochloride), H-1152 (5-[[(2S)-hexahydro-2-methyl-1H-1,4-diazepin-1-yl]sulfonyl]-4-methyl-isoquinoline, dihydrochloride), HA-1100 (Hydroxyfasudil) ([1-(1-Hydroxy-5-isoquinolinesulfonyl)homopiperazine, hydrochloride), Chroman 1 ((3S)-N-[2-[2-(dimethylamino)ethoxy]-4-(1H-pyrazol-4-yl)phenyl]-6-methoxy-3,4-dihydro-2H-chromene-3-carboxamide), Belumosudil (KD025, 2-[3-[4-[(1H-Indazol-5-yl)amino]quinazolin-2-yl]phenoxy]-N-isopropylacetamide), HSD1590 ([2-Methoxy-3-(4,5,10-triazatetracyclo[7.7.0.02,6.012,16]hexadeca-1(9),2(6),3,7,10,12(16)-hexaen-11-yl)phenyl]boronic acid), CRT0066854 ((S)-3-phenyl-N1-(2-pyridin-4-yl-5,6,7,8-tetrahydrobenzo[4,5]thieno[2,3-d]pyrimidin-4-yl)propane-1,2-diamine), RKI1447 (1-(3-hydroxybenzyl)-3-(4-(pyridin-4-yl)thiazol-2-yl)urea), Ripasudil (4-Fluoro-5-[[(2S)-hexahydro-2-methyl-1H-1,4-diazepin-1-yl]sulfonyl]isoquinoline), GSK269962A (N-[3-[2-(4-amino-1,2,5-oxadiazol-3-yl)-1-ethylimidazo[4,5-c]pyridin-6-yl]oxyphenyl]-4-(2-morpholin-4-ylethoxy)benzamide), GSK429286A (N-(6-fluoro-1H-indazol-5-yl)-2-methyl-6-oxo-4-(4-(trifluoromethyl)phenyl)-1,4,5,6-tetrahydropyridine-3-carboxamide), Y-33075 ((R)-4-(1-Aminoethyl)-N-1H-pyrrolo[2,3-b]pyridin-4-ylbenzamide), LX7101 (N,N-Dimethylcarbamic acid 3-[[[4-(aminomethyl)-1-(5-methyl-7H-pyrrolo[2,3-d]pyrimidin-4-yl)-4-piperidinyl]carbonyl]amino]phenyl ester), AT13148 ((alphaS)-alpha-(Aminomethyl)-alpha-(4-chlorophenyl)-4-(1H-pyrazol-4-yl)benzenemethanol), SAR407899 (6-(piperidin-4-yloxy)isoquinolin-1(2H)-one hydrochloride), GSK180736A (4-(4-fluorophenyl)-N-(1H-indazol-5-yl)-6-methyl-2-oxo-1,2,3,4-tetrahydropyrimidine-5-carboxamide), Hydroxyfasudil (1-(1-hydroxy-5-isoquinolinesulfonyl)homopiperazine, HCl), bdp5290 (4-Chloro-1-(4-piperidinyl)-N-[3-(2-pyridinyl)-1H-pyrazol-4-yl]-1H-pyrazole-3-carboxamide), sr-3677 (N-[2-[2-(Dimethylamino)ethoxy]-4-(1H-pyrazol-4-yl)phenyl-2,3-dihydro-1,4-benzodioxin-2-carboxamidehydrochloride), CCG-222740 (N-(4-Chlorophenyl)-5,5-difluoro-1-(3-(furan-2-yl)benzoyl)piperidine-3-carboxamide), ROCK inhibitor-2 (N-[(1R)-1-(3-methoxyphenyl)ethyl]-4-pyridin-4-ylbenzamide), Rho-Kinase-IN-1 (N-[1-[(4-methylsulfanylphenyl)methyl]piperidin-3-yl]-1H-indazol-5-amine), ZINC00881524 (N-(4,5-dihydronaphtho[1,2-d]thiazol-2-yl)-2-(3,4-dimethoxyphenyl)acetamide), SB772077B ((3S)-1-[[2-(4-Amino-1,2,5-oxadiazol-3-yl)-1-ethyl-1H-imidazo[4,5-c]pyridin-7-yl]carbonyl]-3-pyrrolidinamine dihydrochloride), Verosudil (N-(1,2-Dihydro-1-oxo-6-isoquinolinyl)-alpha-(dimethylamino)-3-thiopheneacetamide), GSK-25 (4-(4-chloro-2-fluorophenyl)-2-(2-chloropyridin-4-yl)-1-(6-fluoro-1H-indazol-5-yl)-6-methyl-4H-pyrimidine-5-carboxamide) and derivatives of these and the like. As the cell protectant, a cell protectant after preparation can also be used. Examples of the cell protectant after preparation include RevitaCell Supplement (manufactured by Thermo Fisher Scientific), and CloneR (manufactured by Stemcell Technologies). These substances may be used alone or in combination. In the first step, when ROCK inhibiting substance Y-27632 is added as a cell protectant, it is added into the culture environment at a concentration of generally about 10 nM to about 10 mM, preferably about 100 nM to about 1 mM, more preferably about 1 µM to about 100 µM. In the first step, when ROCK inhibiting substance Chroman 1 is added as a cell protectant, it is added into the culture environment at a concentration of generally about 10 pM to about 1 mM, preferably about 100 pM to about 100 µM, more preferably about 1 nM to about 10 µM.

As a cell dissociation solution to be used for the cell dissociation solution treatment, a solution containing an enzyme such as trypsin, collagenase, hyaluronidase, elastase, pronase, DNase, papain and so on, and at least one chelating agent such as ethylenediaminetetraacetic acid and so on can be mentioned. A commercially available cell dissociation solution such as TripLE Select (manufactured by Thermo Fisher Scientific), TripLE Express (manufactured by Thermo Fisher Scientific), Accumax (manufactured by Innovative Cell Technologies) can also be used. A cell dissociation solution preferred for the treatment of pluripotent stem cells obtained after step (a) is TrypLE Select or phosphate buffer (PBS) added with 5 mM EDTA, but it is not limited thereto.

As a method of mechanical dispersion treatment, a pipetting treatment or scraping by a scraper can be mentioned.

The dispersed cells are suspended in the above-mentioned medium.

A method for dispersing pluripotent stem cells includes, for example, a method involving treating a colony of pluripotent stem cells with TrypLE Select, ethylenediaminetetraacetic acid, or Accumax in the presence of a ROCK inhibiting substance, and further dispersing them by pipetting.

When suspension culturing is performed in the first step, a suspension of the dispersed pluripotent stem cells is seeded in a cell non-adhesive culture vessel. When the culture vessel is non-adhesive, the cells are suspension cultured and a plurality of pluripotent stem cells gather and form cell aggregates.

In suspension culturing, plural cell aggregates may be simultaneously formed in one culture vessel by seeding the dispersed pluripotent stem cells in a comparatively large culture vessel such as a 10 cm dish. To prevent easy occurrence of size dispersion of each cell aggregate, for example, a given amount of the dispersed pluripotent stem cells are preferably seeded in each well of a multiwell plate (U-bottom, V-bottom) such as a cell non-adhesive 96-well microplate, and static culturing is performed, whereby the cells rapidly aggregate to form one cell aggregate in each well. For example, a culture vessel can be made non-cell-adhesive by processing such as coating the surface of the culture vessel with a superhydrophilic polymer. Examples of the non-cell-adhesive multiwell plate include PrimeSurface 96V bottom plate (MS-9096V, manufactured by SUMITOMO BAKELITE CO., LTD.). Centrifugation may be performed to form cell aggregates more quickly. By collecting cell aggregates formed in each well from a plurality of wells, a uniform population of cell aggregates can be obtained. When the cell aggregates are uniform, the production efficiency for each well and each repeated experiment can be more stabilized in the subsequent steps, and cells constituting the pituitary can be produced with higher reproducibility.

In another embodiment for forming cell aggregates from dispersed pluripotent stem cells, a culture vessel where the cells settle in one place on the bottom and the formation of aggregates is promoted, such as mortar, downward facing square pyramid, concave shape processing, grids, ridges, and the like formed in plurality, a culture vessel that has been treated to allow cells to adhere to only a part of the bottom surface thereof to facilitate the formation of aggregates, and the like can also be used. Examples of the above-mentioned culture vessel include, but are not limited to, embryoid formation plate AggreWell (manufactured by STEMCELL Technologies), PAMCELL (manufactured by ANK), spheroid microplate (manufactured by Corning), NanoCulture Plate/Dish (manufactured by Organogenix), Cell-able (manufactured by Toyo Gosei Co., Ltd.), EZSPHERE (manufactured by AGC TECHNO GLASS CO., LTD.), SPHERICALPLATE 5D (manufactured by Mitokogyo Corporation), TASCL (manufactured by Cymss-bio Co., Ltd.), and the like.

As the culture vessel, a three-dimensional cell culture container permitting exchange of the medium of the whole plate while cell aggregates are contained in each well is also preferred. Examples of such three-dimensional cell culture container include PrimeSurface 96 Slit well plate (manufactured by SUMITOMO BAKELITE CO., LTD.) and the like. This plate has narrow openings (slits) that allow the medium to enter and exit at the top of each of the 96 wells. The slits are set to have a width that makes it difficult for cell aggregates to pass through, which makes it possible to replace the medium in the entire plate at once while preventing cell aggregates from adhering to each other, and improve operation efficiency and quality of cell aggregates.

The concentration of the pluripotent stem cells in the first step can be appropriately set so that cell aggregates can be more uniformly and efficiently formed. For example, when human pluripotent stem cells (e.g., human iPS cell obtained in step (a)) are suspension cultured using a 96-well microwell plate, a liquid prepared to achieve generally about 1 x 10³ to about 1 x 10⁵ cells, preferably about 3 x 10³ to about 5 x 10⁴ cells, more preferably about 4 x 10³ to about 2 x 10⁴ cells, further preferably about 4 x 10³ to about 1.6 x 10⁴ cells, particularly preferably about 8 x 10³ to about 1.2 x 10⁴ cells, per well is added to each well, and the plate is left to stand to form cell aggregates. The number of cells can be determined by counting with a hemocytometer.

The period for suspension culturing necessary for forming a cell aggregate can be determined as appropriate according to the pluripotent stem cell to be used. To form uniformed cell aggregates, it is desirably as short as possible. The steps for the dispersed cells to form cell aggregates can be divided into a step for gathering cells, and a step for forming aggregates from the gathered cells. From seeding the dispersed cells (i.e., at the time of the start of suspension culturing) to allowing for gathering of the cells in case of human pluripotent stem cell (human iPS cell, etc.) is, for example, preferably within about 24 hr, more preferably within about 12 hr. In the step of seeding the dispersed cells (i.e., at the time of the start of suspension culturing) to allow for forming a cell aggregate in the case of human pluripotent stem cells (e.g., human iPS cells), it is, for example, preferably within about 72 hr, more preferably within about 48 hr. The time up to cell aggregate formation can be appropriately adjusted by adjusting tools for causing aggregation of cells, centrifugation conditions, and the like.

When a cell aggregate is formed by rapidly gathering pluripotent stem cells, an epithelium-like structure can be formed with good reproducibility in the cells induced and differentiated from the formed aggregate. Examples of the experimental operation to form a cell aggregate include a method involving keeping cells in a small space by using a plate with small wells (e.g., plate with wells having a base area of about 0.1 to 2.0 cm² when calculated in terms of flat bottom), micropore and so on, a method involving aggregating cells by centrifugation for a short time using a small centrifugation tube. As a plate with small wells, for example, 24 well plate (area of about 1.88 cm² when calculated in terms of flat bottom), 48 well plate (area of about 1.0 cm² when calculated in terms of flat bottom), 96 well plate (area of about 0.35 cm² when calculated in terms of flat bottom, inner diameter about 6 to 8 mm), and 384 well plate can be mentioned. Preferred is 96 well plate. As a shape of the plate with small wells, the shape of the bottom surface when the well is seen from above is, for example, polygon, rectangle, ellipse, true circle, preferably true circle. As a shape of the plate with small wells when the well is seen from the side, the shape of the bottom surface is preferably a structure having high outer circumference and low concave inner part, which is, for example, U-bottom, V-bottom or M-bottom, preferably U-bottom or V-bottom, most preferably V-bottom. As a plate with small wells, a cell culture dish (e.g., 60 mm to 150 mm dish, culture flask) with a concave convex, or dent on the bottom surface may also be used. The bottom surface of a plate with small wells is preferably a non-cell-adhesive bottom surface, preferably a non-cell-adhesive-coated bottom surface.

As another method for forming cell aggregates, it is also preferable to use a three-dimensional printing machine or a 3D printer. A cell population with a desired morphology can be prepared by a method in which spheroids consisting of a single dispersed cell or multiple cells are suspended in biocompatible ink (bioink) and output using a bio 3D printer (e.g., BIO X manufactured by Celllink, etc.), or a cell population is pricked with a needle and stack it up (Spike manufactured by Cyfuse Biomedical K.K., etc.).

Formation of cell aggregates can be determined based on the size and cell number of the cell aggregate, macroscopic morphology of the aggregate, microscopic morphology by tissue staining analysis and uniformity thereof, expression of differentiation and undifferentiation markers and uniformity thereof, control of expression of differentiation marker and synchronism thereof, reproducibility of differentiation efficiency between aggregates, and so on.

At the beginning of the first step, in a preferred embodiment, adhesion culturing is performed. The pluripotent stem cells on the culture vessel after step (a) may be used as they are in the first step, or the pluripotent stem cells may be dispersed into single cells and then seeded again on the adhesive culture vessel. During re-seeding after dispersing pluripotent stem cells into single cells, appropriate extracellular matrix or synthetic cell adhesion molecules may be used as a scaffold. With the scaffold, pluripotent stem cells can be adhesion cultured in a culture vessel with a coated surface. The extracellular matrix is preferably Matrigel or laminin. As the synthetic cell adhesion molecule, synthetic peptides containing cell adhesive domain, such as poly-D-lysine, RGD sequence, and the like can be mentioned. The number of cells seeded is not particularly limited as long as they differentiate into the pituitary. From the aspect of reproducing adhesion and interaction between cells, a density that allows the cell density to reach semi-confluence, which is equivalent to 60% or more of the culture space of the culture vessel, within 72 hours after seeding into the culture vessel is also preferred.

It is also preferable to use a micropatterned culture vessel as the adhesive culture vessel. The micropattern on the culture vessel can be composed of a cell-adhesive region and a cell-non-adhesive region, and cells are preferably adhesion cultured in the cell-adhesive region. The shapes of the cell adhesive region and the cell non-adhesive region are not limited as long as they can be developed on the culture vessel. Only one region of a cell adhesive region and a cell non-adhesive region may be formed or a plurality thereof may be formed on one culture vessel. The cell adhesive region is preferably artificially treated for the purpose of improving adhesiveness. Examples of culture vessel with micropatterns include CYTOOchip (manufactured by CYTOO), ibidi Micropatterning (manufactured by ibidi), and the like. Culture vessels can also be prepared using PDMS mold, matrix, and the like. Alternatively, culture vessels coated with an extracellular matrix, a substrate that promotes cell adhesion, and the like are processed with a laser or the like using a cell processing device (Model: CPD-017, manufactured by Kataoka Seisakusho Co., Ltd.) to form cell adhesive region and cell-non-adhesive region in any shape. When culturing the pluripotent stem cells obtained in step (a) on a micropatterned culture vessel, it can be performed, for example, with reference to previously reported methods (Nature protocols, 11(11), 2223-2232.).

It is also preferable that the culture vessel has a flow path (micro flow path) for perfusing the culture medium, and cells may be cultured in a perfusion environment in the first step and the steps thereafter. Such culture vessel is also referred to as a microfluid chip. The culture vessel (e.g., microfluid chip) may be connected by a flow path to another culture vessel (for example, microfluid chip) for culturing cells or tissues other than the cells cultured in the production method of the present invention. This reproduces the interaction between the pituitary and other cells or tissues. Other cells or tissues to be co-cultured with the pituitary include, but are not limited to, tissues that are regulated by hormones secreted by the pituitary, tissues that promote growth, differentiation, maturation, and survival of the pituitary, such as cells or tissues of the brain, blood vessel, bone, muscle, fat, thyroid, liver, adrenal gland, testes, ovary, and breast. Examples of methods for perfusing the medium include, but are not limited to, use of a magnetic stirrer, a peristaltic pump, and the like.

Culture vessel may have a membrane that is permeable to oxygen or a medium. Culture vessel may be capable of forming a concentration gradient of compounds, growth factors, and the like. The membrane is, for example, a porous membrane. In a culture vessel having such a membrane, cells can be cultured by the production method of the present invention on one side separated by the membrane, and other cells or tissues, feeder cells, and the like can be cultured on the other side. This makes it possible to culture cells constituting the pituitary, or progenitor cells thereof, and cell populations containing them without contaminating other cells or tissues.

In the first step and the steps thereafter, when a medium change operation is performed, for example, it can be performed by an operation to add a fresh medium without discarding the existing medium (medium addition operation), an operation to discard about a half amount of the existing medium (about 30 - 90%, for example, about 40 - 60% of the volume of the existing medium) and add about a half amount of a fresh medium (about 30 - 90%, for example, about 40 - 60% of the volume of the existing medium) (half-medium change operation), and an operation to discard about the whole amount of the existing medium (not less than 90% of the volume of the existing medium) and add about the whole amount of a fresh medium (not less than 90% of the volume of the existing medium) (full-medium change operation) and the like.

When a particular component is added at a certain time point, for example, an operation to calculate the final concentration, to discard about a half amount of the existing medium, and to add about a half amount of a fresh medium containing a particular component at a concentration higher than the final concentration (half amount medium change operation) may be performed. When the concentration of a component contained in the existing medium is to be decreased by dilution at a certain time point, for example, the medium change operation may be performed plural times per day, preferably plural times (e.g., 2 - 3 times) within 1 hr. Also, when the concentration of a component contained in the existing medium is to be decreased by dilution at a certain time point, the cell or cell aggregate may be transferred to another culture container. While the tool used for the medium change operation is not particularly limited, for example, pipetter, pipetteman (registered trademark), multichannel pipetteman, and continuous dispenser, can be mentioned. For example, when a 96 well plate is used as a culture vessel, a multichannel pipetter may be used.

The period for culturing in the first step is generally about 8 hr to 6 days, preferably about 12 hr to 60 hr.

In the first step and the steps thereafter, from the aspect of improving the production efficiency of pituitary, it is also preferable to add a compound for promoting differentiation into a placodal region. As a compound having the above-mentioned action, for example, BRL-54443, Phenanthroline and Parthenolide described in US20160326491A1, and the like can be mentioned. When BRL-54443 is used as a compound for promoting differentiation into a placodal region, it is used at a concentration of generally about 10 nM to about 100 µM; when Phenanthroline is used, it is used at a concentration of generally about 10 nM to about 100 µM; and when Parthenolide is used, it is used at a concentration of generally about 10 nM to about 100 µM.

In the first step, from the aspect of improving the efficiency of differentiation induction into pituitary, culturing may also be performed in the presence of a Shh signal transduction pathway activating substance. As the Shh signal transduction pathway activating substance used in the first step, one similar to those exemplified in step (a) can be used. The Shh signal transduction pathway activating substances in step (a) and step (1) may be the same or different, preferably the same, and is preferably SAG.

The concentration of Shh signal transduction pathway activating substance in the medium can be appropriately determined according to the substances used within a range capable of achieving the aforementioned effects. When SAG is used as the Shh signal transduction pathway activating substance in the first step, it is generally used at a concentration of generally about 1 nM to about 3 µM, preferably about 10 nM to about 2 µM, more preferably about 30 nM to about 1 µM, further preferably about 50 nM to about 500 nM.

### <Step (2)>

In step (2), the cell population obtained in the first step is cultured in the presence of a BMP signal transduction pathway activating substance and a Shh signal transduction pathway activating substance. When cells are suspension cultured in the first step, the cell aggregates formed may be suspension cultured also in step (2). When cells are adhesion cultured in the first step, the cells may be continuously adhesion cultured also in step (2). After the cells are suspension cultured in the first step, they may be adhesion cultured in step (2).

The BMP signal transduction pathway activating substance is a substance capable of enhancing signal transduction mediated by BMP. Examples of the substance capable of enhancing signal transduction mediated by BMP include a substance that stabilizes BMP ligand in the culture environment and improves the titer, a substance that binds to type I BMP receptors ALK-1, ALK-2, ALK-3, and ALK-6 and activates and induces intracellular signal transduction downstream of the receptors, a substance that induces phosphorylation of Smad-1, Smad-5, Smad-8, and Smad-9 involved in intracellular BMP signal transduction, a substance that induces or enhances functions such as activation and suppression of gene transcription by Smad-1/5/8/9, and the like. Examples of the BMP signal transduction pathway activating substance include BMP proteins such as BMP2, BMP4, and BMP7, GDF proteins such as GDF5, 6 and 7, anti-BMP receptor antibody, BMP partial peptide, and the like. These substances may be used alone or in combination. As a definition of a BMP signal transduction pathway activating substance from the viewpoint of biological activity, for example, substances that have the ability to induce differentiation of cells such as the mouse pre-chondrogenic cell line ATDC5, mouse cranial vault-derived cell line MC3T3-E1, mouse striated muscle-derived cell line C2C12, and the like into osteoblast-like cells, and alkali phosphatase production induction potency. Examples of the substance with the above-mentioned activity include BMP2, BMP4, BMP5, BMP6, BMP7, BMP9, BMP10, BMP13/GDF6, BMP14/GDF5, GDF7 and the like.

BMP2 protein and BMP4 protein are available from, for example, R&D Systems, BMP7 protein is available from, for example, Biolegend, GDF5 protein is available from, for example, PeproTech, GDF6 protein is available from, for example, PrimeGene, and GDF7 protein is available from, for example, FUJIFILM Wako Pure Chemical Corporation. The BMP signal transduction pathway activating substance preferably contains at least one protein selected from the group consisting of BMP2, BMP4, BMP7, BMP13, and GDF7, more preferably BMP4.

The concentration of a BMP signal transduction pathway activating substance in the medium can be appropriately determined according to the substances used within a range capable of achieving the aforementioned effects. From the aspect of improving the production efficiency of cells constituting the pituitary, when BMP4 is used as a BMP signal transduction pathway activating substance, it is generally used at a concentration of about 1 pM to about 100 nM, preferably about 10 pM to about 50 nM, more preferably about 25 pM to about 25 nM, further preferably about 25 pM to about 5 nM, particularly preferably about 100 pM to about 5 nM, most preferably about 500 pM to about 2 nM. When a BMP signal transduction pathway activating substance other than BMP4 is used, it is desirably used at a concentration that shows BMP signal transduction pathway promoting activity equivalent to that of BMP4 at the aforementioned concentration. When using, for example, a commercially available recombinant BMP protein and the like as a BMP signal transduction pathway activating substance, those of ordinary skill in the art can easily determine the concentration of the BMP signal transduction pathway activating substance to be added by comparing the activity described in the product attachment, for example, the ED50 value of the ability to induce alkaline phosphatase production against the mouse pre-chondrogenic cell line ATDC5, and the concentration and activity of the aforementioned BMP4.

As the BMP signal transduction pathway activating substance, a compound well known to those of ordinary skill in the art can also be used. Examples of the BMP signal transduction pathway activating substance include Smurf1 inhibiting substance, Chk1 inhibiting substance, phosphorylation Smad stabilizing substance, and the like. Examples of the compound having the above-mentioned activity include A-01 ([4-[[4-Chloro-3-(trifluoromethyl)phenyl]sulfonyl]-1-piperazinyl] [4-(5-methyl-1H-pyrazol-1-yl)phenyl]methanone), PD407824 (9-Hydroxy-4-phenyl-pyrrolo[3,4-c]carbazole-1,3(2H,6H)-dione), SB4 (2-[[(4-Bromophenyl)methyl]thio]benzoxazole), SJ000291942 (2-(4-Ethylphenoxy)-N-(4-fluoro-3-nitrophenyl)-acetamide) and derivatives of these, and the like.

As the Shh signal transduction pathway activating substance used in step (2), one similar to those exemplified in step (a) can be used. The Shh signal transduction pathway activating substances in step (a) and step (2) may be the same or different, preferably the same, and is preferably SAG.

The concentration of Shh signal transduction pathway activating substance in the medium can be appropriately determined according to the substances used within a range capable of achieving the aforementioned effects. When SAG is used as the Shh signal transduction pathway activating substance in step (2), it is generally used at a concentration of about 1 nM - about 5 µM, preferably about 10 nM - about 4.5 µM, more preferably about 50 nM - about 4 µM, further preferably about 100 nM - about 3 µM.

The medium used in step (2) is not particularly limited as long as it contains a Shh signal transduction pathway activating substance and a BMP signal transduction pathway activating substance. The medium to be used in step (2) includes, for example, the media listed in the first step.

From the aspect of improving the production efficiency of the pituitary, the timing of starting step (2) is preferably from 0.5 hr to 6 days, more preferably 0.5 hr to 72 hr, further preferably 24 hr to 60 hr, from the start of culture in the first step. When performing suspension culturing, when step (2) is started during the above-mentioned period in the presence of a Wnt signal pathway inhibiting substance, a non-neural epithelial-like tissue is formed on the surface of the cell aggregate, and the pituitary can be formed extremely efficiently.

When suspension culturing is performed in the first step, the timing of starting step (2) from the aspect of improving the production efficiency of the cells constituting the pituitary is a period when 10% or more, more preferably 30% or more, further preferably 50% or more, of cells in the surface layer of the cell aggregate formed in the first step form tight junctions with each other. Those of ordinary skill in the art can easily determine whether tight junctions are formed in cell aggregates by, for example, microscopic observation or methods such as immunostaining using an anti-ZO-1 antibody, and the like.

To start the culture in the presence of a BMP signal transduction pathway activating substance in step (2), the above-mentioned medium exchange operation (e.g., medium addition operation, half-volume medium exchange operation, full-volume medium exchange operation, etc.) may be performed using the culture vessel in which the first step has been performed, or the cells may be transferred to another culture vessel.

The period of culturing in the medium containing the BMP signal transduction pathway activating substance in step (2) can be set as appropriate. The culture period in step (2) is generally not less than 8 hr, preferably not less than 10 hr, more preferably not less than 12 hr, further preferably not less than 14 hr, most preferably not less than 16 hr.

The period of culturing in the medium containing the Shh signal transduction pathway activating substance in step (2) can be set as appropriate. When the suspension culturing in step (1) is performed further in the presence of a Shh signal transduction pathway activating substance, the culture period in the presence of the Shh signal transduction pathway activating substance in step (1) and step (2) is preferably 30 days.

In step (2) and the steps thereafter, it is also preferable to add an FGF signal transduction pathway activating substance to the culture environment from the aspect of promoting differentiation into pituitary placode. The FGF signal transduction pathway activating substance is not particularly limited as long as it is a substance capable of enhancing the signal transduction pathway mediated by FGF (fibroblast growth factor). Examples of the FGF signal transduction pathway activating substance include FGF proteins such as FGF1, FGF2 (sometimes referred to as bFGF), FGF3, FGF8, FGF10, and the like, anti-FGF receptor antibody, FGF partial peptide and the like. These substances may be used alone or in combination.

FGF2 protein and FGF8 protein are available from, for example, FUJIFILM Wako Pure Chemical Corporation. The FGF signal transduction pathway activating substance preferably contains at least one selected from the group consisting of FGF2, FGF3, FGF8 and FGF10, and variant thereof, more preferably contains FGF2, further preferably contains recombinant human FGF2.

The concentration of FGF signal transduction pathway activating substance in the medium can be appropriately determined according to the substances used within a range capable of achieving the aforementioned effects. From the aspects of differentiation into cells constituting nasal cavity epithelium, and promotion of differentiation and survival and proliferation of the cell, when FGF2 is used as the FGF signal transduction pathway activating substance, it is used at a concentration of generally about 1 pg/ml - about 100 µg/ml, preferably about 10 pg/ml - about 50 µg/ml, more preferably about 100 pg/ml - about 10 µg/ml, further preferably about 500 pg/ml - about 1 µg/ml, most preferably about 1 ng/ml - about 200 ng/ml. When an FGF signal transduction pathway activating substance other than FGF2 is used, it is desirably used at a concentration showing FGF signal transduction pathway promoting activity equivalent to that of FGF2 at the above-mentioned concentration. The FGF signal transduction pathway promoting activity of a substance to be added can be measured by, for example, a method such as cell proliferation test using 3T3 cell and the like.

To retain the activity of FGF protein in a medium, it is also preferable to add heparin, heparan sulfate to the medium containing the FGF protein. Heparin is available as a sodium salt from, for example, FUJIFILM Wako Pure Chemical Corporation. The concentration of heparin or heparan sulfate in the medium can be appropriately determined to fall within a range capable of achieving the aforementioned effects. The concentration of heparin sodium in the medium is generally about 1 ng/ml - about 100 mg/ml, preferably about 10 ng/ml - about 50 mg/ml, more preferably about 100 ng/ml - about 10 mg/ml, further preferably about 500 ng/ml - about 1 mg/ml, most preferably about 1 µg/ml - about 200 µg/ml. When heparan sulfate is used, it is preferably a concentration showing FGF protein protecting activity equivalent to that of heparin at the above-mentioned concentration. To retain the activity of FGF protein under a cell culture environment such as 37°C, for example, it is also preferable to use modified FGF such as Thermostable FGF2 described in US8772460B2 or FGF2 sustained-release beads such as StemBeads FGF2 in which FGF2 is bound to a biodegradable polymer. Thermostable FGF2 is available from, for example, HumanZyme, inc. StemBeads FGF2 is available from, for example, StemCulture.

The timing of adding the FGF signal transduction pathway activating substance is added in step (2) and the steps thereafter can be set as appropriate. In preferred one embodiment, an FGF signal transduction pathway activating substance is added 6 hr, more preferably 12 hr, further preferably 18 hr, after the addition of the BMP signal transduction pathway activating substance in step (2).

It is also preferable to continuously add in step (2) the additives used in step (a) or the first step, such as JNK signal transduction pathway inhibiting substance, Wnt signal transduction pathway inhibiting substance, TGFβ signal transduction pathway inhibiting substance, TAK1 inhibiting substance, and the like. The JNK signal transduction pathway inhibiting substance, Wnt signal transduction pathway inhibiting substance, or TGFβ signal transduction pathway inhibiting substance to be added in step (2) may be different from the substance used in the previous steps but preferably the same. The concentration and kind of the additive can be appropriately adjusted. These substances may be added at the same time as the start of step (2) or at different times.

### <Step (b)>

The following step (b) may be further included after step (2). In step (b), the cell population obtained in step (2) is cultured under addition conditions of a BMP signal transduction pathway inhibiting substance. When cells are suspension cultured in step (2), the cell aggregates formed may be continuously suspension cultured also in step (b). When cells are adhesion cultured in step (2), the cells may be continuously adhesion cultured also in step (b).

The BMP signal transduction pathway inhibiting substance is not limited as long as it can suppress signal transduction induced by BMP family proteins. It may be any of nucleic acid, protein and low-molecular organic compound. Examples of the substance include a substance that inhibits BMP processing and extracellular secretion, a substance that directly acts on BMP (e.g., protein, antibody, and aptamer), a substance that suppresses expression of a gene encoding BMP (e.g., antisense oligonucleotide, and siRNA), a substance that suppresses binding of BMP receptor and BMP, and a substance that suppresses physiological activity caused by signal transduction by BMP receptor. The BMP receptor includes type I BMP receptor and type II BMP receptor. As the type I BMP receptor, BMPR1A, BMPR1B, and ACVR are known; as the type II BMP receptor, TGF-beta R-II, ActR-II, ActR-IIB, BMPR2, and MISR-II are known.

As a protein known as a BMP signal transduction pathway inhibiting substance, for example, Noggin, Chordin, Follistatin, Gremlin, Inhibin, Twisted Gastrulation, Coco, secretory protein belonging to the DAN family can be mentioned. In the above-mentioned step (2), a BMP signal transduction pathway activating substance is added to the culture medium. To more effectively inhibit the BMP signal transduction pathway thereafter, the BMP signal transduction pathway inhibiting substance in step (b) preferably contains a substance that inhibits a signal transduction pathway after the extracellular secretion of BMP, for example, a substance that inhibits the binding between BMP receptor and BMP, a substance that inhibits physiological activity caused by signal transduction by BMP receptors, more preferably an inhibitor of type I BMP receptor.

As the BMP signal transduction pathway inhibiting substance, a compound well known to those of ordinary skill in the art can also be used. Examples of the BMP signal transduction pathway inhibiting substance include an inhibiting substance of BMP type I receptor. Examples of the compound having the above-mentioned activity include K02288 (3-[(6-Amino-5-(3,4,5-trimethoxyphenyl)-3-pyridinyl]phenol, 3-[6-Amino-5-(3,4,5-trimethoxyphenyl)-3-pyridinyl]-phenol), Dorsomorphin (6-[4-[2-(1-Piperidinyl)ethoxy]phenyl]-3-(4-pyridinyl)pyrazolo[1,5-a]pyrimidine), LDN-193189 (4-[6-[4-(1-Piperazinyl)phenyl]pyrazolo[1,5-a]pyrimidin-3-yl]quinoline dihydrochloride), LDN-212854 (5-[6-[4-(1-Piperazinyl)phenyl]pyrazolo[1,5-a]pyriMidin-3-yl]quinoline), LDN-214117 (1-(4-(6-methyl-5-(3,4,5-trimethoxyphenyl)pyridin-3-yl)phenyl)piperazine), ML347(5-[6-(4-Methoxyphenyl)pyrazolo[1,5-a]pyrimidin-3-yl]quinoline)), DMH1 (4-(6-(4-Isopropoxyphenyl)pyrazolo[1,5-a]pyrimidin-3-yl)quinoline), DMH2 (4-[6-[4-[2-(4-Morpholinyl)ethoxy]phenyl]pyrazolo[1,5-a]pyrimidin-3-yl]-quinoline), compound 1 (3-(1,2,3-benzothiadiazol-6-yl)-1-[2-(cyclohex-1-en-1-yl)ethyl]urea), VU0465350 (7-(4-isopropoxyphenyl)-3-(1H-pyrazol-4-yl)imidazo[1,2-a]pyridine), VU0469381 (5-(6-(4-methoxyphenyl)pyrazolo[1,5-a]pyrimidin-3-yl)quinolone), OD36 (4-chloro-7,10-dioxa-13,17,18,21-tetrazatetracyclo[12.5.2.12,6.017,20]docosa-1(20),2(22),3,5,14(21),15,18-heptaene), OD52, E6201 ((3S,4R,5Z,8S,9S,11E)-14-(ethylamino)-8,9,16-trihydroxy-3,4-dimethyl-3,4,9,10-tetrahydro-1H-benzo[c][1]oxacyclotetradecine-1,7(8H)-dione), Saracatinib (N-(5-chloro-1,3-benzodioxol-4-yl)-7-[2-(4-methylpiperazin-1-yl)ethoxy]-5-(oxan-4-yloxy)quinazolin-4-amine), BYL719 ((2S)-1-N-[4-methyl-5-[2-(1,1,1-trifluoro-2-methylpropan-2-yl)pyridin-4-yl]-1,3-thiazol-2-yl]pyrrolidine-1,2-dicarboxamide), and the like. These substances may be used alone or in combination.

The BMP signal transduction pathway inhibiting substance is preferably BMP type I receptor inhibitor, more preferably contains at least one selected from the group consisting of K02288, Dorsomorphin, LDN-193189, LDN-212854, LDN-214117, ML347, DMH1, and DMH2, further preferably contains K02288 to LDN-193189.

The concentration of the BMP signal transduction pathway inhibiting substance in the medium can be appropriately determined to fall within a range capable of achieving the aforementioned effects and according to the substances to be used. From the aspect of pituitary tissue formation efficiency, when K02288 is used as a BMP signal transduction pathway inhibiting substance in step (b), it is used at a concentration of generally about 1 nM to about 100 µM, preferably about 10 nM to about 50 µM, more preferably about 100 nM to about 50 µM, further preferably about 500 nM to about 25 µM. When LDN-193189 is used as a BMP signal transduction pathway inhibiting substance, it is used at a concentration of generally about 1 nM to about 100 µM, preferably about 10 nM to about 10 µM, more preferably about 25 nM to about 1 µM, further preferably about 100 nM to about 500 nM. When LDN-212854 is used as a BMP signal transduction pathway inhibiting substance, it is used at a concentration of generally about 1 nM to about 100 µM, preferably about 10 nM to about 10 µM, more preferably about 25 nM to about 5 µM, further preferably about 250 nM to about 3 µM. When ML-347 is used as a BMP signal transduction pathway inhibiting substance, it is used at a concentration of generally about 1 nM to about 100 µM, preferably about 10 nM to about 50 µM, more preferably about 100 nM to about 50 µM, further preferably about 1 µM to about 25 µM. When DMH2 is used as a BMP signal transduction pathway inhibiting substance, it is used at a concentration of generally about 1 nM to about 100 µM, preferably about 10 nM to about 10 µM, more preferably about 25 nM to about 5 µM, further preferably about 250 nM to about 3 µM. When a BMP signal transduction pathway inhibiting substance other than K02288 is used, it is desirably used at a concentration showing BMP signal transduction pathway inhibitory activity equivalent to that of K02288 at the above-mentioned concentration.

The timing of starting step (b) after step (2) can be set as appropriate. The timing of starting step (b) is generally not less than 8 hr and within 15 days, preferably not less than 10 hr and within 12 days, more preferably not less than 12 hr and within 9 days, further preferably not less than 14 hr and within 8 days, most preferably not less than 16 hr and within 7 days, from the start of step (2).

In step (2) and the steps thereafter, the cell population may be treated with corticosteroids by adding corticosteroids to the medium. The treatment with corticosteroids promotes differentiation of pituitary placode and/or Rathke's pouch into pituitary hormone-producing cells other than ACTH-producing cells (i.e., GH-producing cells, PRL-producing cells, TSH-producing cells, LH-producing cells, FSH-producing cells, etc.). Examples of the corticosteroids include, but are not limited to, natural glucocorticoids such as hydrocortisone, cortisone acetate, fludrocortisone acetate, and the like; artificially-synthesized glucocorticoids such as dexamethasone, betamethasone, predonisolone, methylprednisolone, triamcinolone, and the like, and the like.

The concentration of corticosteroids in the medium is not particularly limited as long as it can promote differentiation of pituitary placode and/or Rathke's pouch into pituitary hormone-producing cells (excluding ACTH-producing cells). It can be appropriately determined according to the kind of the corticosteroids. For example, in the case of hydrocortisone, it is generally 100 ng/ml or more, preferably 1 µg/ml or more. The upper limit of the hydrocortisone concentration is not particularly set as long as differentiation into pituitary hormone-producing cells (excluding ACTH-producing cells) is not adversely affected. From the aspect of culture cost, it is generally 1000 µg/ml or less, preferably 100 µg/ml or less. In one embodiment, the concentration of hydrocortisone in the medium is generally about 100 ng/ml to about 1000 µg/ml, preferably about 1 to about 100 µg/ml. When dexamethasone is used as the corticosteroids, the concentration thereof in the medium can be set to about 1/25 that of hydrocortisone.

In step (2) and the steps thereafter, the timing of adding corticosteroids to the medium is not particularly limited as long as it can promote differentiation of pituitary placode and/or Rathke's pouch into pituitary hormone-producing cells (excluding ACTH-producing cells). Corticosteroids may be added to the medium from the start of the second step, or corticosteroids may be added to the medium after culturing for a certain period of time in a medium free of addition of corticosteroids after the start of the second step. Preferably, after the start of the second step, corticosteroids are added to the medium when the emergence of ACTH-producing cells is confirmed in the cell population. That is, the cell aggregates are cultured in a medium free of addition of corticosteroids until the emergence of ACTH-producing cells is confirmed in the cell aggregates, and the step (2) and the steps thereafter are continued in a medium containing corticosteroids, after the emergence of ACTH-producing cells is confirmed. The emergence of ACTH-producing cells can be confirmed by immunohistological staining using an antibody against ACTH. When human pluripotent stem cells are used, the emergence of ACTH-producing cells can be generally expected after 30 days from the start of the first step. In one embodiment, therefore, corticosteroids are added to the medium after 30 days from the start of the first step.

The period of treatment of cell aggregates with corticosteroids is not particularly limited as long as it can promote differentiation of pituitary placode and/or Rathke's pouch into pituitary hormone-producing cells (excluding ACTH-producing cells). In general, cell aggregates are treated with corticosteroids until promotion of differentiation into pituitary hormone-producing cells (excluding ACTH-producing cells) is confirmed in the corticosteroid treatment group as compared with a corticosteroid non-treatment group. The treatment period is generally 7 days or more, preferably 12 days or more. The upper limit of the treatment period is not particularly set and the corticosteroids may be removed from the medium at the stage when promotion of differentiation into pituitary hormone-producing cells (excluding ACTH-producing cells) is confirmed in the corticosteroid treatment group as compared with the corticosteroid non-treatment group.

Step (2) and the steps thereafter are also preferably performed in the presence of a retinoic acid signal transduction pathway activating substance, from the aspect of promoting differentiation into pituitary and differentiation into growth hormone-producing cells. Examples of the retinoic acid transduction pathway activating substance include substances that bind to retinoic acid receptor (RAR) or retinoid X receptor (RXR) and activate transcription in the downstream and the like. Examples of the compound having the above-mentioned action include all-trans-retinoic acid, isotretinoin, 9-cis retinoic acid, TTNPB (4-[(E)-2-[(5,5,8,8-Tetramethyl-5,6,7,8-tetrahydronaphthalene)-2-yl]-1-propenyl]benzoic acid), Ch55 (4-[(E)-3-(3,5-di-tert-butylphenyl)-3-oxo-1-propenyl]benzoic acid), EC19 (3-[2-(5,6,7,8-Tetrahydro-5,5,8,8-tetramethyl-2-naphthalenyl)ethynyl]benzoic acid), EC23 (4-[2-(5,6,7,8-Tetrahydro-5,5,8,8-tetramethyl-2-naphthalenyl)ethynyl)-benzoicacid), Fenretinide (4-hydroxyphenylretinamide), Acitretin ((all-e)-9-(4-methoxy-2,3,6-trimethylphenyl)-3,7-dimethyl-2,4,6,8-nonatetraen), Trifarotene, Adapalene, AC 261066 (4-[4-(2-Butoxyethoxy-)-5-methyl-2-thiazolyl]-2-fluorobenzoicacid), AC 55649 (4-N-Octylbiphenyl-4-carboxylic acid), AM 580 (4-[(5,6,7,8-Tetrahydro-5,5,8,8-tetramethyl-2-naphthalenyl)carboxamido]benzoic acid), AM80 (4-[(5,5,8,8-Tetramethyl-6,7-dihydronaphthalen-2-yl)carbamoyl]benzoic acid), BMS 753 (4-[[(2,3-Dihydro-1,1,3,3-tetramethyl-2-oxo-1H-inden-5-yl)carbonyl]amino]benzoicacid), BMS 961 (3-Fluoro-4-[(r)-2-hydroxy-2-(5,5,8,8-tetramethyl-5,6,7,8-tetrahydro-naphthalen-2-yl)-acetylamino]-benzoic acid), CD1530 (4-(6-Hydroxy-7-tricyclo[3.3.1.13,7]dec-1-yl-2-naphthalenyl)benzoicacid), CD2314 (5-(5,6,7,8-Tetrahydro-5,5,8,8-tetramethyl-2-anthracenyl)-3-thiophenecarboxylic acid), CD437 (2-naphthalenecarboxylicacid,6-(4-hydroxy-3-tricyclo(3.3.1.1(3,7))dec-1-ylphen), CD271 (6-[3-(1-Adamantyl)-4-methoxyphenyl]-2-naphthalene carboxylic acid) and derivatives of these, and the like. These substances may be used alone or in combination.

The retinoic acid transduction pathway activating substance in step (2) and the steps thereafter preferably contains all-trans-retinoic acid or EC23. The concentration of retinoic acid transduction pathway activating substance in the medium is not particularly limited as long as the aforementioned effects can be achieved. When EC23 is used as the retinoic acid transduction pathway activating substance, for example, the concentration of EC23 is about 10 pM to about 30 µM, preferably about 100 pM to about 20 µM, more preferably about 10 nM to about 10 µM, further preferably about 100 nM to about 5 µM. When a retinoic acid transduction pathway activating substance other than EC23 is used, it is desirably used at a concentration showing retinoic acid transduction pathway action activity equivalent to that of EC23 at the above-mentioned concentration.

From the aspect of regulating the differentiation tendency, it is also preferable to perform step (2) and the steps thereafter in the presence of a Notch signal transduction pathway inhibiting substance. In the present invention, the Notch signal transduction pathway shows a signal transduction pathway activated by direct interaction between Notch protein, which is a receptor expressed on a cell membrane, and Notch ligand (Delta, Jagged, etc.) expressed on the membrane of adjacent cells. In cells in which a Notch signal is transmitted, the Notch protein is stepwisely processed and the intracellular domain excised on the membrane is transported into the nucleus and controls the expression of downstream genes.

The Notch signal transduction pathway inhibiting substance is not particularly limited as long as it can suppress signal transduction mediated by Notch. It may be any of nucleic acid, protein and low-molecular organic compound. Examples of the substance include functionally deficient Notch receptor and ligand, substances that inhibit Notch processing (S1 cleavage), substances that inhibit sugar chain modification of Notch and Notch ligand, substances that inhibit cell membrane transfer, substances (γ-secretase inhibitors) that inhibit processing (S2 cleavage, S3 cleavage) of Notch intracellular domain (NICD) , substances that decompose NICD, substances that inhibit NICD-dependent transcription, and the like.

As the Notch signal transduction pathway inhibiting substance, a compound well known to those of ordinary skill in the art can also be used. As a compound having the activity as a Notch signal transduction pathway inhibiting substance, for example, DAPT (N-[N-(3,5-difluorophenacetyl)-1-alanyl]-S-phenylglycine t-butyl ester), DBZ ((2S)-2-[[2-(3,5-difluorophenyl)acetyl]amino]-N-[(7S)-5-methyl-6-oxo-7H-benzo[d][1]benzazepin-7-yl]propanamide), MDL28170 (benzyl N-[(2S)-3-methyl-1-oxo-1-[[(2S)-1-oxo-3-phenylpropan-2-yl]amino]butan-2-yl]carbamate), FLI-06 (cyclohexyl 2,7,7-trimethyl-4-(4-nitrophenyl)-5-oxo-1,4,6,8-tetrahydroquinoline-3-carboxylate), L-685,458 (tert-butyl N-[6-[[1-[(1-amino-1-oxo-3-phenylpropan-2-yl)amino]-4-methyl-1-oxopentan-2-yl]amino]-5-benzyl-3-hydroxy-6-oxo-1-phenylhexan-2-yl]carbamate), CB-103 (6-(4-tert-butylphenoxy)pyridin-3-amine) and derivatives of these, the substance described in Onco Targets Ther.2013; 6:943-955, and the like can be mentioned. The Notch signal transduction pathway inhibiting substance preferably contains DAPT.

The concentration of the Notch signal transduction pathway inhibiting substance in the medium is not particularly limited as long as it is within a range capable of achieving the aforementioned effects. For example, when DAPT is used as the Notch signal transduction pathway inhibiting substance, for example, the concentration of DAPT is about 100 pM to about 50 µM, preferably about 1 nM to about 30 µM, more preferably about 100 nM to about 20 µM, further preferably about 1 µM to about 10 µM. When a Notch signal transduction pathway inhibiting substance other than DAPT is used, it is desirably used at a concentration showing Notch signal transduction pathway inhibitory activity equivalent to that of DAPT at the above-mentioned concentration.

### <Step (3)>, <step (3')>: the third step

In the third step, the cell population cultured in step (2) or step (b) is cultured under non-addition conditions of a Shh signal transduction pathway activating substance. The third step in which the cell population cultured in the second step is cultured in the absence of a Shh transduction pathway activating substance to obtain a cell population containing pituitary tissue is step (3), and the third step in which the cell population cultured in step (b) is cultured in the absence of a Shh signal transduction pathway activating substance to obtain a cell population containing pituitary tissue is step (3'). When cells are suspension cultured in step (2) or step (b), the cell aggregates formed may be suspension cultured in the third step. When cells are adhesion cultured in step (2) or step (b), the cells may be continuously adhesion cultured also in the third step. After the cells are suspension cultured in step (2) or step (b), they may be adhesion cultured in the third step.

The medium used in the third step is not particularly limited as long as it does not contain a Shh signal transduction pathway activating substance. The non-addition condition of a Shh signal transduction pathway activating substance in this step refers to a condition in which a Shh signal transduction pathway activating substance is not intentionally added to the culture environment of the cell population, and the non-addition condition of a Shh signal transduction pathway activating substance includes even when a Shh signal transduction pathway activating substance is unintentionally included in the culture environment due to autocrine secretion by a cell population. Examples of the medium used in the third step include the medium listed in the first step, the gfCDM medium containing 10% to 20% KSR, and the like.

The third step and the steps thereafter may include a step of embedding and culturing the cell aggregate in a gel, from the aspect of promoting survival and differentiation maturation of cells constituting the pituitary. Examples of the gel include gels using agarose, methylcellulose, collagen, Matrigel, and the like, and it is preferable to use Matrigel.

In the step of embedding and culturing the cell in a gel in the third step and the steps thereafter, the cell aggregate may be embedded as it is or the cells after dispersion and isolation may be seeded in the gel. The cells may be seeded after sorting out specific cell tumors such as basal cell by using a cell sorter or the like. As a further embodiment of the culture method of embedding in a gel, co-culture with cells other than the pituitary such as fibroblast, mesenchymal cell, vascular cell, and the like can also be performed. Gel-embedding culture as described above can be performed with reference to, for example, Nature 501, 373-379 (2013), Nature, 499, 481-484 (2013), Nat Protoc 14, 518-540 (2019), Genes 2020, 11, 603, and the like.

In the third step and the steps thereafter, it is also preferable to perform a culture method in which the cells are physically shaken, for the purpose of improving nutrition and oxygen supply to the cells and improving substance exchange. Examples of such culture method include methods other than static culture, such as shaking culture, rotation culture, stirring culture, and the like. The means for performing shaking culture, rotation culture, stirring culture, and the like are not particularly limited. For example, they can be performed by placing the culture vessel, in which the cells are cultured, on a rotator, shaker, or the like, or by placing the cells in an environment where a stirrer or the like is rotating. Those skilled in the art can appropriately set the parameters such as the speed and the like of shaking culture, rotation culture, and stirring culture within a range that does not cause damage to cells. For example, when shaking culture is performed using a waveform fluctuation 3D shaker (e.g., Mini-Shaker 3D, manufactured by Biosan), for example, the shaking speed range can be set within the range of 5 to 60 rpm, preferably 5 to 40 rpm, more preferably 5 to 20 rpm. When shaking culture is performed using a reciprocating type shaker (e.g., NS-LR, manufactured by AS ONE Corporation), for example, the shaking speed range can be set within the range of 15 to 60 rpm, preferably 15 to 50 rpm, more preferably 15 to 45 rpm. When shaking culture is performed using a seesaw type shaker (e.g., NS-S, manufactured by AS ONE Corporation), for example, the shaking speed range can be set within the range of 5 to 50 rpm, preferably 5 to 40 rpm, more preferably 5 to 30 rpm. When cell aggregates are cultured by shaking culture or rotation culture, for example, a spinner flask (e.g., 3152, manufactured by Corning Incorporated) is set on a magnetic stirrer, and culture can also be performed at a rotation speed at which cell aggregates do not form sediment visually. Culture can also be performed using a three-dimensional rotating suspension culture device (e.g., CellPet CUBE, manufactured by JTEC Corporation; Clinostar, manufactured by Celvivo). From the aspect of suppressing physical damage such as friction to cells and the like, it is also preferable to culture the aforementioned cell aggregates embedded in the gel by shaking culture, rotation culture, stirring culture.

In the third step (3) and the steps thereafter, it is also preferable to perform culturing under a high oxygen atmosphere from the aspect of suppression of cell death and promotion cell proliferation. The high oxygen conditions in the culturing process can be realized, for example, by connecting an oxygen cylinder to an incubator for culturing cells and artificially supplying oxygen. The oxygen concentration for such purpose is generally 25% to 80%, more preferably 30% to 60%.

In the third step and the steps thereafter, it is possible to use a culture vessel with high gas exchange efficiency from the aspect of increasing the amount of oxygen supply to the medium for culturing cell aggregates. Examples of such culture vessel include cell culture dish, Lumoxdish with a gas permeable film as bottom surface of the plate (manufactured by Sarstedt K.K.), VECELL 96 well plate (manufactured by Vessel CO. LTD.) and the like. It is also preferable to use same in combination with culture under the aforementioned high oxygen concentration conditions.

In the third step and the steps thereafter, from the aspect of maintaining the structure of epithelial tissue in the cell aggregate, a cell protectant can also be added to the medium. As the cell protectant to be used in step (3) and the steps thereafter, the aforementioned FGF signal transduction pathway activating substance, heparin, ROCK inhibiting substance, basement membrane preparation, myosin inhibiting substance, polyamines, ISR inhibitor, caspase inhibitor, serum, serum replacement, and the like can be mentioned. As the myosin inhibiting substance, for example, Blebbistatin as an inhibiting substance of nonmuscle myosin II ATPase, ML-7, ML-9, and W-7 as inhibiting substance of myosin light chain kinase (MLCK), MLCK inhibitor peptide 18, derivatives of these, and the like can be mentioned. The cell protectant to be added may be different from the one added in the first step, but is preferably the same. As a preferred cell protectant, ROCK inhibiting substance can be mentioned. In the third step and the steps thereafter, when Y-27632, which is a ROCK inhibiting substance, is added as a cell protectant, it is added into the culture environment to achieve a concentration of generally about 10 nM to about 10 mM, preferably about 100 nM to about 1 mM, more preferably about 1 µM to about 100 µM. When Chroman 1, which is a ROCK inhibiting substance, is added, it is added into the culture environment to achieve a concentration of generally about 10 pM to about 1 mM, preferably about 100 pM to about 100 µM, more preferably about 1 nM to about 10 µM. When Blebbistatin, which is an inhibiting substance of nonmuscle myosin II ATPase, is added as a cell protectant, it is added into the culture environment to achieve a concentration of generally about 10 nM to about 10 mM, preferably about 100 nM to about 1 mM, more preferably about 1 µM to about 100 µM.

In the third step and the steps thereafter, a substance other than cell protectant that has the action of maintaining the structure of epithelial tissue can also be added. Examples of the above-mentioned substance include substances that promote cell adhesion, substances that promote the synthesis of base membrane components, substances that inhibit the decomposition of base membrane components, and the like. The substances that promote cell adhesion may be those that promote any of cell-to-cell adhesion, cell-to-base membrane adhesion, cell-to-culture vessel adhesion, and the like or the production of factors involved in cell adhesion. For example, as the substances that promote cell adhesion, Adhesamine, Adhesamine-RGDS derivative, Pyrintegrin, Biotin tripeptide-1, Acetyl Tetrapeptide-3, RGDS Peptide and derivatives of these, and the like can be mentioned. For example, as the substances that promote synthesis of base membrane components, ascorbic acid derivative and the like can be mentioned. Examples of the ascorbic acid derivative include sodium ascorbate phosphate, magnesium ascorbate phosphate, ascorbic acid 2-glucoside, 3-O-ethyl ascorbic acid, tetrahexyldecanic acid ascorbyl, ascorbyl palmitate, ascorbyl stearate, ascorbyl 2-phosphate 6-palmitate, glyceryl octyl ascorbic acid, and the like. As the substances that inhibit decomposition of the base membrane components, for example, inhibitors of matrix metalloprotease and serine protease and the like can be mentioned. When ascorbic acid 2-phosphate, which is one kind of ascorbic acid derivative which is a substance that promotes synthesis of base membrane components, is added, it is added into the culture environment to achieve a concentration of generally not less than 10 pg/ml and not more than 1000 µg/ml, preferably not less than 30 µg/ml and not more than 500 µg/ml, further preferably not less than 50 µg/ml and not more than 300 µg/ml. When other ascorbic acid, ascorbic acid derivatives, and the like are added, they may be added so that their molar equivalents are approximately the same as the above-mentioned concentrations.

In the third step and the steps thereafter, from the aspect of promoting the survival of pituitary cells, it is also preferable to add a substance having an action of reducing oxidative stress. As substances with the above-mentioned activity, for example, antioxidants, substances with free radical scavenging action, NADPH oxidase inhibiting substances, cyclooxygenase inhibiting substance, lipoxygenase (LOX) inhibiting substance, superoxide dismutase (SOD)-like substances, Nrf2 activator, and the like can be mentioned. Examples of the substance having the above-mentioned activity include, but are not limited to, ascorbic acid, N-acetyl-L-cysteine, (±)-α-tocopherol acetate, Apocynin (4'-Hydroxy-3'-methoxyacetophenone), nicotine amide, taurine (2-aminoethanesulfonic acid), IM-93 (1-Isopropyl-3-(1-methyl-1H-Indol-3-yl)-4-(N,N-dimethyl-1,3-propanediamine)-1H-Pyrrole-2, 5H-dione), Caffeic Acid (3,4-Dihydroxycinnamic Acid), Celastrol (3-Hydroxy-24-nor-2-oxo-1(10),3,5,7-friedelatetraen-29-oic Acid; Tripterin), Ebselen (2-Phenyl-1,2-benzisoselenazol-3(2H)-one), (-)-Epigallocatechin Gallate((2R,3R)-2-(3,4,5-Trihydroxyphenyl)-3,4-dihydro-1[2H]-benzopyran-3,5,7-triol-3-(3,4,5-trihydroxybenzoate)), EUK-8 (N,N'-Bis(salicylideneamino)ethane-manganese(II)), Edaravone (3-Methyl-1-phenyl-2-pyrazolin-5-one), MnTBAP (Mn(III)tetrakis(4-benzoic acid)porphyrin Chloride), Nordihydroguaiaretic Acid, Resveratrol (trans-3,4,5-Trihydroxystilbene) and derivatives of these, and the like. Reagents prepared for cell culture (e.g., antioxidant supplement, manufactured by Sigma Aldrich, A1345) can also be used. The substance having an action of reducing oxidative stress to be used in the present invention preferably contains at least one selected from the group consisting of ascorbic acid, N-acetyl-L-cysteine, and derivatives of these. Ascorbic acid can be added to the medium at a concentration of, for example, as ascorbic acid 2 phosphate (derivative thereof), about 1 nM to about 1 M, preferably about 10 nM to about 100 mM, more preferably about 100 nM to about 10 mM, further preferably about 1 µM to about 3 mM, and N-acetyl-L-cysteine can be added to the medium at a concentration of, for example, about 1 nM to about 1 M, preferably about 10 nM to about 100 mM, more preferably about 100 nM to about 10 mM, further preferably about 1 µM to about 5 mM.

In the third step and the steps thereafter, from the aspect of promoting survival of the pituitary cell, it is also preferable to add a substance that inhibits stress-response signal transduction pathway (substance that inhibits intracellular signal transduction mechanism for the stress). The stress-responsive MAP kinase pathway (stress-activated protein kinase: SAPK) is one of the major intracellular signal transduction mechanisms against stress. As inhibitors of stress-responsive MAP kinase pathway, for example, MAP3K inhibitor, MAP2K inhibitor, ASK inhibitor, MEK inhibitor, Akt inhibitor, Rho family kinase inhibitor, JNK inhibitor, p38 inhibitor, MSK inhibitor, STAT inhibitor, NF-κB inhibitor, CAMK inhibitor and the like can be mentioned.

Examples of the MEK inhibitor include Selumetinib (AZD6244, 6-(4-bromo-2-chloroanilino)-7-fluoro-N-(2-hydroxyethoxy)-3-methylbenzimidazole-5-carboxamide), Mirdametinib (PD0325901, N-[(2R)-2,3-dihydroxypropoxy]-3,4-difluoro-2-(2-fluoro-4-iodoanilino)benzamide), Trametinib (GSK1120212, N-[3-[3-cyclopropyl-5-(2-fluoro-4-iodoanilino)-6,8-dimethyl-2,4,7-trioxopyrido[4,3-d]pyrimidin-1-yl]phenyl]acetamide), U0126 (1,4-diamino-2,3-dicyano-1,4-bis(2-aminophenylthio)butadiene), PD184352 (CI-1040, 2-(2-chloro-4-iodoanilino)-N-(cyclopropylmethoxy)-3,4-difluorobenzamide), PD98059 (2-(2-amino-3-methoxyphenyl)chromen-4-one), BIX 02189 (3-[N-[3-[(dimethylamino)methyl]phenyl]-C-phenylcarbonimidoyl]-2-hydroxy-N,N-dimethyl-1H-indole-6-carboxamide), Pimasertib (AS-703026, N-[(2S)-2,3-dihydroxypropyl]-3-(2-fluoro-4-iodoanilino)pyridine-4-carboxamide), Pelitinib (EKB-569, (E)-N-[4-(3-chloro-4-fluoroanilino)-3-cyano-7-ethoxyquinolin-6-yl]-4-(dimethylamino)but-2-enamide), BIX 02188 (3-[N-[3-[(dimethylamino)methyl]phenyl]-C-phenylcarbonimidoyl]-2-hydroxy-1H-indole-6-carboxamide), TAK-733 (3-[(2R)-2,3-dihydroxypropyl]-6-fluoro-5-(2-fluoro-4-iodoanilino)-8-methylpyrido[2,3-d]pyrimidine-4,7-dione), AZD8330 (2-(2-fluoro-4-iodoanilino)-N-(2-hydroxyethoxy)-1,5-dimethyl-6-oxopyridine-3-carboxamide), Binimetinib (MEK162, 6-(4-bromo-2-fluoroanilino)-7-fluoro-N-(2-hydroxyethoxy)-3-methylbenzimidazole-5-carboxamide), SL-327 ((Z)-3-amino-3-(4-aminophenyl)sulfanyl-2-[2-(trifluoromethyl)phenyl]prop-2-enenitrile), Refametinib (RDEA119, N-[3,4-difluoro-2-(2-fluoro-4-iodoanilino)-6-methoxyphenyl]-1-[(2S)-2,3-dihydroxypropylJcyclopropane-1-sulfonamide), GDC-0623 (5-(2-fluoro-4-iodoanilino)-N-(2-hydroxyethoxy)imidazo[1,5-a]pyridine-6-carboxamide), BI-847325 (3-[3-[N-[4-[(dimethylamino)methyl]phenyl]-C-phenylcarbonimidoyl]-2-hydroxy-1H-indol-6-yl]-N-ethylprop-2-ynamide), RO5126766 (CH5126766, 3-[[3-fluoro-2-(methylsulfamoylamino)pyridin-4-yl]methyl]-4-methyl-7-pyrimidin-2-yloxychromen-2-one), Cobimetinib (GDC-0973, [3,4-difluoro-2-(2-fluoro-4-iodoanilino)phenyl]-[3-hydroxy-3-[(2S)-piperidin-2-yl]azetidin-1-yl]methanone) and derivatives of these, and the like.

Examples of the p38 inhibitor include SB203580 (4-[4-(4-fluorophenyl)-2-(4-methylsulfinylphenyl)-1H-imidazol-5-yl]pyridine), Doramapimod (BIRB 796, 1-[5-tert-butyl-2-(4-methylphenyl)pyrazol-3-yl]-3-[4-(2-morpholin-4-ylethoxy)naphthalen-1-yl]urea), SB202190 (FHPI, 4-[4-(4-fluorophenyl)-5-pyridin-4-yl-1H-imidazol-2-yl]phenol), Ralimetinib dimesylate (5-[2-tert-butyl-4-(4-fluorophenyl)-1H-imidazol-5-yl]-3-(2,2-dimethylpropyl)imidazo[4,5-b]pyridin-2-amine; methanesulfonic acid), VX-702 (6-(N-carbamoyl-2,6-difluoroanilino)-2-(2,4-difluorophenyl)pyridine-3-carboxamide), PH-797804 (3-[3-bromo-4-[(2,4-difluorophenyl)methoxy]-6-methyl-2-oxopyridin-1-yl]-N,4-dimethylbenzamide), Neflamapimod (VX-745, 5-(2,6-dichlorophenyl)-2-(2,4-difluorophenyl)sulfanylpyrimido[1,6-b]pyridazin-6-one), TAK-715 (N-[4-[2-ethyl-4-(3-methylphenyl)-1,3-thiazol-5-yl]pyridin-2-yl]benzamide), PD 169316 (4-[4-(4-fluorophenyl)-2-(4-nitrophenyl)-1H-imidazol-5-yl]pyridine), TA-02 (4-[2-(2-fluorophenyl)-4-(4-fluorophenyl)-1H-imidazol-5-yl]pyridine), SD 0006 (1-[4-[3-(4-chlorophenyl)-4-pyrimidin-4-yl-1H-pyrazol-5-yl]piperidin-1-yl]-2-hydroxyethanone), Pamapimod (6-(2,4-difluorophenoxy)-2-(1,5-dihydroxypentan-3-ylamino)-8-methylpyrido[2,3-d]pyrimidin-7-one), BMS-582949 (4-[5-(cyclopropylcarbamoyl)-2-methylanilino]-5-methyl-N-propylpyrrolo[2,1-f][1,2,4]triazine-6-carboxamide), SB239063 (4-[4-(4-fluorophenyl)-5-(2-methoxypyrimidin-4-yl)imidazol-1-yl]cyclohexan-1-ol), Skepinone-L (13-(2,4-difluoroanilino)-5-[(2R)-2,3-dihydroxypropoxy]tricyclo[9.4.0.03,8]pentadeca-1(11),3(8),4,6,12,14-hexaen-2-one), DBM 1285 (N-cyclopropyl-4-[4-(4-fluorophenyl)-2-piperidin-4-yl-1,3-thiazol-5-yl]pyrimidin-2-amine; dihydrochloride), SB 706504 (1-cyano-2-[2-[[8-(2,6-difluorophenyl)-4-(4-fluoro-2-methylphenyl)-7-oxopyrido[2,3-d]pyrimidin-2-yl]amino]ethyl]guanidine), SCIO 469 (2-[6-chloro-5-[(2R,5S)-4-[(4-fluorophenyl)methyl]-2,5-dimethylpiperazine-1-carbonyl]-1-methylindol-3-yl]-N,N-dimethyl-2-oxoacetamide), Pexmetinib (1-[5-tert-butyl-2-(4-methylphenyl)pyrazol-3-yl]-3-[[5-fluoro-2-[1-(2-hydroxyethyl)indazol-5-yl]oxyphenyl]methyl]urea), UM-164 (2-[[6-[4-(2-hydroxyethyl)piperazin-1-yl]-2-methylpyrimidin-4-yl]amino]-N-[2-methyl-5-[[3-(trifluoromethyl)benzoyl]amino]phenyl]-1,3-thiazole-5-carboxamide), p38 MAPK Inhibitor (4-(2,4-difluorophenyl)-8-(2-methylphenyl)-7-oxido-1,7-naphthyridin-7-ium), p38 MAP Kinase Inhibitor III (4-[5-(4-fluorophenyl)-2-methylsulfanyl-1H-imidazol-4-yl]-N-(1-phenylethyl)pyridin-2-amine), p38 MAP Kinase Inhibitor IV (3,4,6-trichloro-2-(2,3,5-trichloro-6-hydroxyphenyl)sulfonylphenol), CAY105571 (4-[5-(4-fluorophenyl)-2-[4-(methylsulfonyl)phenyl]-1H-imidazol-4-yl]-pyridine) and derivatives of these, and the like.

As the JNK inhibitor, for example, those similar to the ones described in the first step can be mentioned. The substance that inhibits the intracellular signal transduction mechanism against stress to be used in the present invention is preferably one or more selected from the group consisting of MEK inhibitor, p38 inhibitor, and JNK inhibitor. When SB203580 is used as the p38 inhibitor, it can be added to the medium at a concentration of generally about 1 nM to about 1 mM, preferably about 10 nM to about 100 µM, more preferably about 100 nM to about 10 µM, further preferably about 500 nM to about 5 µM. When PD0325901 is used as the MEK inhibitor, it can be added to the medium at a concentration of generally about 1 nM to about 1 mM, preferably about 10 nM to about 100 µM, more preferably about 100 nM to about 10 µM, further preferably about 500 nM to about 5 µM. When JNK-IN-8 is used as the JNK inhibitor, it can be added to the medium at a concentration similar to that described in the first step. When other MEK inhibitor, p38 inhibitor, or JNK inhibitor is used, it is preferably used at a concentration showing inhibitory activity equivalent to that of the addition concentration of the above-mentioned inhibitor.

In step (A), the pluripotent stem cells to be cultured under the conditions permitting differentiation induction into a cell aggregate containing a hypothalamic cell, a pituitary progenitor cell, and a pituitary hormone-producing cell may be an aggregate of pluripotent stem cells. When pluripotent stem cell aggregates are cultured in the presence of a differentiation-inducing factor, for example, they can be cultured according to the method described in WO 2016/013669.

The culture period in step (A) is a period in which the cell aggregate containing a hypothalamic cell, a pituitary progenitor cell, and a pituitary hormone-producing cell obtained in step (A) contains pituitary hormone-producing cells that secrete ACTH and does not contain pituitary hormone-producing cells that secrete GH or TSH. The cell aggregate containing a hypothalamic cell, a pituitary progenitor cell, and a pituitary hormone-producing cell obtained in step (A) may contain other pituitary hormone-producing cells as long as it contains pituitary hormone-producing cells that secrete ACTH and does not contain pituitary hormone-producing cells that secrete GH or TSH, and is not particularly limited. For example, it may contain at least one type of pituitary hormone-producing cell selected from the group consisting of pituitary hormone-producing cells that secrete PRL, pituitary hormone-producing cells that secrete LH, and pituitary hormone-producing cells that secrete FSH. Examples of such cell aggregate include (i) cell aggregate containing pituitary hormone-producing cells that secrete ACTH and pituitary hormone-producing cells that secrete PRL, and not containing pituitary hormone-producing cells that secrete GH or TSH, (ii) cell aggregate containing pituitary hormone-producing cells that secrete ACTH, pituitary hormone-producing cells that secrete PRL, and pituitary hormone-producing cells that secrete LH, and not containing pituitary hormone-producing cells that secrete GH or TSH, (iii) cell aggregate containing pituitary hormone-producing cells that secrete ACTH, pituitary hormone-producing cells that secrete PRL, and pituitary hormone-producing cells that secrete FSH, and not containing pituitary hormone-producing cells that secrete GH or TSH, (iv) cell aggregate containing pituitary hormone-producing cells that secrete ACTH, pituitary hormone-producing cells that secrete PRL, pituitary hormone-producing cells that secrete LH, and pituitary hormone-producing cells that secrete FSH, and not containing pituitary hormone-producing cells that secrete GH or TSH, and the like. Therefore, the culture period in step (A) may be a period in which the cell aggregates of the above-mentioned (i) to (iv) are obtained.

Whether or not the cell aggregate containing a hypothalamic cell, a pituitary progenitor cell, and a pituitary hormone-producing cell obtained in step (A) contains pituitary hormone-producing cells can be confirmed by a known means and, for example, can be detected by a method such as immunohistochemistry using GH producing cell marker (anti-Pit1 antibody, anti-GH antibody, etc.), PRL producing cell marker (anti-Pit1 antibody, anti-PRL antibody, etc.), ACTH producing cell marker (anti-T-Pit antibody, anti-NeuroD1 antibody, anti-ACTH antibody, etc.), TSH producing cell marker (anti-GATA2 antibody, anti-TSH antibody, etc.), FSH producing cell and LH producing cell marker (anti-GATA2 antibody, anti-SF1 antibody, anti-FSH antibody, anti-LH antibody, etc.), ELISA for secreted hormones, and the like.

When a pluripotent stem cell or a cell aggregate thereof is cultured under conditions permitting differentiation induction into a cell aggregate containing a hypothalamic cell, a pituitary progenitor cell, and a pituitary hormone-producing cell, pituitary hormone-producing cells that secrete ACTH begin to appear about 30 days after the start of differentiation induction. In addition, pituitary hormone-producing cells that secrete GH or TSH begin to appear about 100 days after the start of differentiation induction. Therefore, the culture period of step (A) may be not less than about 30 days and not more than about 100 days. It is preferably not less than about 60 days and not more than about 100 days, since differentiation into pituitary hormone-producing cells that secrete ACTH proceeds. A culture period of step (A) of not less than about 60 days and not more than about 100 days is preferred since a cell aggregate containing pituitary hormone-producing cells that secrete ACTH is always present and the ACTH secretory capacity is not less than 0.5 pg/mL (early stage pituitary tissue: not less than 0.5 pg/mL, middle stage: not less than 2 pg/mL).

The cell aggregate containing a hypothalamic cell, a pituitary progenitor cell, and a pituitary hormone-producing cell obtained in step (A) as described above are dispersed into a cell population in step (B) (this step is sometimes collectively referred to as "intermediate purification").

In step (B), the obtained cell aggregates may be first pre-treated with a ROCK inhibitor. For the pre-treatment, a ROCK inhibitor is preferably added before the start of the pre-treatment in order to suppress cell death induced by dispersing of the cell aggregate thereafter. The ROCK inhibitor is added, for example, at least 24 hr before, at least 12 hr before, at least 6 hr before, at least 3 hr before, at least 2 hr before, at least 1 hr before, the start of the treatment. As the ROCK inhibitor, Y-27632((+)-(R)-trans-4-(1-aminoethyl)-N-(4-pyridyl)cyclohexanecarboxamide dihydrochloride) and the like can be mentioned. The concentration of the ROCK inhibitor used for the treatment is a concentration that can suppress cell death induced by dispersing of the cell aggregate thereafter. For example, for Y-27632, such concentration is generally about 0.1 to 200 µM, preferably about 2 to 50 µM. The concentration of the ROCK inhibitor may be varied during the period of addition, and, for example, the concentration can be halved in the latter half of the period.

As the medium to perform the pre-treatment, the medium used in the culture step of step (A) can be used. When a ROCK inhibitor is already added to the above-mentioned medium at a desired concentration, the pre-treatment step is not necessary.

Then, the cell aggregates that underwent the above-mentioned pre-treatment are dispersed by an enzyme treatment. Specifically, the cell aggregates that underwent the above-mentioned pre-treatment are transferred to a culture vessel containing PBS and washed with the same medium. The enzyme for dispersion is not particularly limited as long as it can disperse the cells. For example, papain, EDTA; enzyme such as trypsin, collagenase (collagenase type I - VII), metalloprotease, hyaluronidase, elastase, dispase, deoxyribonuclease, and the like, and a mixture of these can be mentioned. A preferred enzyme is papain. The conditions (temperature, time, etc.) of the enzyme treatment can be appropriately set according to the enzyme to be used and the like. To promote the enzyme treatment, a step of physically (e.g., scalpel, scissors, etc.) shredding the cell aggregate, or physically (e.g., scalpel, scissors, etc.) incising the cell aggregates may be performed before the treatment.

After the above-mentioned enzyme treatment, floating cells are recovered, and the above-mentioned enzyme treatment may be performed again. It may be repeated multiple times. The enzyme treatment can also be performed using the aforementioned enzymes and the like. A preferred enzyme includes EDTA; enzyme such as trypsin, collagenase (collagenase type I - VII), metalloprotease, hyaluronidase, elastase, dispase, deoxyribonuclease, and the like, and a mixture of these. A preferred enzyme is collagenase, and more preferred enzyme is collagenase type I. The conditions (temperature, time, etc.) of the enzyme treatment can be appropriately set according to the enzyme to be used and the like.

After the above-mentioned enzyme treatment, floating cells are recovered, and the above-mentioned enzyme treatment is performed again. The enzyme treatment can be performed using the aforementioned enzyme and the like, and preferred enzymes are EDTA; trypsin, more preferably, EDTA; trypsin and deoxyribonuclease. In addition, a commercially available product such as TrypLE (Invitrogen) or the like may also be used instead of EDTA; trypsin. The conditions (temperature, time, etc.) of the enzyme treatment can be appropriately set according to the enzyme to be used and the like. A single cell suspension can be prepared by the above-mentioned series of enzyme treatments. When preparing a single cell suspension, dead cells may be removed by a method known per se.

In step (1) of the present invention, cells expressing EpCAM, which is a cell surface marker specific for pituitary progenitor cells and pituitary hormone-producing cells, are separated from the dispersed cell population including a hypothalamic cell, a pituitary progenitor cell, and a pituitary hormone-producing cell obtained as described above. As a method for separating the desired cells that express EpCAM from a dispersed cell population, methods using flow cytometry and mass cytometry, magnetic cell separation methods, and the like can be mentioned. These methods can be carried out using a method known per se. For example, cell that expresses EpCAM can be separated by a method including a step of contacting the cells and a substance (e.g., antibody, etc.) that specifically binds to EpCAM molecule. The above-mentioned substance includes those with detectable labels (e.g., GFP, PE) attached to themselves and those without labels attached to themselves. When the above-mentioned substance does not have a label attached thereto, the separation can be achieved by further using a substance with a detectable label attached thereto that directly or indirectly recognizes the substance. For example, when the aforementioned substance is an antibody, then a fluorescent dye, a metal isotope, or a bead (e.g., magnetic bead) is directly or indirectly carried on the antibody to label a cell surface marker. The cells can be separated based on the label. The antibody used at this time may be one kind of antibody, or two or more kinds of antibodies.

The method of the present invention includes a step of suspension culturing the separated EpCAM-expressing cell (the cell is sometimes collectively referred to as "purified intermediate") for not less than 14 days under conditions that allow the pituitary progenitor cell to mature into a pituitary hormone-producing cell (step (2) of the present invention). The conditions under which pituitary progenitor cells can mature into pituitary hormone-producing cells are culture conditions under which pituitary progenitor cells differentiate into pituitary hormone-producing cells and the hormone production ability of the differentiated pituitary hormone-producing cells is maintained or improved. As the conditions under which pituitary progenitor cells can mature into pituitary hormone-producing cells, one embodiment is culturing in a medium (about 0.18 to about 20 mL) that can be used for culturing pituitary hormone-producing cells, preferably serum-free medium containing KSR (e.g., IMDM, DMEM, EMEM, αMEM, GMEM, F-12 medium, DMEM/F12, IMDM/F12, epithelial cell medium (e.g., CnT-Prime epithelial culture medium), or mixed medium of these, etc.). The culture may be adhesion culturing or suspension culturing, and suspension culturing is preferred.

The period of suspension culturing of pituitary progenitor cells under conditions permitting maturation into pituitary hormone-producing cells is generally, not less than 14 days, preferably not less than 30 days.

One embodiment of the method of the present invention is, for example, the following production method.

A method for producing a cell aggregate containing pituitary hormone-producing cells, which includes the following steps:
(1) a step of separating cells expressing EpCAM from a dispersed cell population derived from a pluripotent stem cell and including one to four types of pituitary hormone-producing cells (e.g., cell population that contains pituitary hormone-producing cells that secrete ACTH but does not contain pituitary hormone-producing cells that secrete GH or TSH), and
(2) a step of suspension culturing the separated EpCAM-expressing cell for not less than 14 days under conditions that allow the pituitary progenitor cell to mature into a pituitary hormone-producing cell.

Another embodiment of the method of the present invention is, for example, the following production method.

A method for producing a cell aggregate containing pituitary hormone-producing cells, which includes the following steps:
(1) a step of separating cells expressing EpCAM from a cell population obtained by dispersing a cell aggregate derived from pluripotent stem cells and containing early stage pituitary tissue or middle stage pituitary tissue, and
(2) a step of suspension culturing the separated EpCAM-expressing cell for not less than 14 days under conditions that allow the pituitary progenitor cell to mature into a pituitary hormone-producing cell.

Another embodiment of the method of the present invention is, for example, the following production method.

A method for producing a cell aggregate containing pituitary hormone-producing cells, which includes the following steps:
(1) a step of separating cells expressing EpCAM from a cell population obtained by dispersing a cell aggregate derived from pluripotent stem cells and containing not less than one and not more than 4 kinds of pituitary hormone-producing cells, and
(2) a step of suspension culturing the separated EpCAM-expressing cell for not less than 14 days under conditions that allow the pituitary progenitor cell to mature into a pituitary hormone-producing cell.

A still another embodiment of the method of the present invention is, for example, the following production method. A method for producing a cell aggregate containing pituitary hormone-producing cells, which includes the following steps:
(1) a step of separating cells expressing EpCAM from a cell population obtained by dispersing a cell aggregate derived from pluripotent stem cells and containing not less than one and not more than 4 kinds of pituitary hormone-producing cells and containing Nkx2.1-expressing cells in hypothalamic tissue, and
(2) a step of suspension culturing the separated EpCAM-expressing cell for not less than 14 days under conditions that allow the pituitary progenitor cell to mature into a pituitary hormone-producing cell.

### [Cell aggregate containing pituitary hormone-producing cells]

The cell aggregate containing pituitary hormone-producing cells obtained by the method of the present invention (the cell aggregate of the present invention) has the following characteristics.
(1) The percentage of cells positive for at least one of EpCAM-positive cell and E-cadherin in the cell aggregate is not less than 80%.
(2) The percentage of neural cells in the cell aggregate is not more than 20%.
(3) The percentage of ACTH-positive cells in the cell aggregate is not less than 5%.
(4) The cell density of the cell aggregate is 3,000 to 20,000 cells/mm².
(5) The major axis of the cell aggregate is 200 to 3,000 µm.
(6) On the surface of the cell aggregate, cells positive for at least one of EpCAM-positive cell and E-cadherin form a plane attachment to each other to form an epithelial structure.
(7) The cell aggregate forms a sponge-like structure.

The cell aggregate obtained by the method of the present invention undergoes a step of separating cells that express EpCAM, and therefore, EpCAM positive cells account for many of the cells in the cell aggregate. In addition, the cell aggregate obtained by the method of the present invention also contains E-cadherin-positive cells. Some or all of the EpCAM-positive cells may be EpCAM-positive and E-cadherin-positive cells. The proportion of the number of cells positive for at least one of EpCAM and E-cadherin to the total number of cells present in the cell aggregate depends on the separation efficiency of the step of separating cells expressing EpCAM, and is, for example, not less than 80%, preferably not less than 85%, not less than 90%, or not less than 95%.

EpCAM and E-cadherin are not expressed in neural cells constituting the hypothalamus. (e.g., neuron, glial cell, and the like, hypothalamic progenitor cells which are progenitor cells of these; these are sometimes called "hypothalamic cells"). Therefore, by going through the step of separating cells expressing EpCAM, these cells are removed. That is, the proportion of the number of neural cells (hypothalamic cells) to the total number of cells present in the cell aggregate (the ratio of neural cells present) depends on the separation efficiency of the step of separating cells expressing EpCAM, and may be, for example, not more than 20%, preferably not more than 15%, not more than 10%, not more than 5%. The markers expressed in neural cells constituting the hypothalamus include Rx, NKx2.1, Nestin, and Pax6. In other words, the proportion of the number of cells expressing Rx, NKx2.1, Nestin and/or Pax6 to the total number of cells present in the cell aggregate may be, for example, not more than 20%, preferably not more than 15%, not more than 10%, or not more than 5%.

The cell aggregate contains pituitary hormone-producing cells. The proportion of the number of ACTH-positive (producing) cells to the total number of cells present in the cell aggregate (the proportion of the number of ACTH-positive cells present) may be not less than 5%, preferably not less than 10%. ACTH positive cells simultaneously express NeuroD1 and Tbx19. Therefore, the proportion of the number of NeuroD1-positive and/or Tbx19-positive cells to the total number of cells present in the cell aggregate may also be equivalent to the proportion of the number of ACTH-positive cells.

The ACTH secretory capacity of the cell aggregate may be 200 to 1,000,000 pg/mL. In one embodiment, it may be 1,000 to 500,000 pg/mL, 5,000 to 300,000 pg/mL, or 10,000 to 100,000 pg/mL.

The ACTH secretory capacity is affected by the number of ACTH cells, culture conditions, and the like. The ACTH secretory capacity in the present specification is expressed as the concentration of ACTH secreted into the medium from one cell aggregate under specific culture conditions. The specific culture conditions means conditions in which (1) a medium that can be used for culturing the aforementioned pituitary hormone-producing cells, preferably serum-free medium containing KSR (e.g., IMDM, DMEM, EMEM, αMEM, GMEM, F-12 medium, DMEM/F12, IMDM/F12, epithelial cell medium (e.g., CnT-Prime epithelial culture medium), or mixed medium of these, etc.) is used, (2) external substances that affect ACTH production (e.g. Notch signal inhibitors that increase ACTH production) are not added, (3) the medium volume is set to 0.18 to 20 mL, and (4) culture is performed for 3 to 4 days. The medium after culturing under the conditions is collected and the concentration of ACTH in the medium is measured. Those skilled in the art can measure the concentration of ACTH by using known method such as ELISA.

The cell aggregate may contain pituitary hormone-producing cells other than ACTH producing cells. Specifically, PRL-producing cells, FSH-producing cells, LH-producing cells, GH-producing cells, TSH-producing cells, and the like can be mentioned. In one embodiment, two types of ACTH-producing cells and PRL-producing cells may be contained, and in another embodiment, ACTH-producing cells and PRL-producing cells, as well as 1 or 2 cells selected from the group consisting of FSH-producing cells and LH-producing cells may be contained (that is, containing 3 types or 4 types of pituitary hormone-producing cells). In another embodiment, ACTH-producing cells, PRL-producing cells, FSH-producing cells and LH-producing cells, as well as 1 or 2 cells selected from the group consisting of GH-producing cells and TSH-producing cells (that is, containing 5 types or 6 types of pituitary hormone-producing cells).

The pituitary hormone-producing cells contained in the cell aggregate depends on the aforementioned differentiation stage, i.e. the timing of separating cells expressing EpCAM and the subsequent culture period. The presence of various pituitary hormone-producing cells can be confirmed by confirming the state of production of the aforementioned hormones using ELISA or immunostaining, and the like. Therefore, those of ordinary skill in the art can adjust the separation timing and culture period to obtain cell aggregates containing the necessary pituitary hormone-producing cells.

The cell aggregate may further contain pituitary stem cells. In the present invention, the "pituitary stem cell" refers to an undifferentiated multipotent stem cell or progenitor cell that is present in the pituitary gland and contributes to regeneration of pituitary tissue and supply of pituitary hormone-producing cells.

The proportion of the number of pituitary stem cells to the total number of cells present in the cell aggregate (proportion of the number of pituitary stem cells) may be not less than 1%, preferably not less than 3%, or not less than 5%. The pituitary stem cell simultaneously expresses pituitary stem cell markers such as Sox2, Sox9, E-Cadherin, Nestin, S100β, GFRu2, Prop1, CD133, β-Catenin, Klf4, Oct4, Pax6, Coxsackievirus and adenovirus common receptor (CXADR), PRRX1/2, Ephrin-B2, and ACE. Therefore, the proportion of the number of pituitary stem cells can be determined by measuring the proportion of the number of cells positive to the above-mentioned pituitary stem cell markers (e.g., CXADR) to the total number of cells present in the cell aggregate.

The cell density of the cell aggregate may be 3,000 to 20,000 cells/mm², preferably 5,000 to 10,000 cells/mm², more preferably 6,000 to 9,000 cells/mm².

The cell density of the cell aggregate in the present specification is expressed as the number of cells per unit area on a cut surface of a cell aggregate, for example, at a position within 200 um from the center. Those of ordinary skill in the art can measure the cell density of cell aggregates by using a known method. Specifically, a section obtained by cutting a cell aggregate is prepared, the number of cells is measured by a method such as immunostaining using DAPI, anti-E-cadherin antibody, and the like, and the number is divided by the area of the section.

In one embodiment, the major axis (or circle equivalent diameter) of the cell aggregate may be, for example, 200 to 3,000 µm, preferably 300 to 2,000 µm, more preferably 400 to 1,500 µm.

The method of measuring the major axis, minor axis, and height of the cell aggregate is not particularly limited, and may be measured, for example, from an image taken under a microscope. For example, cell aggregates cultured in a 96-well culture plate can be imaged using a Keyence inverted microscope with a 10x lens, and measurements can be performed from the image. Here, the major axis means the longest line segment and its length among the line segments connecting the two end points of the cell aggregate in the captured image. The circle equivalent diameter means the diameter of a perfect circle that corresponds to the area of a figure (circle or ellipse) obtained when projected onto a two-dimensional surface. As described later, the aggregate is a spherical aggregate, and the major axis and circle equivalent diameter are close to each other, and the difference in the major axis and circle equivalent diameter becomes smaller as the aggregate becomes closer to the sphere-like aggregate close to true sphere.

The aggregate is a sphere-like aggregate. The "sphere-like cell aggregate" in the present specification means a cell aggregate having a three-dimensional shape close to a spherical shape. The three-dimensional shape close to a spherical shape is a shape that has a three-dimensional structure, and includes, for example, a spherical shape that exhibits a circular or elliptical shape when projected onto a two-dimensional surface.

The aggregate is preferably a spherical aggregate that is close to a true sphere. A spherical aggregate that is close to a true sphere means a cell aggregate having a three-dimensional shape close to a true sphere. A three-dimensional shape that is close to a true sphere is a spherical shape in which multiple circular shapes obtained when projected onto a two-dimensional surface from multiple directions all have the same length from the center of the circular shape to the outer circumference, or a spherical shape in which the difference between the longest line segment and the shortest line segment is not more than 10%, not more than 5%, not more than 3%, not more than 2%, or not more than 1%.

On the surface of the cell aggregate, cells positive for at least one of EpCAM-positive cells and E-cadherin may form a plane attachment to each other to form an epithelial structure.

The "plane adhesion" means that adjacent cells adhere to each other on the surface. More specifically, the plane adhesion means that the proportion of the surface area of one cell that is adhered to the surface of another cell is, for example, not less than 1%, preferably not less than 3%, more preferably not less than 5%, further preferably not less than 10%. In one embodiment, the surface of a cell refers to the surface of a cell membrane. Cell surfaces can be observed by immunostaining for cell adhesion factors (e.g., E-cadherin, EpCAM). An example of a state other than plane adhesion is point adhesion. Point adhesion means that a cell and a cell are adhered at points.

The "epithelial structure" refers to a structure characteristically possessed by epithelial tissues, such as the apical surface or base membrane. It can be confirmed by immunostaining using the apical surface and base membrane markers described below.

The epithelial tissue becomes polarized, forming an "apical surface" and a "base membrane". The "base membrane" refers to a 50-100 nm base membrane containing a lot of base membrane markers laminin and type IV collagen in which a basal layer (basal membrane) produced by epithelial cells exists. The "apical surface" refers to a surface (superficial surface) formed on the opposite side to the "base membrane". Such apical surface can be identified by immunostaining method and the like well known to those skilled in the art, by using antibodies against apical surface markers (e.g., atypical-PKC (hereinafter abbreviated as "aPKC"), E-cadherin, N-cadherin).

The "epithelial tissue" is a tissue formed by cells covering the surface of the body, lumens (gastrointestinal tract and the like), body cavity (pericardial cavity and the like), and the like without clearance. Cells that form epithelial tissue are called epithelial cells. Epithelial cells have an apical-basal polarity. Epithelial cells can form a cell layer by forming strong connections between themselves through adherens junction and/or tight junction. Tissue consisting of one to a dozen or more overlapping cell layers is an epithelial tissue.

The cell aggregate has a plurality of island-like structures formed by plane adhesion of EpCAM-positive cells and/or E-cadherin-positive cells to each other. In the cell aggregate, a plurality of island-like structures assemble to form a sponge-like structure.

The "islet-like structure" is a structure formed by plane adhesion of about 2 to 20 (e.g., 3, 4, 5, 6, 7, 8, 9, 10, 15, 20) EpCAM-positive cells and/or E-cadherin-positive cells to each other. The number of cells included in each islet-like structure may be different for each islet-like structure. The plane adhesion means that adjacent cells adhere to each other on their surfaces. More specifically, the plane adhesion means that the proportion of cells that adhere to each other on a surface is, for example, not less than 1%, preferably not less than 3%, more preferably not less than 5%, further preferably not less than 10%. The major axis of the cells present in the islet-like structure may be, for example, 2 to 50 um, preferably 4 to 30 um, and the minor axis may be, for example, 0.5 to 20 um, preferably 1 to 10 µm.

The islet-like structure may contain pituitary hormone-producing cells. Specifically, ACTH-positive cell, PRL-positive cell, FSH-positive cell, LH-positive cell, GH-positive cell, TSH-positive cell, and the like can be mentioned.

The "sponge-like structure" is a structure in which a plurality of islet-like structures are assembled and voids exist between the islet-like structures. A void is not necessarily present between all the islet-like structures. Since nutrients and oxygen from the culture medium can reach the inside of the cell aggregate due to the presence of voids, the proportion of necrotic cells is small even in the center of the cell aggregate.

The "islet-like structure" and "sponge-like structure" are not observed in cell aggregates obtained before sorting by EpCAM, that is, by suspension culturing of pluripotent stem cells. The "islet-like structure" and "sponge-like structure" are structures that are first confirmed by reaggregation after sorting by EpCAM.

In one embodiment, the GH secretory capacity of the cell aggregate may be 0.05 to 100 ng/mL, and in one embodiment, it may be 0.1 to 50 ng/mL, 0.3 to 30 ng/mL, 0.5 to 10 ng/mL.

The GH secretory capacity is affected by the number of GH cells, culture conditions, and the like. The GH secretory capacity in the present specification is expressed as the concentration of GH secreted into the medium from one cell aggregate under specific culture conditions. The specific culture conditions means conditions in which (1) a medium that can be used for culturing the aforementioned pituitary hormone-producing cells, preferably serum-free medium containing KSR (e.g., DMEM, MEM, GMEM, epithelial cell medium (e.g., CnT-Prime epithelial culture medium) is used, (2) external substances that affect GH production (e.g. corticosteroids (e.g., dexamethasone) that increase GH production) are not added, (3) the medium volume is set to 0.18 to 20 mL, and (4) culture is performed for 3 to 4 days. The medium after culturing under the conditions is collected and the concentration of GH in the medium is measured. Those skilled in the art can measure the concentration of GH by using known method such as ELISA.

Depending on the purpose of use of the cell aggregate, the secretion amount of a specific pituitary hormone production can be increased by culturing in the presence of a specific substance.

For example, in the above-mentioned method, the GH secretory capacity is improved by changing the conditions of (2) to the condition of culturing in a medium containing corticosteroids. As corticosteroids, artificially synthesized glucocorticoids such as dexamethasone, betamethasone, predonisolone, methylprednisolone, and triamcinolone; natural glucocorticoids such as hydrocortisone, cortisone acetate, and fludrocortisone acetate, and the like can be mentioned. Those of ordinary skill in the art can appropriately determine the concentration of corticosteroids. In one embodiment, the concentration of dexamethasone may be 4 ng/mL to 40 pg/mL, preferably 40 ng/mL to 4 pg/mL.

The GH secretory capacity of a cell aggregate containing pituitary hormone-producing cells after culturing in the presence of corticosteroids may be, in one embodiment, 0.1 to 10,000 ng/mL, 0.5 to 5,000 ng/mL, 5 to 1,000 ng/mL, 20 to 500 ng/mL. In this case, the ACTH secretory capacity is 10 to 500,000 pg/mL, preferably 100 to 100,000 pg/mL, more preferably 500 to 30,000 pg/mL, further preferably 1,000 to 10,000 pg/mL.

In this way, a cell aggregate containing pituitary hormone-producing cells after culturing in the presence of corticosteroids contains more pituitary hormone-producing cells than before the start of differentiation induction. In one embodiment, when cultured in the presence of corticosteroids, the proportion of the number of ACTH-positive (producing) cells to the total number of cells present in the cell aggregate (the proportion of the number of ACTH-positive cells present) may decrease. In this case, the proportion of the number of ACTH positive cells is not less than 2%, preferably not less than 3%, more preferably not less than 5%.

The present invention also provides a cell population containing the cell aggregate of the present invention at not less than 40% of the total number of cell aggregates (the cell population of the present invention). The cell population of the present invention may contain the cell aggregate of the present invention at generally not less than 40%, preferably not less than 60%, more preferably not less than 80%, of the total number of cell aggregates. In one embodiment, the proportion of the cell aggregate of the present invention to all the cell aggregates can vary to a certain degree depending on the cell line, differentiation induction method, and maturation culture.

The cell population of the present invention may have the following characteristics.
(1) the percentage of EpCAM-positive cells and E-cadherin-positive cells is not less than 80%,
(2) the percentage of neural cells is not more than 20%; and
(3) the ACTH secretory capacity of the cell aggregate is 200 to 1,000,000 pg/mL.

### [Pharmaceutical composition, treatment method and therapeutic drug]

One embodiment of the present invention is a pharmaceutical composition (composition for transplantation, tissue for transplantation, or Transplant) containing the cell aggregate of the present invention or a portion thereof. The pharmaceutical composition preferably further contains, in addition to the cell aggregate of the present invention or a portion thereof, a pharmaceutically acceptable carrier. A portion of the cell aggregate is a portion of the cell aggregate that can be used in a pharmaceutical composition, and can be obtained by cutting out pituitary tissue of a size necessary for transplantation from the cell aggregate.

As a pharmaceutically acceptable carrier, a physiological aqueous solvent (physiological saline, buffer, serum-free medium, etc.) can be used. Where necessary, the pharmaceutical composition may contain preservative, stabilizer, reducing agent, tonicity agent, and the like that are generally used in medicines containing tissues or cells to be transplanted in transplantation therapy.

The present invention also provides a therapeutic drug for a disease due to a disorder of pituitary gland, containing the cell aggregate of the present invention or a part thereof, or the cell population of the present invention (the therapeutic drug of the present invention).

Examples of the therapeutic drug for a disease due to a disorder of pituitary gland include a graft containing a suspension containing the cell population of the present invention.

Examples of the suspension include a liquid obtained by suspending the cell population of the present invention in an artificial lacrimal fluid or physiological saline. The suspension may contain pituitary hormone-producing cells isolated from the cell population of the present invention, and may also contain a factor that promotes adhesion of the cells, such as extracellular matrix, and hyaluronic acid.

Furthermore, a method for treating a disease due to a disorder of pituitary gland, including a step of transplanting an effective amount of pituitary hormone-producing cells from the cell aggregate or a part thereof, or the cell population of the present invention, to a target in need of the transplantation can be provided.

The aforementioned disease due to a disorder of pituitary gland may be an animal disease due to a disorder of pituitary gland, or a disease due to a disorder of pituitary gland in a non-human animal. As the disease due to a disorder of pituitary gland, panhypopituitarism, pituitary dwarfism, hypoadrenocorticism, partial hypopituitarism, pituitary anterior hormone isolated deficiency, insufficient pituitary function/hormone secretion after surgery for pituitary adenoma and the like, craniopharyngioma, and the like can be specifically mentioned.

The present invention is explained in more detail in the following by referring to Examples, which are not to be construed as limiting the scope of the present invention. Unless particularly limited, the reagents and materials to be used are commercially available.

### [Example]

### Example 1: Example of forming cell aggregate containing hypothalamic cell, pituitary progenitor cell, and pituitary hormone-producing cell from human ES cells

Fig. 1 shows the diagram of entire steps (step 1 differentiation, step 2 intermediate purification, step 3 maturation culture) of producing cell aggregates containing pituitary hormone-producing cells from human ES/iPS cells.

In the following, human ES cells were induced to differentiate into cell aggregates containing hypothalamic cells, pituitary progenitor cells, and pituitary hormone-producing cells, and the composition and structure of the cell aggregates at each number of days after differentiation induction were investigated.

Human ES cells (KhES-1 strain, obtained from Kyoto University) were cultured under feeder-free conditions according to the method described in Scientific Reports, 4, 3594 (2014). As the feeder-free medium, StemFit medium (AK03, manufactured by Ajinomoto Co., Inc.) was used and, as the feeder-free scaffold, Laminin 511-E8 (manufactured by Nippi, Inc.) was used.

As specific maintenance culturing operation, subconfluent human ES cells (KhES-1 strain) were first washed with PBS and dispersed into single cells by using TrypLE Select (manufactured by Life Technologies). Thereafter, the aforementioned human ES cells dispersed into single cells were seeded in a plastic culture dish coated with Laminin 511-E8, and cultured under feeder-free conditions in StemFit medium in the presence of Y27632 (ROCK inhibiting substance, 10 µM). When a 6-well plate (manufactured by Iwaki, for cell culturing, culture area 9.4 cm²) was used as the aforementioned plastic culture dish, the number of plated cells of the aforementioned human ES cells dispersed into single cells was adjusted to 2.4 × 10⁴. One day after seeding, the entire amount of the medium was changed with StemFit medium free of Y27632. Thereafter, once in 1 - 2 days, the entire amount of the medium was changed with StemFit medium free of Y27632. Thereafter, the cells were cultured until 7 days after seeding when they reached subconfluence (60% of culture area is covered with cells).

The protocol for inducing differentiation of human ES cells into cell aggregates containing hypothalamic cells, pituitary progenitor cells, and pituitary hormone-producing cells is shown in Fig. 2. For the differentiation induction operation, human ES cells (KhES-1 strain) were seeded using StemFit medium, and 6 days after the seeding, SB431542 (5 µM) and SAG (300 nM) were added at the same time as the exchange of StemFit medium, and the cells were cultured for 24 hr. The prepared subconfluent human ES cells were treated with a cell dissociation solution using TrypLE Select (manufactured by Life Technologies), and further dispersed into single cells by a pipetting operation. Thereafter, the aforementioned human ES cells dispersed into single cells were suspended in 100 µl of a serum-free medium at 1.0 × 10⁴ cells per well of a non-cell-adhesive 96-well culture plate (PrimeSurface 96V-bottom plate, MS-9096V, manufactured by SUMITOMO BAKELITE), and suspension cultured under conditions of 37°C, 5% CO₂. As the serum-free medium (gfCDM+KSR) therefor, a serum-free medium which is a 1:1 mixture of F-12 medium and IMDM medium supplemented with 5% KSR, 450 µM 1-mono thioglycerol, 1xChemically defined lipid concentrate was used. At the time of the start of suspension culturing (day 0 after the start of differentiation induction), Y27632 (final concentration 20 µM), IWP-2 (0.5 µM), SB431542 (1 µM), and SAG (100 nM) were added to the aforementioned serum-free medium.

On day 2 after the start of the differentiation induction, a serum-free medium not containing Y27632 and containing IWP-2, SB431542, BMP4 (0.5 nM), and SAG (700 nM) was added at 100 µl per well. Thereafter, a half amount of the medium was changed with a serum-free medium not containing Y27632 or BMP4 and containing IWP-2, SB431542, and SAG was added at days 6, 9, 12, 15, 19, 22, and 26 after the start of differentiation induction. After day 19, the oxygen partial pressure during culture was set to 40%.

On day 29 after the start of differentiation induction, the cell aggregates were transferred to a 10 cm suspension culture dish at a rate of 60 cell aggregates per dish, and suspension culturing was continued using a serum-free medium under conditions of 40% O₂, 5% CO₂. Culture was performed using a serum-free medium supplemented with 10% KSR from day 29 to day 50 after the start of differentiation induction, and a serum-free medium supplemented with 20% KSR after day 50 after the start of differentiation induction.

The aforementioned cell aggregates at days 29, 61, and 103 after the start of differentiation induction were each fixed with 4% para-formaldehyde, and cryosections were prepared. These cryosections were subjected to immunostaining using pituitary precursor markers Pitx1 (anti-Pitx1 antibody, homemade (Non Patent Literatures 1 and 2), Guinea pig) and Lhx3 (anti-Lhx3 antibody, homemade (Non Patent Literatures 1 and 2), rabbit), epithelial cell markers E-cadherin (anti-E-cadherin antibody, manufactured by TAKARA, rat), EpCAM (anti-EpCAM antibody, manufactured by R&D, goat), and ventral hypothalamic marker Nkx2.1 (anti-Nkx2. 1 antibody, manufactured by Progen, mouse), pituitary hormone-producing cell marker adrenocorticotropic hormone (ACTH) (anti-ACTH antibody, manufactured by Fitzgerald, mouse), and nervous system cell marker Nestin (anti-Nestin antibody, manufactured by R&D, mouse). Cell nuclei were stained with DAPI. These stained sections were observed using a confocal laser scanning microscope (manufactured by OLYMPUS CORPORATION), and immunostained images were obtained. As a result, Nkx2.1-positive ventral hypothalamic tissue was present inside the cell aggregates induced by the above differentiation induction method, and on day 29 after the start of differentiation induction (Fig. 3F), and EpCAM-, E-cadherin-positive thickened epithelial tissue was formed on the outside (Fig. 3B, D). Furthermore, some of the epithelial tissues were Pitx1- and Lhx3-positive, indicating that pituitary precursor tissues were formed (Fig. 3C, E).

Furthermore, in cell aggregates on days 61 and 103 after the start of differentiation induction, ACTH-positive pituitary hormone-producing cells (Fig. 3I O) were present in some E-cadherin-, EpCAM-positive epithelial tissues (Fig. 3J, K, P, Q). It was also found that Nestin-positive neural tissue was formed inside the cell aggregates (Fig. 3L).

### Example 2: Example of purifying EpCAM-positive cell population from cell aggregate containing hypothalamic cell, pituitary progenitor cell, and pituitary hormone-producing cell and producing cell aggregate containing pituitary hormone-producing cells by long-term culture after purification

It was found that the cell aggregates produced by the differentiation induction method in Example 1 contained not only pituitary precursor tissues and pituitary tissues but also hypothalamic tissues and neural tissues. In order to produce highly pure cell aggregates containing only pituitary tissue from these cell aggregates, purification using EpCAM and long-term culture were performed, and the composition and structure of the cell aggregates obtained after purification were examined.

Cell sorting using an anti-EpCAM antibody was performed on cell aggregates obtained by the same differentiation induction method as in Example 1, and on day 62 and day 100 after the start of differentiation induction. Cell sorting was performed in three steps: (1) pre-treatment, (2) dispersion of cell aggregates, and (3) separation of EpCAM-positive cells.

### (1) Pre-treatment with Y27632

In order to suppress cell death due to dispersion treatment, Y27632 (20 µM) was added to the medium the day before the work day to pre-treat the cell aggregates. For subsequent operation prior to cell sorting, 20 µM Y27632 was added to all solutions to which cells were exposed.

### (2) Dispersion of cell aggregates

In order to promote dispersion by enzyme treatment, the cell aggregates were shredded or incised with a scalpel. Next, the cell aggregates were washed with PBS, a dissociation solution from a neuron dissociation kit (manufactured by Wako) was added, enzyme treatment was performed at 37°C for 30 min, and the cell aggregates were further loosened by pipetting. After the reaction was completed, the mixture was washed with DMEM/F12 (manufactured by Wako), a collagenase solution was added, and the mixture was swirl shaken (140 to 150 rpm) at 37°C for 30 min. The composition of the collagenase solution was the above DMEM/F12 to which 0.2% collagenase type I (manufactured by Wako) and 0.1% BSA (manufactured by Themofisher) were added. After the collagenase treatment was completed, the mixture was washed with PBS, 10x TrypLE Select solution (manufactured by Themofisher) + 0.2 mg/mL DNaseI (manufactured by Roche) was added, treated with enzyme at 37°C for 10 min, and further dispersed into single cells by pipetting. The cells were suspended and neutralized in serum-free medium supplemented with 20% KSR, centrifuged, and resuspended in serum-free medium supplemented with 10 µg/mL DnaseI and 20% KSR. The cells were passed through a 70 µm cell strainer to remove aggregates.

### (3) Separation of EpCAM positive cell by FACS

The single cells obtained above were centrifuged and suspended in DMEM/F-12 solution added with 1 mM EDTA, 1% FBS. PE-labeled anti-EpCAM antibody (manufactured by Miltenyi) was added, the cells were incubated at 4°C for 10 min, fluorescence-activated cell sorting (FACS) was performed, and EpCAM-positive cells were separated and collected. As a cell sorter for FACS, FACS Aria Fusion of BD, which is a Stream-In-Air type model, was used.

The purified EpCAM-positive cells were reaggregated in a cell non-adhesive 96-well culture plate (10×10⁴ cells/200 µL/well) using a serum-free medium supplemented with 20% KSR and 20 µM Y27632and suspension cultured under conditions of 37°C, 40% O₂, 5% CO₂. After the third day from the start of suspension culturing, a serum-free medium not containing Y27632 and supplemented with 20% KSR was used, and the medium was replaced by half the volume once in 3 to 4 days and the cells were cultured for a long period (Fig. 4).

Cell aggregates obtained by purifying cell aggregates on day 62 after the start of differentiation induction and culturing for a long period of 41 days after reaggregation (day 62 sort + day 41) and cell aggregates obtained by purifying cell aggregates on day 100 after the start of differentiation induction and culturing for a long period of 31 days after reaggregation (day 100 sort + day 31) were each fixed with 4% paraformaldehyde to prepare cryosections. These cryosections were subjected to immunostaining using pituitary precursor marker Lhx3, epithelial cell markers EpCAM and E-cadherin, pituitary hormone-producing cell marker adrenocorticotropic hormone (ACTH), prolactin (PRL) (anti-PRL antibody, manufactured by Dako, rabbit), follicle-stimulating hormone (FSH) (anti-FSH antibody, manufactured by Dako, mouse), luteinizing hormone (LH) (anti-LH antibody, manufactured by Dako, mouse), and thyroid-stimulating hormone (TSH) (anti-TSH antibody, manufactured by Dako, mouse). Cell nuclei were stained with DAPI. These stained sections were observed using a confocal laser scanning microscope (manufactured by OLYMPUS CORPORATION), and immunostained images were obtained. As a result, it was found that in cell aggregates of day 62 sort + day 41, EpCAM-, E-cadherin-positive cells formed a plane attachment to each other and an epithelial structure was formed on the entire surface of the aggregates (Fig. 5A, C, D, F, H, I). Furthermore, it was found that many ACTH positive and/or Lhx3 positive pituitary hormone-producing cells or pituitary progenitor cells were present in the cell aggregates (Fig. 5B, E, G, J). In addition, it was found that PRL-, FSH-positive cells were present (Fig. 5L, N).

Also, it was found in the cell aggregates of day 100 sort + day 31 that, the EpCAM-, E-cadherin-positive cells form a plane attachment to each other as in the above, and the epithelial structure was formed on the entire surface of the aggregates (Fig. 6A, C, 6D, 6E, 6G, H). Furthermore, it was found that many ACTH-positive pituitary hormone-producing cells were present inside the cell aggregates (Fig. 6B, F). In addition, it was found that PRL-, FSH-, LH-, TSH-positive cells were present (Fig. 6J, L, N, P). That is, it was found that the cell aggregates obtained by long-term culture after EpCAM purification formed an epithelial structure, and were cell aggregates containing at least one or more kinds of pituitary hormone-producing cells.

Furthermore, the ACTH positive cell rate, cell density, and the major axis in the cell aggregates of day 62 sort + day 41 and the cell aggregates of day 100 sort + day 31 were quantified. In order to quantify the ACTH-positive cell rate, the number of ACTH-positive cells and the number of DAPI-positive cells per aggregate were analyzed from an image taken with a 10x lens of confocal laser scanning microscope (OLYMPUS CORPORATION) of the area where ACTH-positive cells were observed for each aggregate, and the proportion was calculated. In order to quantify the cell density, the number of DAPI-positive cells per aggregate and the surface area of the aggregate were analyzed and calculated using the image taken above. In order to quantify the major axis, the measurement was performed using the image taken with a 10x lens of inverted microscope (manufactured by Keyence)(Fig. 7C, D).

As a result, in the cell aggregates of day 62 sort + day 41 and the cell aggregates of day 100 sort + day 31, the ACTH positive cell rate was respectively 11%, 18% (Fig. 7A), the density was respectively 7880 cells/mm², 7720 cells/mm² (Fig. 7B), and the major axis was respectively 633 µm, 639 um (Fig. 7E) .

### Example 3: Example of producing cell aggregate containing pituitary hormone-producing cells of ACTH and GH by addition of dexamethasone during long period culture after EpCAM purification

From the results of Example 2, it was found that cell aggregates after purification mainly contained ACTH-producing cells. Thus, it was investigated whether it is possible to increase the number of GH-producing cells in the cell aggregate by using dexamethasone as steroid during culture after purification.

Cell aggregates obtained by the same differentiation induction method as in Example 1, and on day 62 and day 100 after the start of differentiation induction were purified under conditions similar to those in Example 2. The obtained EpCAM-positive cells were reaggregated in a cell non-adhesive 96-well culture plate (10×10⁴ cells/200 µL/well) using a serum-free medium supplemented with 20% KSR and 20 µM Y27632 and suspension cultured under conditions of 37°C, 40% O₂, 5% CO₂. On day 3 from the start of suspension culturing, a half amount of the medium was changed with a serum-free medium not containing Y27632 and supplemented with dexamethasone (DX, manufactured by Aspen Japan K.K., 400 ng/mL), and 20% KSR. Long period culture was performed using a serum-free medium supplemented with DX (400 ng/mL) and 20% KSR from day 3 to day 24 from the start of suspension culturing, and a serum-free medium supplemented with 20% KSR after day 24 from the start of suspension culturing (Fig. 8).

Cell aggregates obtained by purifying cell aggregates on day 62 after the start of differentiation induction and culturing for a long period of 41 days after reaggregation (day 62 sort + day 41) and cell aggregates obtained by purifying cell aggregates on day 100 after the start of differentiation induction and culturing for a long period of 31 days after reaggregation (day 100 sort + day 31) were each fixed with 4% paraformaldehyde to prepare cryosections. These cryosections were subjected to immunostaining using epithelial cell marker EpCAM and pituitary hormone-producing cells marker growth hormone (GH) (anti-GH antibody, manufactured by Santacruz, mouse). Cell nuclei were stained with DAPI. These stained sections were observed using a confocal laser scanning microscope (manufactured by OLYMPUS CORPORATION), and immunostained images were obtained. As a result, it was found that in cell aggregates of day 62 sort + day 41 and of day 100 sort + day 31, GH-positive pituitary hormone-producing cells were present even under culture conditions where DX was not added (Fig. 9B, D), but the number of GH-positive cells further increases under culture conditions where DX was added (Fig. 9F, H). That is, it was shown that differentiation into GH-producing cells is induced by adding DX after purification and reaggregation.

### Example 4: Example of ACTH and GH secretion test of cell aggregate containing pituitary hormone-producing cells by long period culture after EpCAM purification

In Example 2 and Example 3, it was found that cell aggregates after purification contain pituitary hormone-producing cells such as ACTH, GH and the like. Thus, the effect of long period culture after purification on ACTH and GH secretory capacity was investigated.

Cell aggregates obtained by the same differentiation induction method as in Example 1, and on day 62 and day 100 after the start of differentiation induction were purified under conditions similar to those in Example 2 or Example 3 and subjected to long period suspension culturing.

Cell aggregates on days 62 and 100 after the start of differentiation induction were purified and reaggregated, the culture supernatants of each of the resulting EpCAM-positive and EpCAM-negative cell aggregates were recovered over time, and the secretion amounts of ACTH and GH were measured. The culture supernatant of the cell aggregates before purification (day 62 sort + day 41 = 103 days after the start of differentiation induction), which corresponds to the same number of days in which the purified cell aggregates were cultured, was also collected and measured for comparison. ACTH and GH concentrations in the collected culture supernatant were measured by the ELISA method used in clinical testing (testing outsourced to SRL Co., Ltd.). Based on the obtained ACTH and GH concentration data (pg/mL, ng/mL), the secretion amount per cell aggregate (pg/aggregate, ng/aggregate) was calculated from the total number of cell aggregates at the time of sampling and the total volume of culture medium, and graphed. Cell aggregates before purification were cultured at 20 to 60 aggregates/20 mL/dish, and cell aggregates after purification were cultured at 1 aggregate/200 µL/well.

As a result, the ACTH secretion amount increased over time in the cell aggregates obtained by purifying and reaggregating cell aggregates on day 62 after the start of differentiation induction and suspension culturing for 69 days (Fig. 10A). Furthermore, it was found that the GH secretion amount increased and not only ACTH but also GH were simultaneously secreted by culturing with DX added after purification (Fig. 10B).

In addition, in the cell aggregates obtained by purifying and reaggregating cell aggregates on day 100 after the start of differentiation induction and suspension culturing for 31 days, the secretion amount of ACTH increased until day 13 from the start of suspension culturing, but decreased thereafter (Fig. 11A). Furthermore, by adding DX and culturing after purification, the amount of GH secreted increased over time, and it was found that not only ACTH but also GH was simultaneously secreted (Fig. 11B) .

From these results, it was found that long period culture for at least 13 days after purification and reaggregation improved the secretion capacity of ACTH and GH.

An ACTH stimulation test using CRH (manufactured by NIPRO ES PHARMA CO., LTD.) was performed using cell aggregates obtained by purifying and reaggregating cell aggregates on day 62 after the start of differentiation induction and suspension culturing for 41 days. Twenty cell aggregates were transferred to a 10 cm suspension culture dish containing a serum-free medium (10 mL) supplemented with 20% KSR, cultured for 24 hr under condition of 37°C, 40% O₂, and the culture supernatant was recovered. The cell aggregates were washed with a serum-free medium supplemented with 20% KSR, transferred to a new 10 cm suspension culture dish containing a serum-free medium (10 mL) supplemented with 20% KSR, and CRH 5 µg/mL was added. After culturing for 24 hr under condition of 37°C, 40% O₂, the culture supernatant was recovered. The ACTH concentration in the collected culture supernatant was measured by the ELISA method used in clinical testing (testing outsourced to SRL, Inc.).

As a result, the addition of CRH increased the amount of ACTH secretion by a little less than two times (Fig. 10C). From the above results, it was found that the cell aggregates obtained by long period culture after purification and reaggregation are cell aggregates containing functional pituitary hormone-producing cells that have not only secretion capacity but also responsiveness.

### Example 5: Example of forming cell aggregate containing hypothalamic cell, pituitary progenitor cell, and pituitary hormone-producing cell from human ES cells

Cell aggregates containing hypothalamic cells, pituitary progenitor cells, and pituitary hormone-producing cells were produced using the same differentiation induction method as in Example 1 (method without cell sorting using anti-EpCAM antibody). In order to confirm the degree of differentiation at each culture day after the start of differentiation induction, changes in the expression of pituitary markers (PITX1, LHX3, POMC (ACTH progenitor cells)) and hypothalamic markers (RAX, NKX2.1 (TTF1)) were examined by quantitative PCR. Specifically, it was performed as follows. RNA was extracted from six cell aggregates per sample using the RNeasy Micro Kit (Qiagen). Quantitative PCR was performed using the StepOne Plus real-time PCR system (Applied Biosystems) or Biomark HD (Fluidigm). As the probes for each gene, GAPDH (Hs02758991_g1), PITX1 (Hs00267528-m1), LHX3 (Hs01033412_m1), POMC (Hs01596743_m1), RAX (Hs00429459-m1), and TTF1 (Hs00968940-m1) (TaqMan Probes; Thermo Fisher Scientific) were used. The obtained data were normalized using GAPDH as an endogenous control, and quantitative results were obtained using the comparative Ct method (ΔΔCt method).

As a result, the expression of LHX3 increased from day 6 to day 60 after the start of differentiation induction, the expression of LHX3 increased from day 19 to day 30 after the start of differentiation induction, and the expression of POMC increased after day 30 after the start of differentiation induction (Fig. 12A). These results show that differentiation into the pituitary was induced in a stepwise manner.

On the other hand, the expression of RAX reached a plateau around day 3 to day 6 after the start of differentiation induction, and thereafter decreased (Fig. 12B). The expression of TTF1 increased from day 6 to day 19 after the start of differentiation induction, and thereafter decreased (Fig. 12B). These results suggest that in the initial stages of differentiation, differentiation into hypothalamus (ventral hypothalamus) proceeds along with the differentiation into pituitary precursor tissue.

### Example 6: Example of purifying EpCAM-positive cell population from cell aggregate containing hypothalamic cell, pituitary progenitor cell, and pituitary hormone-producing cell and producing cell aggregate containing pituitary hormone-producing cells by long period culture after purification

Using the same differentiation induction method as in Example 1, cell aggregates containing hypothalamic cells, pituitary progenitor cells, and pituitary hormone-producing cells were produced. Cell aggregates that were not subjected to cell sorting were cultured until day 131 after the start of differentiation induction. For some cell aggregates, cell sorting using an anti-EpCAM antibody was performed on days 30, 60, and 100 after the start of differentiation induction. Cell sorting was performed in the same manner as in Example 2. The purified EpCAM-positive cells or EpCAM-negative cells were reaggregated in a cell non-adhesive 96-well culture plate (10×10⁴ cells/200 µL/well) using a serum-free medium supplemented with 20% KSR and 20 µM Y27632 and suspension cultured under conditions of 37°C, 40% O₂, 5% CO₂. After the third day from the start of suspension culturing, a serum-free medium not containing Y27632 and supplemented with 20% KSR was used, and the medium was replaced by half the volume once in 3 to 4 days and the cells were cultured up to day 131 after the start of differentiation induction (Fig. 4). Thereafter, the gene expression of POMC, NESTIN and SOX11 in the obtained cell aggregates was quantified by quantitative PCR. The quantitative PCR and analysis thereof were performed in the same manner as in Example 5. As the probes for each gene, GAPDH (Hs02758991_g1), POMC (Hs01596743_m1), NESTIN (Hs04187831-g1), and SOX11 (Hs00846583_s1) (TaqMan Probes; Thermo Fisher Scientific) were used.

As a result, the expression level of POMC was high in the cell aggregates in which EpCAM-positive cells were reaggregated, and almost no expression of POMC was observed in the cell aggregates in which EpCAM-negative cells were reaggregated (Fig. 13A, C, E). This result was the same regardless of the timing of cell sorting using the anti-EpCAM antibody (Figs. 13A, C, E).

On the other hand, when sorting was performed on days 60 and 100 after the start of differentiation induction, the expression of NESTIN and SOX11, which are markers of off-target cells, was low in cell aggregates obtained by reaggregating EpCAM-positive cells, and the expression level of the same marker was high in cell aggregates obtained by reaggregating EpCAM-negative cells (Fig. 13D, F). On the other hand, when sorting was performed on day 30 after the start of differentiation induction, the expression level of NESTIN and SOX11 was high in cell aggregates obtained by reaggregating EpCAM-negative cells but also cell aggregates obtained by reaggregating EpCAM-positive cells (Fig. 13B).

These results revealed that pituitary cells can be purified by cell sorting using the EpCAM antibody. In addition, it was found that, in order to reduce off-target cells, it is preferable to perform sorting after 60 days after the start of differentiation induction in the differentiation induction method of the present application.

### Example 7: Example of purifying EpCAM-positive cell population from cell aggregate containing hypothalamic cell, pituitary progenitor cell, and pituitary hormone-producing cell and producing cell aggregate containing pituitary hormone-producing cells by long period culture after purification

Using the same differentiation induction method as in Example 1, cell aggregates containing hypothalamic cells, pituitary progenitor cells, and pituitary hormone-producing cells were produced. Cell sorting using an anti-EpCAM antibody was performed on day 60 after the start of differentiation induction, after which EpCAM-negative cells and EpCAM-positive cells were respectively reaggregated. Cell sorting and reaggregation were performed in the same manner as in Example 6. Thereafter, the cells were cultured up to day 103 or 107 after the start of differentiation induction, and cryosections were prepared as in Example 2. These cryosections were subjected to immunostaining with antibody described in Example 2, and cell nuclei were stained with DAPI. These stained sections were observed using confocal laser scanning microscopy (manufactured by OLYMPUS CORPORATION) and immunostained images were obtained.

As a result, the same stained image as in Example 2 was obtained for the reaggregate of EpCAM-positive cells. That is, it was found that EpCAM- and E-cadherin-positive cells showed plane adhesion to each other, an epithelial structure was formed on the entire surface of the aggregate, there were many ACTH-positive pituitary hormone-producing cells within the cell aggregate (10.8 ± 1.3% of total cells), and PRL-, FSH-, LH-, TSH-positive cells were found to exist (data not shown). Furthermore, as a result of immunostaining using an anti-ACTH antibody (mouse, 1:200; Fitzgerald) and an anti-CXADR antibody against the pituitary stem cell marker CXADR (rabbit; 1:100; Atlas antibodies), it was found that CXADR-positive and ACTH-negative cells were existed (Fig. 14A, B, C). Therefore, it was found that, in reaggregate of EpCAM-positive cells, not only pituitary hormone-producing cells but also CXADR-positive pituitary stem cells existed, and that CXADR-positive cells existed separately from ACTH-secreting cells.

On the other hand, in the reaggregate of EpCAM-negative cells, it was confirmed that hypothalamic cells (RAX-positive cells: Fig. 14E, F, H) and off-target cells (NESTIN-positive cells, SOX11 cells: Fig. 14E, F, G, I, J) were present and were non-pituitary cells (ACTH-negative, EpCAM-negative, and E-cadherin-negative: Fig. 14D).

Furthermore, after reaggregation of EpCAM-positive cells, cell aggregates cultured until day 201 after the start of differentiation induction were fixed using 4% paraformaldehyde, 1% glutaraldehyde, and 2% sucrose at 4°C for 3 days. After dehydration with an alcohol solution and polymerization and embedding with LR-WHLTE resin (Nissin EM) according to a conventional method, ultrathin sections were prepared and observed with an electron microscope (Hitachi H-7500). As a result, it was found that many island-like structures mutually formed a cluster in the cell aggregates (Fig. 15A, broken line). Many ciliated cells were scattered on the surface of the island-like structures and voids (* in the Figure) were existed between the islet-like structures. In other words, it was found that the entire cell aggregate had a sponge-like structure. Necrosis was not observed even in the cells in the center of the cell aggregate, and the reason therefor is considered to be that nutrients and oxygen from the culture medium can reach the inside due to the presence of voids. Furthermore, compared to unsorted cell aggregates, cells containing secretory granules were found to have weaker polarity toward the inside and outside, and as a result, it was suggested that the structure was more similar to the anterior trabecular structure made by pituitary endocrine cells of a human body (Fig. 15A, B). In addition, similar to the pituitary and hypothalamic organoid, the presence of folliculo-stellate cells (FSCs) was also observed (Fig. 15B, C, broken line).

In addition, the response of EpCAM-positive cell reaggregates to hormones was also examined. On day 60 after the start of differentiation induction, cell sorting was performed using an anti-EpCAM antibody, and then EpCAM-positive cells were reaggregated and cultured until day 103 after the start of differentiation induction. Thereafter, an ACTH stimulation test using CRH (manufactured by NIPRO ES PHARMA CO., LTD.) or dexamethasone (DX, manufactured by Aspen Japan K.K.) was conducted. Specifically, 20 cell aggregates were transferred to a 10 cm suspension culture dish containing 10 mL of serum-free medium supplemented with 20% KSR, and after culturing for 24 hr under conditions of 37°C, 40% O₂, the culture supernatant was recovered. After washing the cell aggregates with serum-free medium supplemented with 20% KSR, the cell aggregates were transferred to a 10 cm suspension culture dish containing 10 mL of fresh serum-free medium supplemented with 20% KSR, and 5 µg/mL CRH or 500 ng/mL dexamethasone was added. In each case, a no addition group was set as a control. After culturing for 24 hr under conditions of 37°C, 40% O₂, the culture supernatant was recovered. The ACTH concentration in the recovered culture supernatant was measured by the ELISA method used in clinical testing (testing outsourced to SRL, Ltd.). As a result, CRH stimulation increased the ACTH secretion amount by about 2.2 times, and dexamethasone stimulation decreased the ACTH secretion amount by about 50% (Fig. 16). These results indicate that hormone-induced homeostasis was maintained in reaggregate of EpCAM-positive cells.

### Example 8: Example of transplanting of EpCAM-positive cell reaggregates into hypopituitary mice to confirm drug efficacy

Using the same differentiation induction method as in Example 1, cell aggregates containing hypothalamic cells, pituitary progenitor cells, and pituitary hormone-producing cells were produced. Cell sorting using an anti-EpCAM antibody was performed on day 60 after the start of differentiation induction, after which EpCAM-positive cells were reaggregated. Cell sorting and reaggregation were performed in the same manner as in Example 6. Thereafter, the cells were cultured up to day 107 after the start of differentiation induction and transplanted to under the capsule of the left kidney of hypopituitary mice (10-17 weeks old) whose pituitary was surgically removed (transplantation group). A control group (sham group) was also set in which only the transplantation procedure was performed and no EpCAM-positive cells were transplanted. In addition, hypopituitary mice whose pituitary gland was surgically removed were produced by performing using a transaural approach on SCID mice (7-9 weeks old, male). 24 weeks after transplantation, the kidneys were removed and treated with various antibodies (human nuclei (mouse, 1:1000; Millipore), ACTH (mouse, 1:200; Fitzgerald), EpCAM (goat; 1:500; R&D Systems), CXADR (rabbit; 1:100; Atlas antibodies)), and immunostaining was performed using conventional methods. As a result, it was found that human nucleus-positive and EpCAM-positive grafts contained ACTH-positive cells (Fig. 17A, B, B', C). Furthermore, expression of CXADR was observed in the graft, particularly in the intercellular spaces, suggesting the existence of a pituitary stem cell niche-like structure for FSCs in the graft (Fig. 17D, E). Furthermore, no tumor formation was observed during the observation period. The scale bar at the bottom right of Fig. 17A, Fig. 17B, Fig. 17B', Fig. 17D, Fig. 17E indicates 50 µm, and the scale bar at the bottom right of Fig. 17C indicates 10 µm.

CRH stimulation was applied to the transplantation group and the sham group, and the effect on ACTH secretion capacity was examined. Specifically, mice were administered with CRH (2 µg/kg, intraperitoneal administration) 4, 12, or 24 weeks after transplantation, and blood was collected before CRH administration and 1 hr after CHR administration. Each blood ACTH concentration was measured using ACTH ELISA kit (MD Bioproducts).

As a result, 4 weeks after transplantation, the blood ACTH concentration in the transplantation group significantly increased compared to the sham group (Fig. 18A). A further increase in blood ACTH concentration due to CRH stimulation was also observed (Fig. 18A). An increase in blood ACTH concentration and a further increase in ACTH concentration due to CRH stimulation were observed until 24 weeks after transplantation (Fig. 18A).

Dexamethasone stimulation was applied to the transplantation group, and the effect on ACTH secretion capacity was examined. Specifically, dexamethasone was administered (0.2 mg, intramuscularly) 6 weeks after transplantation, and blood was collected before and 2 hr after dexamethasone administration. After blood was collected 2 hr after dexamethasone administration, CRH was further administered (2 µg/kg, intraperitoneal administration), and blood was collected 1 hr after CRH administration. Blood ACTH concentration was measured in the same manner as above.

As a result, it was found that the blood ACTH concentration significantly decreased in the group in which dexamethasone was administered to mice 6 weeks after transplantation (Fig. 18B). Furthermore, even when CRH was administered after dexamethasone administration, the blood ACTH concentration remained almost completely suppressed (Fig. 18B). The response to negative feedback signals induced by dexamethasone suggests that when ACTH secretion is no longer needed in the body, secretion stops due to stimulation from downstream glucocorticoid, which is important from the perspective of preventing glucocorticoid excess.

It is known that when a healthy person is under severe stress, such as an infection or surgery, a large amount of ACTH is secreted from the pituitary gland, which stimulates the secretion of corticosteroids, thereby showing resistance to stress. Therefore, LPS (InvivoGen, E. coli origin, serotype O111:B4) stimulation was applied to the transplantation group and the sham group to induce pseudo-infection stress, and the effect on ACTH secretion capacity was examined. Specifically, LPS was administered (2.2 µg/kg, intraperitoneal administration) to mice 4 weeks after transplantation, and blood was collected before LPS administration and 2 hr after LPS administration. Blood ACTH concentration was measured in the same manner as above.

As a result, no increase in ACTH secretion by LPS stimulation was observed in the sham group, but significant increase in ACTH secretion by LPS stimulation was observed in the transplantation group (Fig. 18C). The effect increased by increasing the number of EpCAM-positive cell aggregates to be transplanted, and was found to be dose-dependent (Fig. 18C). Therefore, it was suggested that the transplanted EpCAM-positive cells exhibited a response to infectious stress.

### Example 9: Example of forming cell aggregate containing hypothalamic cell, pituitary progenitor cell, and pituitary hormone-producing cell from human iPS cells

Cell aggregates containing hypothalamic cells, pituitary progenitor cells, and pituitary hormone-producing cells were produced using the same differentiation induction method as in Example 1 (method without cell sorting using anti-EpCAM antibody). Regarding the SAG treatment period, two groups of a group in which SAG is added from immediately after the start of differentiation induction until day 30 (see Fig. 2), and a group in which SAG is added from immediately after the start of differentiation induction and after day 30 (until days 61, 103, and 131) were set, and the ACTH secretion capacity was compared.

As a result, it was found that ACTH secretion capacity was improved by stopping SAG treatment on day 30 after the start of differentiation induction (Fig. 19).

### Example 10: Example of producing cell aggregates containing pituitary hormone-producing cells of ACTH and pituitary hormone-producing cells of GH in different ratios by varying the period of dexamethasone addition during long period culture after EpCAM purification

From the results of Example 3 and Example 4, it was found that differentiation into GH-producing cells could be induced by using DX during culture of purified cell aggregates. Therefore, the effect of varying the period of DX addition during culture on the differentiation of cell aggregates into ACTH-producing cells and GH-producing cells was investigated.

Cell aggregates obtained by the same differentiation induction method as in Example 1, and on day 63 after the start of differentiation induction were purified under conditions similar to those in Example 2. The obtained EpCAM-positive cells were reaggregated in a cell non-adhesive 96-well culture plate (10×10⁴ cells/200 µL/well) using a serum-free medium supplemented with 20% KSR and 20 µM Y27632 and suspension cultured under conditions of 37°C, 40% O₂, 5% CO₂. On day 3 after the start of suspension culturing, a half amount of the medium was changed with a serum-free medium not containing Y27632 and supplemented with DX (400 ng/mL), and 20% KSR. To examine the conditions of the DX addition period, conditions in which DX was added for 3, 7, and 14 days were compared with conditions in which no DX was added. When DX was added from day 3 after the start of suspension culturing, a serum-free medium supplemented with DX (400 ng/mL) and 20% KSR was used, and when DX was not added, a serum-free medium supplemented with 20% KSR was used for long period culture (Fig. 20) .

Cell aggregates obtained by purifying cell aggregates on day 63 after the start of differentiation induction and culturing for a long period of 40 days after reaggregation (day 63 sort + day 40) were each fixed with 4% paraformaldehyde to prepare cryosections. These cryosections were subjected to immunostaining using EpCAM, which is an epithelial cell marker, adrenocorticotropic hormone (ACTH), which is a pituitary hormone-producing cell marker, and growth hormone (GH). As a result, it was found that the number of ACTH-positive cells decreased and the number of GH-positive cells increased in the cell aggregates of day 63 sort + day 40 as the DX addition period became longer (Fig. 21).

In addition, the culture supernatants of EpCAM-positive and EpCAM-negative cell aggregates with DX added, which were obtained by purifying and reaggregating using cell aggregates on day 63 after the start of differentiation induction, were collected over time, and the secretion amounts of ACTH and GH were measured under the same conditions as in Example 4. As a result, in cell aggregates obtained by purifying cell aggregates on day 63 after the start of differentiation induction and culturing for a long period of 40 days after reaggregation (day63 sort + day40), the secretion amount of ACTH decreased in a manner dependent on the DX addition period (Fig. 22A) and the secretion amount of GH increased (Fig. 22B). That is, it was shown that the ratio of differentiation induction into ACTH-producing cells and GH-producing cells can be adjusted by changing the period of DX addition after purification and reaggregation.

Using cell aggregates obtained by purifying and reaggregating cell aggregates on day 63 after the start of differentiation induction, suspension culturing for 40 days, and treating with DX for 14 days, a GH stimulation test using GRF and a GH suppression test using somatostatin were conducted. Fifteen cell aggregates were transferred to a 6-well culture plate (manufactured by SUMITOMO BAKELITE CO., LTD., for suspension culturing, culture area 9.2 cm²) containing a serum-free medium (2 mL) supplemented with 20% KSR, cultured for 24 hr under conditions of 37°C, 40% O₂, and the culture supernatant was recovered. The cell aggregates were washed with a serum-free medium supplemented with 20% KSR, transferred to a 6-well culture plate containing a new serum-free medium (2 mL) supplemented with 20% KSR, and GRF 2 ug/mL or somatostatin 100 ng/mL was added. After culturing for 24 hr under condition of 37°C, 40% O₂, the culture supernatant was recovered. The ACTH concentration in the collected culture supernatant was measured by the ELISA method used in clinical testing (testing outsourced to SRL, Inc.). As a result, the addition of GRF increased the amount of GH secretion by a little less than two times (Fig. 22C), and the addition of somatostatin decreased the amount of GH secretion by 20% (Fig. 22D). From the above results, it was found that the cell aggregates obtained by DX treatment during long period culture after purification and reaggregation contain functional pituitary hormone-producing cells that have not only GH secretion capacity but also responsiveness.

### [Industrial Applicability]

The method of the present invention makes it possible to efficiently obtain a cell aggregate containing pituitary hormone-producing cells, by differentiating pluripotent stem cells into cell aggregates containing hypothalamic cells, pituitary progenitor cells, and pituitary hormone production, efficiently separating functional pituitary hormone-producing cells and/or progenitor cells therefor by using EpCAM marker, and further differentiating the cells. In addition, since separated and purified pituitary hormone-producing cells and the like exhibit superior pituitary hormone secretory capacity by physiological stimulation of pituitary hormone secretion, they can be used for the treatment and the like of diseases relating to pituitary gland.

This application is based on a patent application No. 2021-162253 filed in Japan (filing date: September 30, 2021) and a patent application No. 2022-116715 filed in Japan (filing date: July 21, 2022), the contents of which are incorporated in full herein.

## Claims

1. A cell aggregate containing a pituitary hormone-producing cell, wherein
(1) the percentage of cells positive for at least one of EpCAM and E-cadherin in the cell aggregate is not less than 80%,
(2) the percentage of neural cells in the cell aggregate is not more than 20%;
(3) the percentage of ACTH-positive cells in the cell aggregate is not less than 5%;
(4) the cell density of the cell aggregate is 3,000 to 20,000 cells/mm²,
(5) the major axis of the cell aggregate is 200 to 3,000 µm,
(6) on the surface of the cell aggregate, cells positive for at least one of EpCAM and E-cadherin form a plane attachment to each other to form an epithelial structure, and
(7) the cell aggregate forms a sponge-like structure in the inside.

2. The cell aggregate according to claim 1, wherein the percentage of pituitary stem cells in the cell aggregate is not less than 3%.

3. The cell aggregate according to claim 1, wherein the cell aggregate has an ACTH secretory capacity of 200 to 1,000,000 pg/mL.

4. The cell aggregate according to claim 1, wherein the cell aggregate has a GH secretory capacity of 0.05 to 100 ng/mL.

5. A cell population comprising the cell aggregates according to claim 1 in not less than 40% of the total number of cell aggregates.

6. A therapeutic drug for a disease caused by a disorder of the pituitary gland, comprising the cell aggregate according to claim 1.

7. A method for producing a cell aggregate containing a pituitary hormone-producing cell, including the following steps:
(1) a step of separating a cell expressing EpCAM from a dispersed cell population derived from a pluripotent stem cell and including a hypothalamic cell, a pituitary progenitor cell, and a pituitary hormone-producing cell, and
(2) a step of suspension culturing the separated EpCAM-expressing cell for not less than 14 days under conditions that allow the pituitary progenitor cell to mature into a pituitary hormone-producing cell.

8. The production method according to claim 7, wherein the cell population of step (1) is obtained by the following step (A) and step (B):
(A) a step of culturing a pluripotent stem cell or a cell aggregate thereof under conditions that can induce differentiation into a cell aggregate containing a hypothalamic cell, a pituitary progenitor cell, and a pituitary hormone-producing cell, and
(B) a step of dispersing the cell aggregate containing a hypothalamic cell, a pituitary progenitor cell, and a pituitary hormone-producing cell, obtained in (A), into a cell population.

9. The production method according to claim 8, wherein the step (A) comprises the following steps:
(1') a first step of culturing pluripotent stem cells in the presence of a Wnt signal transduction pathway inhibiting substance to form a cell aggregate,
(2) a second step of culturing the cell aggregate, obtained in the first step, in the presence of a BMP signal transduction pathway activating substance and a Shh signal transduction pathway activating substance to obtain a cell aggregate containing a hypothalamic cell, a pituitary progenitor cell, and a pituitary hormone-producing cell.

10. The production method according to claim 8, wherein the step (A) comprises the following steps:
(a) step a of culturing pluripotent stem cells in the absence of feeder cells and in a medium comprising 1) a TGFβ family signal transduction pathway inhibiting substance and/or a Shh signal transduction pathway activating substance, and 2) a factor for maintaining an undifferentiated state,
(1') a first step of suspension culturing the pluripotent stem cells, obtained in step a, in the presence of a Wnt signal transduction pathway inhibiting substance to form a cell aggregate,
(2) a second step of suspension culturing the cell aggregate, obtained in the first step, in the presence of a BMP signal transduction pathway activating substance and a Shh signal transduction pathway activating substance to obtain a cell aggregate containing a hypothalamic cell, a pituitary progenitor cell, and a pituitary hormone-producing cell.

11. The production method according to claim 8, wherein the step (A) comprises the following steps:
(a) step a of culturing pluripotent stem cells in the absence of feeder cells and in a medium comprising 1) a TGFβ family signal transduction pathway inhibiting substance and/or a Shh signal transduction pathway activating substance, and 2) a factor for maintaining an undifferentiated state,
(1) a first step of suspension culturing the pluripotent stem cells, obtained in step a, in the presence of a JNK signal transduction pathway inhibiting substance and a Wnt signal transduction pathway inhibiting substance to form a cell aggregate,
(2) a second step of suspension culturing the cell aggregate, obtained in the first step, in the presence of a BMP signal transduction pathway activating substance and a Shh signal transduction pathway activating substance to obtain a cell aggregate containing a hypothalamic cell, a pituitary progenitor cell, and a pituitary hormone-producing cell.

12. The production method according to claim 11, wherein the suspension culturing in the first step is further in the presence of a Shh signal transduction pathway activating substance, and a period of the culture in the presence of a Shh signal transduction pathway activating substance in the first step and the second step is 30 days.

13. The production method according to any one of claims 9 to 12, wherein the cell aggregate obtained in the second step is further suspension cultured in the absence of a Shh signal transduction pathway activating substance.

14. The production method according to any one of claims 8 to 12, wherein the culture period in step (A) is a period with which a cell aggregate containing a pituitary hormone-producing cell that secretes ACTH and not comprising a pituitary hormone-producing cell that secretes GH or TSH is obtained.

15. The production method according to any one of claims 8 to 12, wherein the culture period in step (A) is not less than 30 days and not more than 100 days.

16. The production method according to any one of claims 7 to 12, wherein the cell aggregate containing a pituitary hormone-producing cell has the following characteristics:
(1) the percentage of cells positive for at least one of EpCAM and E-cadherin in the cell aggregate is not less than 80%,
(2) the percentage of neural cells in the cell aggregate is not more than 20%;
(3) the percentage of ACTH-positive cells in the cell aggregate is not less than 5%;
(4) the cell density of the cell aggregate is 3,000 to 20,000 cells/mm²,
(5) the major axis of the cell aggregate is 200 to 3,000 µm,
(6) on the surface of the cell aggregate, cells positive for at least one of EpCAM and E-cadherin form a plane attachment to each other to form an epithelial structure, and
(7) the cell aggregate forms a sponge-like structure.

17. The production method according to claim 16, wherein the percentage of pituitary stem cells in the cell aggregate is not less than 3%.

18. The production method according to claim 16, wherein the cell aggregate has an ACTH secretory capacity of 200 to 1,000,000 pg/mL.

19. The production method according to claim 16, wherein the cell aggregate has a GH secretory capacity of 0.05 to 100 ng/mL.
